(19) Europäisches Patentamt
European Patent Office
Office européen des brevets

(11) EP 2 982 669 B1

(12) FASCICULE DE BREVET EUROPEEN

(45) Date de publication et mention de la délivrance du brevet:
**10.05.2017 Bulletin 2017/19**

(21) Numéro de dépôt: **15183767.1**

(22) Date de dépôt: **11.03.2010**

(51) Int Cl.:
*C07D 255/02* (2006.01) *A61K 31/145* (2006.01)
*A61K 31/395* (2006.01) *A61K 38/08* (2006.01)
*C07K 7/06* (2006.01) *A61P 25/28* (2006.01)

# (54) AGENTS CHELATANTS D'IONS METALLIQUES, LEURS PROCEDES DE PREPARATION ET LEURS APPLICATIONS

CHELATBILDNER FÜR METALLIONEN, IHRE HERSTELLUNGSVERFAHREN UND IHRE ANWENDUNGEN

METAL-ION CHELATING AGENTS, METHODS FOR PREPARING SAME AND USES THEREOF

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **13.03.2009 FR 0901193**

(43) Date de publication de la demande:
**10.02.2016 Bulletin 2016/06**

(62) Numéro(s) de document de la (des) demande(s) initiale(s) en application de l'article 76 CBE:
**10712452.1 / 2 406 238**

(73) Titulaire: **COMMISSARIAT À L'ÉNERGIE ATOMIQUE ET AUX ÉNERGIES ALTERNATIVES**
**75015 Paris (FR)**

(72) Inventeurs:
- **DELANGLE, Pascale**
  **38500 VOIRON (FR)**
- **GATEAU, Christelle**
  **38560 CHAMP SUR DRAC (FR)**
- **PUJOL, Anaïs**
  **33510 ANDERNOS LES BAINS (FR)**

(74) Mandataire: **Gevers & Orès**
**41 avenue de Friedland**
**75008 Paris (FR)**

(56) Documents cités:
- **PLUSH SALLY E ET AL: "Aminoacid N-substituted 1,4,7-triazacyclononane and 1,4,7,10-tetraazacyclododecane Zn2+, Cd2+ and Cu2+ complexes. A preparative, potentiometric titration and NMR spectroscopic study.", DALTON TRANSACTIONS (CAMBRIDGE, ENGLAND : 2003) 7 MAY 2004, no. 9, 7 mai 2004 (2004-05-07), pages 1410-1417, XP002549246, ISSN: 1477-9226**
- **MADELEINE SCHULTZ ET AL: "Synthesis of dianionic and trianionic chiral, chelating ligands based on amino acids", AUSTRALIAN JOURNAL OF CHEMISTRY, CSIRO, AU, vol. 61, no. 4, 1 janvier 2008 (2008-01-01), pages 297-302, XP009123961, ISSN: 0004-9425**
- **DEHARO, ERIC ET AL: "Aminothiol Multidentate Chelators against Chagas Disease", EXPERIMENTAL PARASITOLOGY , 94(3), 198-200 CODEN: EXPAAA; ISSN: 0014-4894, 2000, XP002549247,**
- **LOYEVSKY, MARK ET AL: "Aminothiol multidentate chelators as antimalarials", BIOCHEMICAL PHARMACOLOGY , 54(4), 451-458 CODEN: BCPCA6; ISSN: 0006-2952, 1997, XP002549248,**
- **MARTELL ARTHUR E ET AL: "Stability constants of metal complexes of macrocyclic ligands with pendant donor groups", SUPRAMOLECULAR CHEMISTRY, GORDON AND BREACH SCIENCE PUBLISHERS, US, vol. 6, no. 3-4, 1 janvier 1996 (1996-01-01), pages 353-363, XP009123814, ISSN: 1061-0278**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 982 669 B1

• LEWIS M R ET AL: "Maleimidocysteineamido-DOTA Derivatives: New Reagents for Radiometal Chelate Conjugation to Antibody Sulfhydryl Groups Undergo pH-Dependent Cleavage Reactions", BIOCONJUGATE CHEMISTRY, ACS, WASHINGTON, DC, US, vol. 9, no. 1, 1 janvier 1998 (1998-01-01) , pages 72-86, XP002122221, ISSN: 1043-1802

## Description

La présente invention concerne de nouveaux composés de formule (I) susceptibles d'être utilisés comme agents chélatants d'ions métalliques ou comme agents dépolluants, de nouveaux composés de formule (II) ou ($II_{a'}$) susceptibles d'être utilisés comme agents précurseurs des composés de formule (I), ainsi que des composés marqués de formule (III), l'utilisation des composés de formule (I), (II), ($II_{a'}$) ou (III) en tant que médicaments, ainsi que leur utilisation pour le diagnostic, la prévention et le traitement de maladies neurodégénératives, telles que les maladies de Wilson et d'Alzheimer, et pour le diagnostic, la prévention et le traitement d'intoxications avec des ions métalliques tels que les ions argent, cadmium, cobalt, cuivre, mercure, nickel, or, plomb et zinc.

Les maladies liées à des dérèglements du transport du cuivre, comme la maladie de Wilson, conduisent à une accumulation du cuivre dans le foie, qui est l'unique organe capable de l'excréter. Ainsi, bien que le cuivre soit un élément essentiel à la vie, il peut, à l'état libre, induire des réactions d'oxydation de type Fenton, et par conséquent se révéler extrêmement toxique.

Plus particulièrement, la maladie de Wilson est une maladie génétique liée à une déficience d'un transporteur du cuivre conduisant à une accumulation du cuivre dans différentes zones de l'organisme (jusqu'à 20 fois les taux normaux), et se manifestant par des atteintes du foie et du système nerveux. Des troubles psychiques peuvent également apparaître avec des modifications du caractère, conduisant à une hyperémotivité avec une grande labilité de l'humeur, des syndromes dépressifs et des états de psychose.

La maladie de Wilson est induite par la mutation du gène ATP7B, qui code pour une protéine transmembranaire de type ATPase, intervenant dans le transport intra- et extra-cellulaire du cuivre, permettant ainsi de réguler la concentration de ce métal et son excrétion dans la bile. Si la protéine est déficiente, le métal s'accumule alors à l'intérieur des cellules. L'atteinte du foie précède en règle générale l'atteinte neurologique de quelques années.

Les signes neurologiques ou psychiatriques concernent près de 50% des patients atteint de la maladie de Wilson. L'imagerie par résonance magnétique (IRM) montre des lésions de plusieurs structures cérébrales, même en l'absence de tout signe clinique et l'importance de celles-ci semble corréler avec le degré d'avancement de la maladie.

Dans les cas gravissimes d'hépatites fulminantes ou dans les atteintes graves essentiellement hépatiques, une transplantation du foie peut être envisagée.

A l'heure actuelle, il existe des traitements dont l'objectif consiste à éradiquer la toxicité du cuivre accumulé dans l'organisme.

Ces traitements doivent être suivis à vie, et ne doivent jamais être interrompus. Ils sont à base de médicaments chélateurs diminuant l'absorption du cuivre dans l'organisme, ou augmentant l'excrétion de ce métal. Les traitements doivent être soumis à une surveillance périodique, de façon à repérer l'apparition d'effets secondaires indésirables.

Les traitements existants emploient différents principes actifs, tels que :

- la D-pénicillamine (Pen), qui augmente l'excrétion urinaire du cuivre (G. J. Brewer, DDT, 2005, 10, pp. 1103-1109). La D-pénicillamine a une efficacité reconnue mais ses effets secondaires tendent à la faire remplacer par d'autres molécules. De plus, un certain nombre d'articles récents font état d'une aggravation de la maladie de Wilson par la D-pénicillamine et suggèrent de restreindre sa prescription dans cette indication ;
- la triéthylènetétramine (Trien), qui est un chélateur du cuivre souvent mieux toléré que la D-pénicillamine ;
- l'anion de l'ammonium tétrathiomolybdate (TTM), absorbé avec l'alimentation, qui se fixe avec les ions cuivre dans le tube digestif, empêchant ainsi leur absorption ;
- le zinc active la production de protéines, les métallothionéines, qui vont fixer le cuivre dans les cellules de la paroi de l'intestin (entérocytes) empêchant le passage de cet ion dans la circulation sanguine (B. Sarkar, Chem. Rev., 1999, 99, 2535-2544).

Actuellement, ce sont les médicaments à base de D-pénicillamine, dont le mécanisme d'action est encore mal connu, qui sont le plus utilisés. Par sa fonction SH, la D-pénicillamine peut :

- chélater le cuivre et le zinc, mais aussi le mercure et le plomb, et augmenter leur excrétion urinaire,
- réduire les ponts disulfures de certaines molécules : collagène, fibres élastiques, immunoglobulines, et ainsi modifier leur activité biologique,
- se combiner à d'autres molécules soufrées, en particulier la cystéine, en formant des ponts disulfures.

Il apparaît effectivement que la présence d'atomes mous tels que le soufre permet une chélation plus efficace des ions métalliques dits « ions mous » tels que le mercure (II) ou le cuivre (I).

Il existe également d'autres médicaments, dont l'action est à rapprocher de la D-pénicillamine en raison de la ressemblance de leurs propriétés pharmacologiques :

- le pyritinol, qui est une molécule symétrique formée de deux parties liées par un pont disulfure. Dans l'organisme, le pyritinol est coupé en deux molécules comportant chacune un groupement -SH. Toutefois, le pyritinol a été utilisé dans le traitement de la polyarthrite rhumatoïde avec des indications et des effets indésirables du même type que ceux de la D-pénicillamine,
- la tiopronine, qui est utilisée dans le traitement de fond de la polyarthrite rhumatoïde et de la lithiase cystinique.

Toutefois, les effets indésirables de la D-pénicillamine et des médicaments ayant un mode d'action similaire sont assez nombreux :

- cutanéo-muqueux précoces et peu graves : érythème, stomatite,
- cutanéo-muqueux tardifs et graves : toxicodermie, pemphigus, dermatomyosite,
- hématologiques : thrombopénie, leucopénie, agranulocytose, anémie hémolytique, justifiant la surveillance hématologique des malades traités,
- digestifs : agueusie,
- rénaux : protéinurie.

Les métaux sont également considérés comme des cibles thérapeutiques d'intérêt pour le diagnostic, la prévention et le traitement de maladies neurodégénératives telle que la maladie d'Alzheimer, pour laquelle la dérégulation de l'homéostasie du zinc et du cuivre joue un rôle critique. Le cuivre Cu(II) est complexé et réduit en cuivre Cu(I) par la protéine APP et le peptide Aβ, le cuivre Cu(I) s'accumulant alors dans les plaques amyloïdes avec le fer et le zinc (E. Gaggelli, H. Kozlowski, D. Valensin, G. Valensin, Chem. Rev., 2006, 106, pp. 1995-2044).

Le cuivre peut se présenter sous deux degrés d'oxydation différents : le cuivre Cu(I) ayant un degré d'oxydation +I, stable en milieu réducteur, et le cuivre Cu(II) ayant un degré d'oxydation +II, stable en milieu oxydant. Le cuivre présent dans les cellules humaines est principalement du cuivre Cu(I).

Des molécules, autres que la D-pénicillamine (Pen), peuvent donc également être utilisées pour chélater le cuivre *in vivo.* Il s'agit, par exemple, de l'acide 2,3-dimercaptosuccinique (DMSA) et de l'acide 2,3-dimercapto-1-propanesulfonique (DMPS) (O. Andersen, Chem. Rev., 1999, 99, pp. 2683-2710), du 2,3-dimercaptopropanol (BAL), de la triéthylènetétramine (Trien), de l'anion de l'ammonium de tétrathiomolybdate (TTM) (G. J. Brewer, F. K. Askari, J. Hepatol., 2005, 42, pp. S13-S21) et de l'acide éthylène-diamine-tétraacétique (EDTA), répondant aux formules semi-développées suivantes :

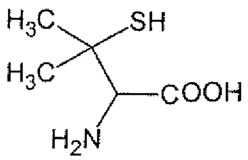

Pen

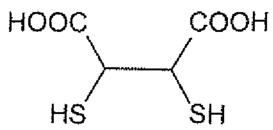

DMSA

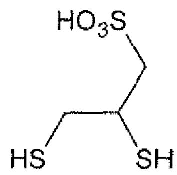

DMPS

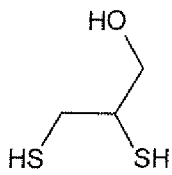

BAL

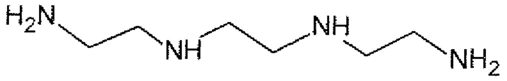

Trien

$MoS_4$ 2-

TTM

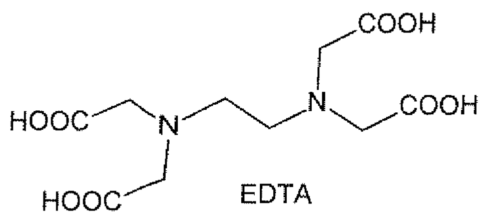

EDTA

Ces composés sont des agents chélatants connus du cuivre Cu(I) et/ou du cuivre Cu(II), bloquant l'absorption intestinale du cuivre.

Toutefois, ces composés conduisent à des effets secondaires indésirables, et ne permettent pas le traitement de patients pour lesquels les maladies ont été détectées à un stade déjà avancé (détection non précoce), et pour lesquels il existe une accumulation intracellulaire importante du cuivre.

De plus, certains agents chélatants, tels que le Trien et l'EDTA, sont des agents chélatants très forts, chélateurs de nombreux ions métalliques, et dont l'un des principaux inconvénients est leur manque de sélectivité.

Ainsi, il existe aujourd'hui un besoin en agents chélatants plus sélectifs, en particulier à l'égard du cuivre Cu(I) intracellulaire, et surtout moins toxiques, dont les effets secondaires seraient moins violents que ceux des molécules

actuellement utilisées.

Les inventeurs ont trouvé de manière surprenante que les nouveaux composés de l'invention décrits ci-après apparaissent comme une meilleure alternative, en particulier en terme de sélectivité, par rapport aux molécules précédemment développées, pour le diagnostic, la prévention et le traitement de maladies neurodégénératives et d'intoxications avec des ions métalliques tels que les ions argent, cadmium, cobalt, cuivre, mercure, nickel, or, plomb et zinc.

Ainsi, la présente invention concerne de nouveaux composés de formule (I) susceptibles d'être utilisés comme agents chélatants d'ions métalliques ou comme agents dépolluants, de nouveaux composés de formule (II) ou ($II_{a'}$) susceptibles d'être utilisés comme agents précurseurs des composés de formule (I) de l'invention, et des composés marqués de formule (III). La présente invention a également pour objet, l'utilisation des composés de formule (I), (II), ($II_{a'}$) ou (III) en tant que médicaments, ainsi que leur utilisation pour le diagnostic, la prévention et le traitement de maladies neurodégénératives, telles que les maladies de Wilson et d'Alzheimer.

Les composés de l'invention peuvent ainsi être utilisés pour le diagnostic et la prévention de maladies neurodégénératives chez des personnes présentant un risque plus important, du fait de facteurs génétiques ou environnementaux.

La présente invention a donc pour objet de nouveaux composés de formule (I) pouvant être utilisés comme agents chélatants d'ions métalliques, lesdits composés de formule (I) étant dérivés de l'acide 1,4,7-triazacyclononane-1,4,7-triacétique (NOTA), le NOTA étant déjà connu pour complexer des ions métalliques dans de nombreuses applications industrielles ou médicales.

Le NOTA est un composé organique cyclique de formule $C_{12}H_{21}N_3O_6$, qui dérive du cyclononane dans lequel trois groupes $CH_2$ équidistants ont été remplacés par des groupes N-$CH_2$-COOH. Le NOTA est un ligand hexadenté, ce qui signifie qu'il possède 6 atomes susceptibles de se lier à des ions métalliques. Cet agent chélatant est également très utilisé dans les compositions détergentes et pour le traitement de l'eau.

Ainsi, le premier objet de la présente invention concerne les composés de formule (I) suivante :

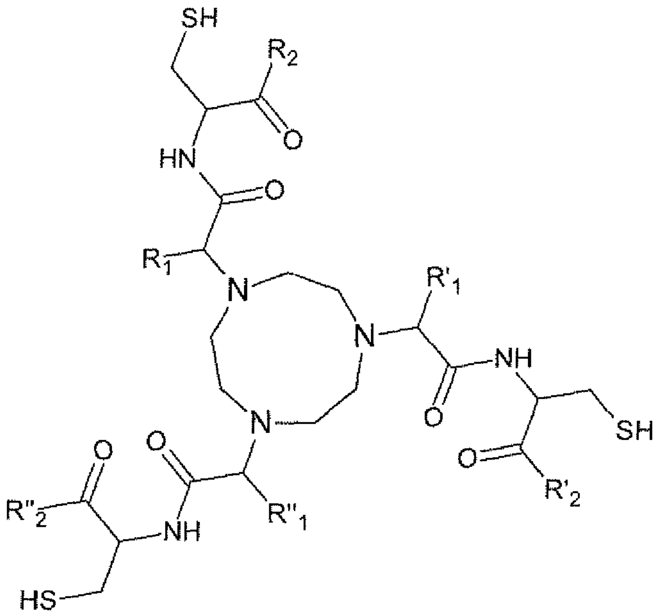

(I)

dans laquelle :

- les radicaux $R_1$, $R'_1$ et $R''_1$, identiques ou différents, représentent un atome d'hydrogène ou un radical alkyle ayant 1 à 12 atomes de carbone, les radicaux $R_1$, $R'_1$ et $R''_1$ étant de préférence des atomes d'hydrogène,
- les radicaux $R_2$, $R'_2$ et $R''_2$, identiques ou différents, sont choisis parmi les groupements -OH, -OR, -NHR et -NRR' dans lesquels R et R', identiques ou différents, représentent un atome d'hydrogène ou un radical alkyle ayant 1 à 12 atomes de carbone, les radicaux $R_2$, $R'_2$ et $R''_2$ étant de préférence des groupements -$NH_2$, -OH ou -OR dans lesquels R représente un atome d'hydrogène ou un radical alkyle ayant 1 à 12 atomes de carbone, et de manière encore plus préférée un radical éthyle.

Des agents chélatants dérivés du NOTA porteurs de résidus phénylalanines ou tryptophanes et des agents

chélatants dérivés du NTA porteurs de glycine, leucine, phénylalanine ou acide aspartique, ont également été décrits dans l'art antérieur (Dalton Trans., 2004, No. 9, 1410-1417 ; Aust. J. Chem., 2008, 61, 297-302). Ces composés, exempts de fonctions thiols, présentent néanmoins une faible affinité vis-à-vis de certains ions ($Cd^{2+}$ et $Cu^{2+}$ notamment), et une sélectivité insuffisante.

La présente invention concerne également un procédé de préparation des composés de formule (I) tel que défini dans la revendication 2.

Le présent exposé décrit un procédé de préparation des composés de formule (I) comprenant au moins les étapes suivantes :

(i) réaction d'un équivalent d'acide nitrilotriacétique (NTA) avec trois équivalents d'un dérivé de la cystéine de formule :

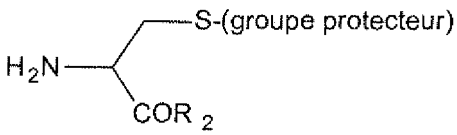

en présence d'un solvant polaire, tel que la diméthylformamide, le dichlorométhane, le chloroforme, le méthanol et l'éthanol, de préférence à une température comprise entre -10°C et 30°C pendant une durée comprise typiquement entre 12 et 48 heures,
(ii) de façon optionnelle, hydrolyse de la fonction $-COR_2$ du produit obtenu lors de l'étape (i) en fonction acide par addition d'une base forte, telle que l'hydroxyde de lithium (LiOH), la soude (NaOH), la potasse (KOH), la quantité de base forte ajoutée étant de préférence égale à quatre équivalents,
(iii) déprotection de la fonction -S-(groupe protecteur) en fonction thiol -SH, ladite déprotection pouvant être réalisée par addition d'un acide fort en large excès, tel que l'acide trifluoroacétique lorsque le groupe protecteur est le triphénylméthane $C(C_6H_5)_3$, de préférence à une température comprise entre 20°C et 40°C pendant une durée comprise typiquement entre 15 minutes et 1 heure.

Les groupes protecteurs des fonctions thiols sont bien connus de l'homme du métier, et peuvent être choisis parmi ceux mentionnés dans l'ouvrage de référence Protective groups in Organic Synthesis de T. W. Greene et P. G. M. Wuts, 3ème édition, Wiley, 1999. Les groupes protecteurs les plus préférés sont choisis parmi les groupements triphénylméthane - $C(C_6H_5)_3$, terbutyle $-C(CH_3)_3$, thio-terbutyle $-S-C(CH_3)_3$, 3-nitro-2-pyridinesulfonyle (Npys) et acétamidométhyle $-CH_2NHCOCH_3$, chacun de ces groupements ayant un mode de déprotection qui lui est propre tel que décrit dans la référence précédemment citée.

Lorsque les composés de l'invention répondent à la structure (I), les radicaux $R_2$, $R'_2$ et $R''_2$ peuvent avantageusement être des groupements $-NH_2$, -OH ou des groupements -OR dans lesquels R représente un atome d'hydrogène ou un radical alkyle ayant 1 à 12 atomes de carbone. De manière encore plus préférée, R est un groupement éthyle.

Le procédé de préparation des composés de formule (I) de l'invention peut être généralisé selon le schéma réactionnel suivant (lorsque $R_1 = R'_1 = R''_1$) :

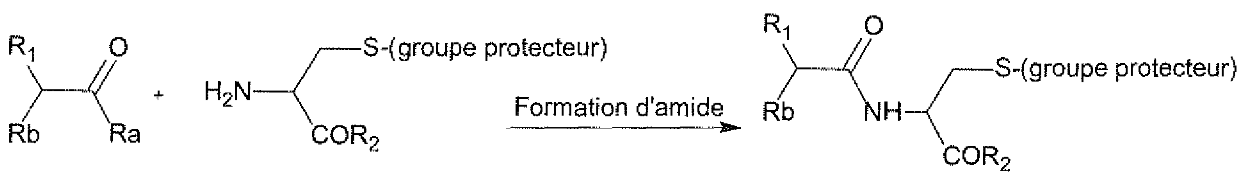


où :

- $R_a$ est sélectionné parmi les atomes d'halogène, de préférence les atomes de chlore ou de brome, les groupements hydroxyles -OH, $-OCOR_{a'}$ dans lesquels $R_{a'}$ représente un groupement alkyle ayant 1 à 12 atomes de carbone, $R_{a'}$ étant de préférence un groupement méthyle ou éthyle,
- $R_b$ est un groupe partant sélectionné parmi les atomes d'halogène, de préférence les atomes de chlore ou de brome, les groupements tosylates tels que le paratoluènesulfonate, et les groupements mésylates tels que le méthanesulfonate et le trifluorométhanesulfonate.

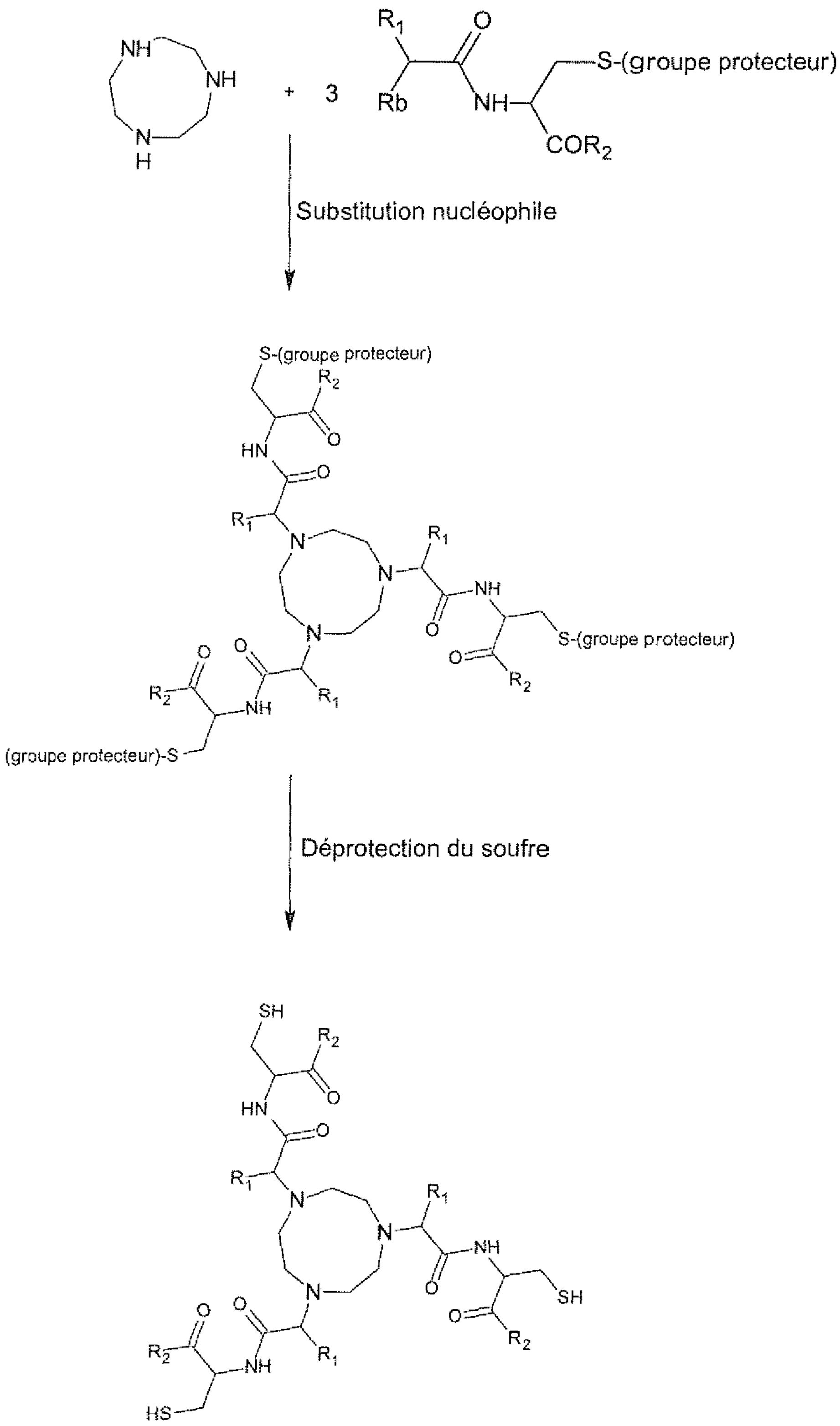


La présente invention concerne également un procédé de préparation des composés de formule (I) comprenant au moins les étapes suivantes :

(i) réaction d'un équivalent d'une molécule $R_bCHR_lC(O)R_a$ ($R_a$ et $R_b$ étant tels que définis ci-dessus) avec un équivalent d'un dérivé de la cystéine de formule :

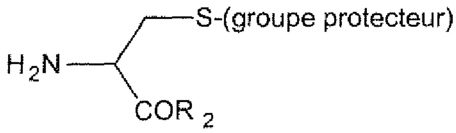

en présence d'une base faible, telle que l'hydrogénocarbonate de potassium ($KHCO_3$), l'hydrogénocarbonate de sodium ($Na_2CO_3$), le carbonate de potassium ($K_2CO_3$), le carbonate de sodium ($Na_2CO_3$) et les amines tertiaires comme la diisopropyléthylamine ($((CH_3)_2HC)_2$-N-$CH_2CH_3$) ou la triéthylamine ($N(CH_2CH_3)_3$), et en milieu solvant, ledit solvant pouvant être choisi parmi les solvants polaires tels que le dichlorométhane, le chloroforme, l'acétate d'éthyle, l'acétonitrile, la diméthylformamide et l'eau, de préférence à une température comprise entre -10°C et 10°C, pendant une durée comprise typiquement entre 30 minutes et 2 heures,

(ii) réaction d'un équivalent de 1,4,7-triazacyclononane (TCN) avec trois équivalents d'un dérivé bromo-acétamide obtenu lors de l'étape (i), de formule :

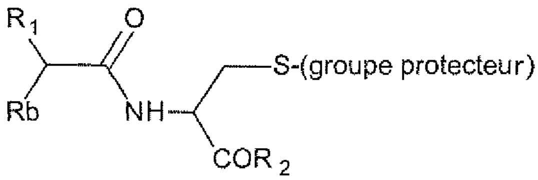

en présence d'une base faible, telle que l'hydrogénocarbonate de potassium ($KHCO_3$), l'hydrogénocarbonate de sodium ($Na_2CO_3$), le carbonate de potassium ($K_2CO_3$), le carbonate de sodium ($Na_2CO_3$) et les amines tertiaires comme la diisopropyléthylamine ($((CH_3)_2HC)_2$-N-$CH_2CH_3$) ou la triéthylamine ($N(CH_2CH_3)_3$), et en milieu solvant, ledit solvant pouvant être choisi parmi le dichlorométhane, le chloroforme, l'acétate d'éthyle, l'acétonitrile et la diméthylformamide,

(iii) déprotection de la fonction -S-(groupe protecteur) en fonction thiol -SH, ladite déprotection pouvant être réalisée par addition d'un acide fort en large excès, tel que l'acide trifluoroacétique lorsque le groupe protecteur est le triphénylméthane $C(C_6H_5)_3$, de préférence à une température comprise entre 20°C et 40°C pendant une durée comprise typiquement entre 15 minutes et 1 heure.

Un autre objet de l'invention concerne l'utilisation non thérapeutique des composés de formule (I) de l'invention comme agents chélatants des ions métalliques de la classification périodique de Mendeleiev, plus préférentiellement comme agents chélatants des ions mous et intermédiaires tels que définis dans R. G. Pearson, J. Am. Chem. Soc., 1963, vol. 85, pp. 3533-3539, et encore plus préférentiellement comme agents chélatants des ions Ag(I), Cd(II), Co(II), Cu(I), Hg(II), Ni(II), Au(I), Pb(II) et Zn(II), et plus particulièrement des ions cuivre Cu(I) intracellulaires.

Une autre utilisation possible des composés de formule (I) de l'invention est leur utilisation comme agents dépolluants pour dépolluer les eaux contaminées par des métaux. Lorsque les composés de formule (I) de l'invention sont utilisés comme agents dépolluants, la dépollution est alors de préférence réalisée en milieu réducteur. La valeur du pH limite du milieu dépend de l'ion métallique à complexer. Ainsi, pour les ions Hg(II) et Cu(I) le pH du milieu réducteur est de préférence supérieur ou égal à 1, et pour les ions Zn(II), Pb(II) et Cd(II) le pH du milieu réducteur est de préférence supérieur ou égal à 4 ou 6.

La présente invention concerne également des composés susceptibles d'être utilisés comme agents précurseurs des composés de formule (I), répondant à la formule (II) suivante :

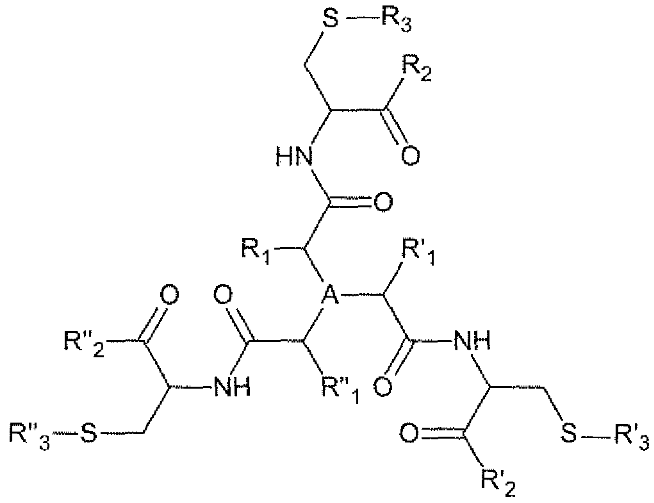

(II)

dans laquelle le groupement A représente :

- soit un atome d'azote,
- soit un cycle répondant à la formule ci-dessous, et dans lequel la substitution se fait sur les atomes d'azote :

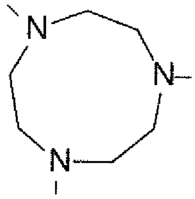

et dans laquelle

- les radicaux $R_1$, $R'_1$, $R''_1$, $R_2$, $R'_2$ et $R''_2$ ont la même signification que dans la formule (I) ci-dessus, et
- les radicaux $R_3$, $R'_3$ et $R''_3$, identiques ou différents, permettent de protéger les agents précurseurs tout en présentant des propriétés de complexation masquées, et représentent un groupement -S-W ou -S-E-L, où :

  ✓ S est un atome de soufre,
  ✓ W est un radical alkyle ayant 1 à 12 atomes de carbone,
  ✓ E est un bras espaceur sélectionné parmi les groupements alkyles ayant 1 à 12 atomes de carbone, et les polyols tels que le polyéthylène glycol ayant de préférence 1 à 8 motifs oxyéthylène OE,
  ✓ L est un ligand biologique, et de préférence un ligand de cellules hépatiques ou neuronales, sélectionné parmi les sucres tels que le glucose, le galactose et le N-acétylgalactosamine.

Ainsi, les composés de formule (II) de l'invention utilisés comme agents précurseurs peuvent également être assimilés à des « prodrugs » présentant des propriétés de complexation masquées via leurs fonctions thiols masquées.

Le procédé de préparation des composés de formule (II) de l'invention peut être généralisé selon le schéma réactionnel suivant :

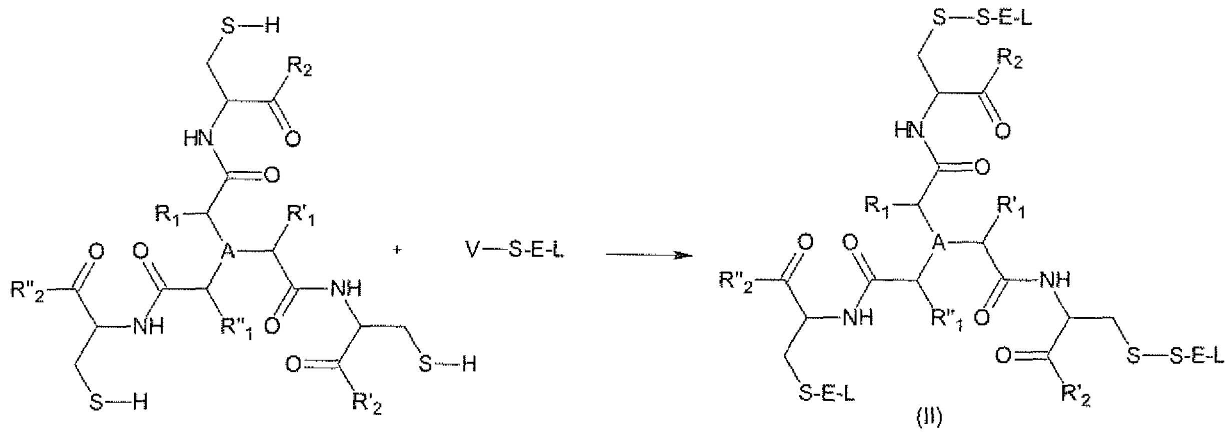

(II)

ou

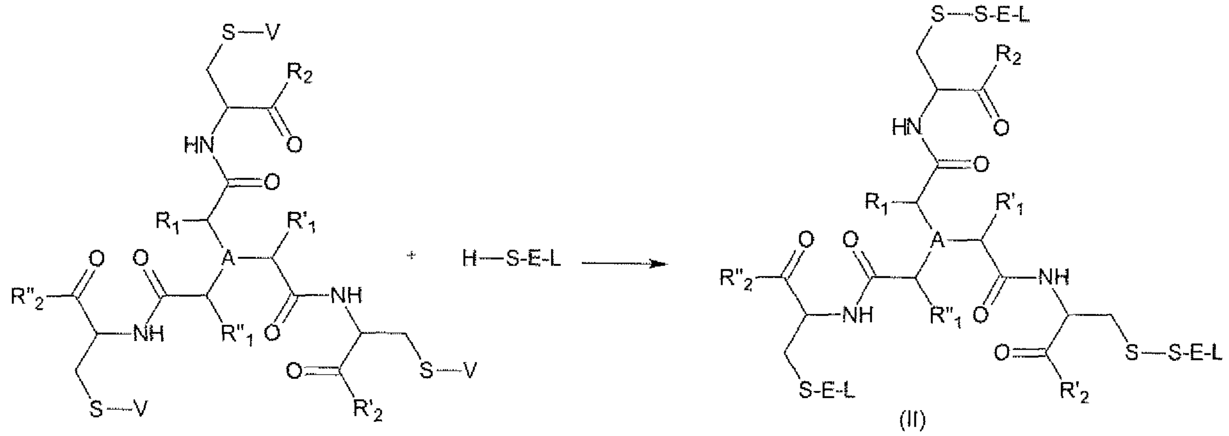

(II)

où V est un groupement activateur de la formation de ponts disulfure S-S choisi parmi les groupements tosyle, phényle, pyridine, nitro-pyridine et en particulier les groupements orthonitrophényle, tolyle, et en particulier les groupements paratolyle, 2-pyridinesulfonyle, et en particulier le groupement 3-nitro-2-pyridinesulfonyle (Npys), et tout autre groupement aromatique similaire éventuellement substitué.

Les composés de formule (II) de l'invention sont transformés en milieu réducteur en composés de formule (I), selon la réaction suivante :

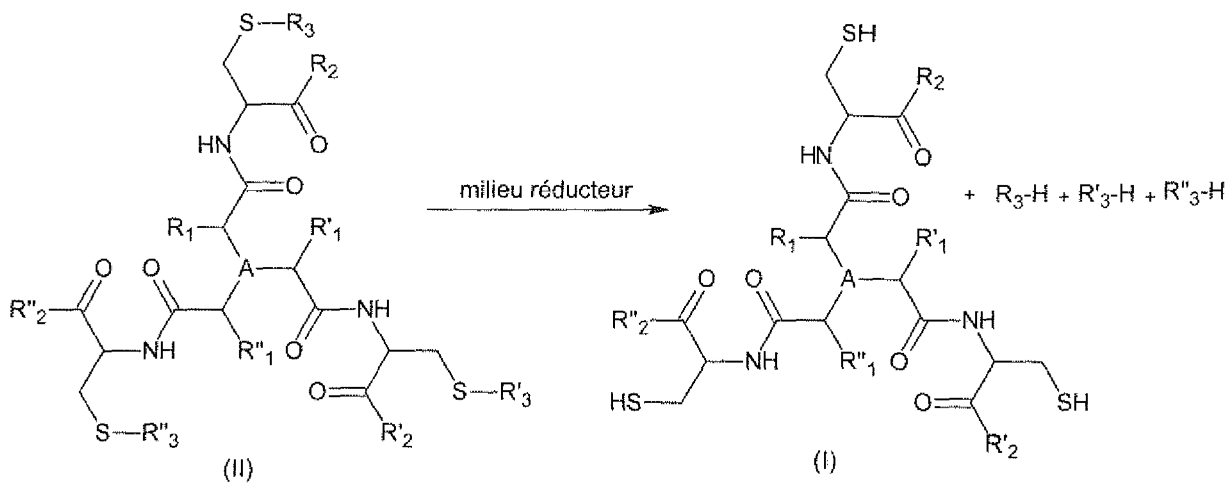


(II) (I)

ladite réaction permettant la libération des fonctions thiols, et par conséquent la libération des agents chélatants dans l'organisme, et plus particulièrement dans les cellules ciblées.

L'agent réducteur permettant l'obtention des composés de formule (I) peut être une molécule porteuse d'une fonction thiol, telle que l'éthanedithiol (EDT), le glutathion (GSH) et le dithiotréitol (DTT), l'acide ascorbique ou un de ses sels, ou une molécule porteuse d'une fonction phosphine telle que le tris(2-carboxyéthyl)phosphine (TCEP).

Ainsi, la libération des agents chélatants se fait par réduction *in vivo* dans l'organisme, par exemple dans les cellules hépatiques où le glutathion, qui est présent à environ 1 mM, peut jouer le rôle de réducteur, ou par exemple dans le cerveau où l'ascorbate, qui est présent à environ 200-400 μM, peut également jouer le rôle de réducteur.

Lorsque le groupement A des composés de formule (II) représente un atome d'azote, ces derniers peuvent être représentés par une structure de formule ($II_a$) spécifique :

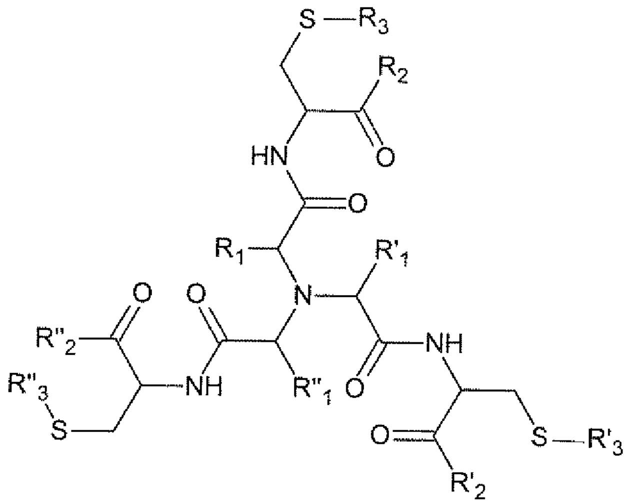

($II_a$)

Selon un mode de réalisation préféré, les composés de formule ($II_a$) répondent à la formule spécifique ($II_{a1}$) suivante :

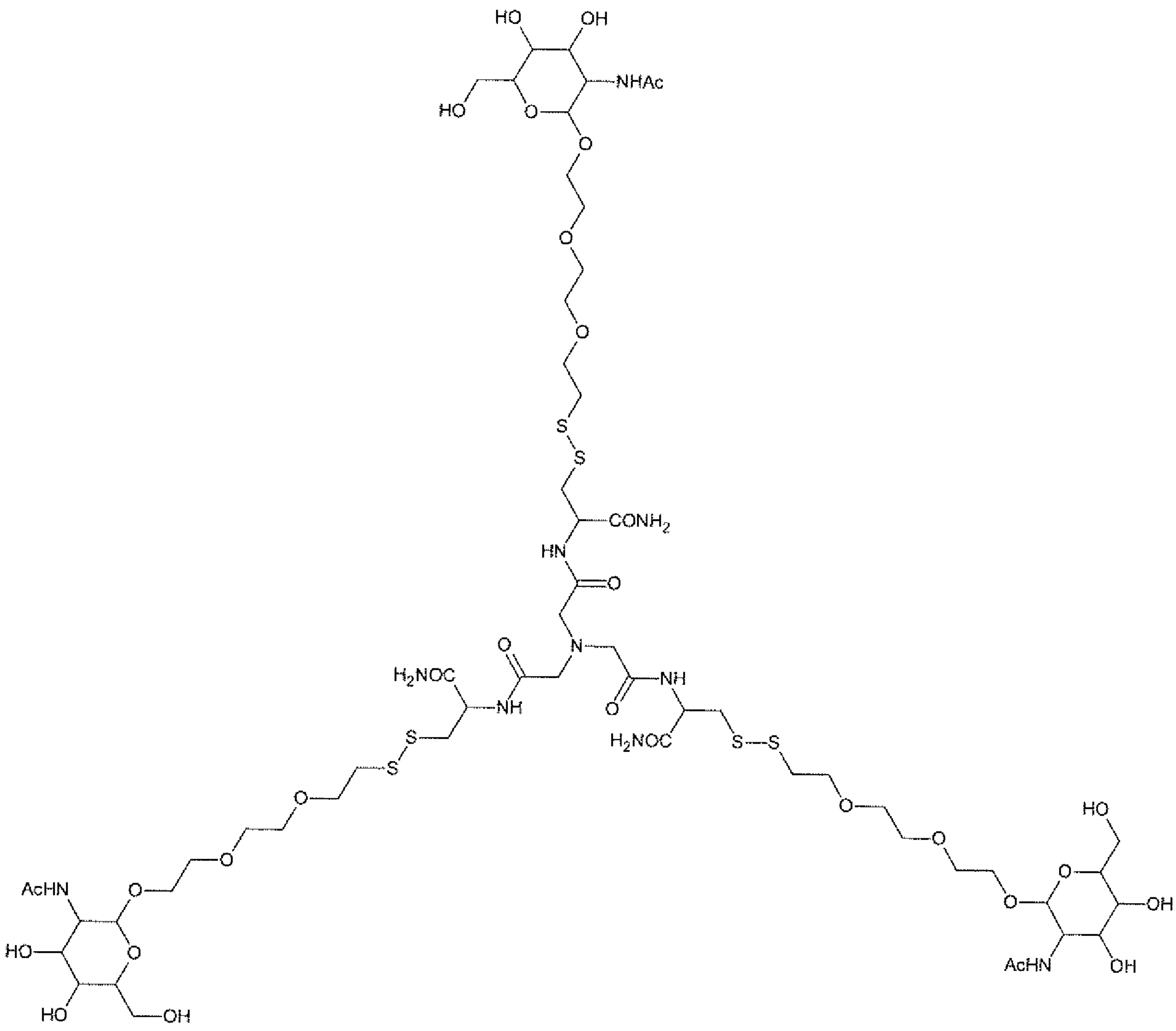

$(II_{a1})$

dans laquelle :

- les radicaux $R_1$, $R'_1$ et $R''_1$ sont des atomes d'hydrogène,
- les radicaux $R_2$, $R'_2$ et $R''_2$ sont des groupements $-NH_2$, et
- les radicaux $R_3$, $R'_3$ et $R''_3$, identiques ou différents, représentent un groupement -SE-L, où :

  ✓ S est un atome de soufre,
  ✓ E représente un polyéthylène glycol ayant 3 motifs oxyéthylène OE, et
  ✓ L représente le N-acétylgalactosamine.

Lorsque le groupement A des composés de formule (II) représente un cycle dérivé du cyclononane, ces derniers peuvent être représentés par une structure de formule $(II_b)$ spécifique :

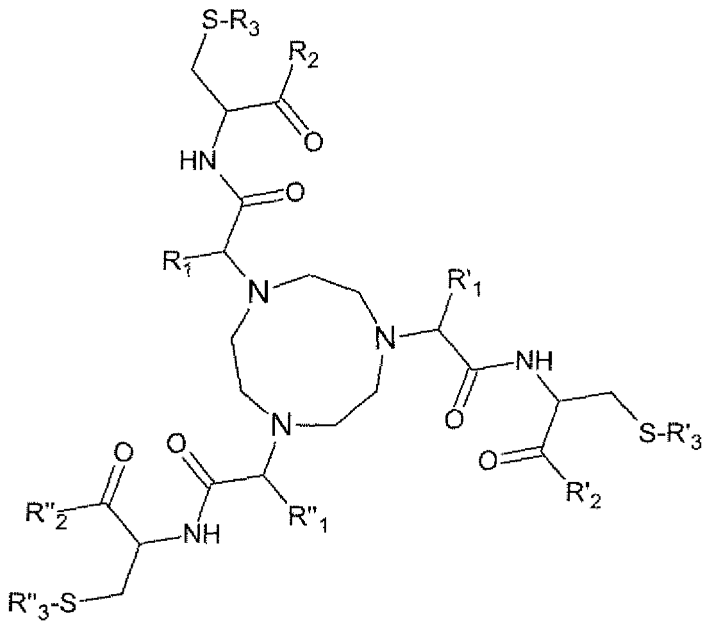

$(II_b)$

La présente invention a encore pour objet des composés répondant à la formule ($II_{a'}$) suivante :

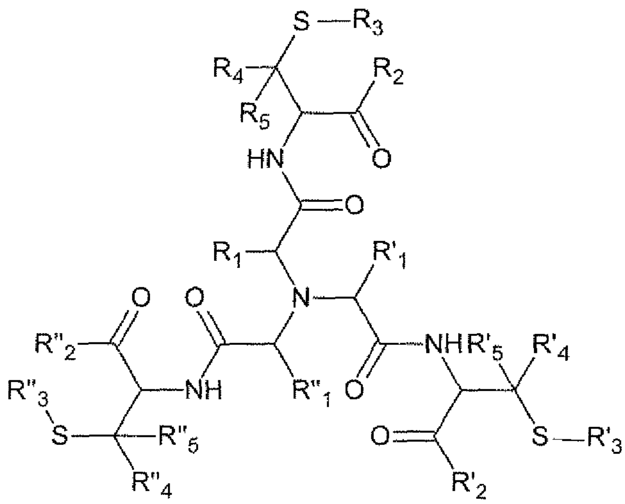

$(II_{a'})$

dans laquelle :

- les radicaux $R_1$, $R'_1$, $R''_1$, $R_2$, $R'_2$, $R''_2$, $R_3$, $R'_3$ et $R''_3$, ont la même signification que dans la formule (II) ci-dessus, et
- les radicaux $R_4$, $R'_4$, $R''_4$, $R_5$, $R'_5$, $R''_5$, identiques ou différents, représentent un atome d'hydrogène ou un radical alkyle ayant 1 à 12 atomes de carbone.

La présente invention a également pour objet des composés marqués répondant à la formule (III) suivante :

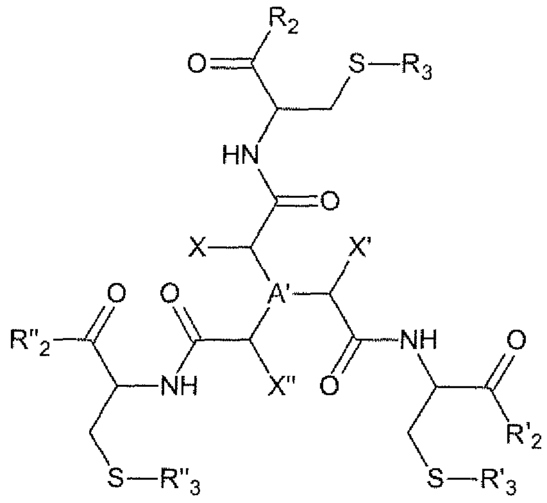

(III)

dans laquelle le groupement A' représente :

- soit un atome d'azote,
- soit un cycle répondant à la formule ci-dessous, et dans lequel la substitution se fait sur les atomes d'azote :

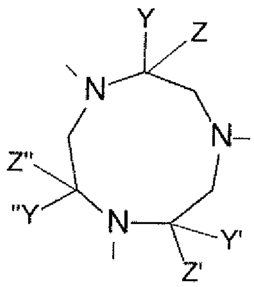

dans laquelle :

- les radicaux $R_2$, $R'_2$, $R''_2$, $R_3$, $R'_3$ et $R''_3$ ont la même signification que dans la formule (II) ci-dessus, et
- les groupements X, X', X", Y, Y', Y", Z, Z' et Z", identiques ou différents, représentent un atome d'hydrogène, un groupement $-(CH_2)_n$-NH-CO-marqueur, un groupement $-(CH_2)_n$-NH-C(S)NH-marqueur, un groupement $-(CH_2)_n$-NH-$SO_2$-marqueur, un groupement $-(CH_2)_n$-N=C-marqueur, un groupement - $(CH_2)_n$-NH-Ar-marqueur, ou $-(CH_2)_n$-triazole-marqueur où Ar est un groupement aryle choisi parmi le phényle, le triazole, l'oxadiazole, l'oxazole, l'imidazole, le thiadiazole, le pyrrole, le tétrazole, le furane, le thiophène, le pyrazole, la pyrazoline, la pyrazidine, le thiazole, l'isothiazole, la pyridine, la pyrimidine, la pipéridine, le pyranne, la pyrazine et la pyridazine, , et dans lesquels n est compris entre 1 et 12, et à la condition qu'au moins un desdits groupements X, X', X", Y, Y', Y", Z, Z' ou Z" soit un groupement $-(CH_2)_n$-NH-CO-marqueur, $-(CH_2)_n$-NH-C(S)NH-marqueur, $-(CH_2)_n$-NH-$SO_2$-marqueur, $-(CH_2)_n$-N=C-marqueur ou $-(CH_2)_n$-NH-Ar-marqueur ou $-(CH_2)_n$-triazole-marqueur.

Selon un mode de réalisation avantageux, les composés de formule (III) répondent à la formule spécifique ($III_1$) suivante :

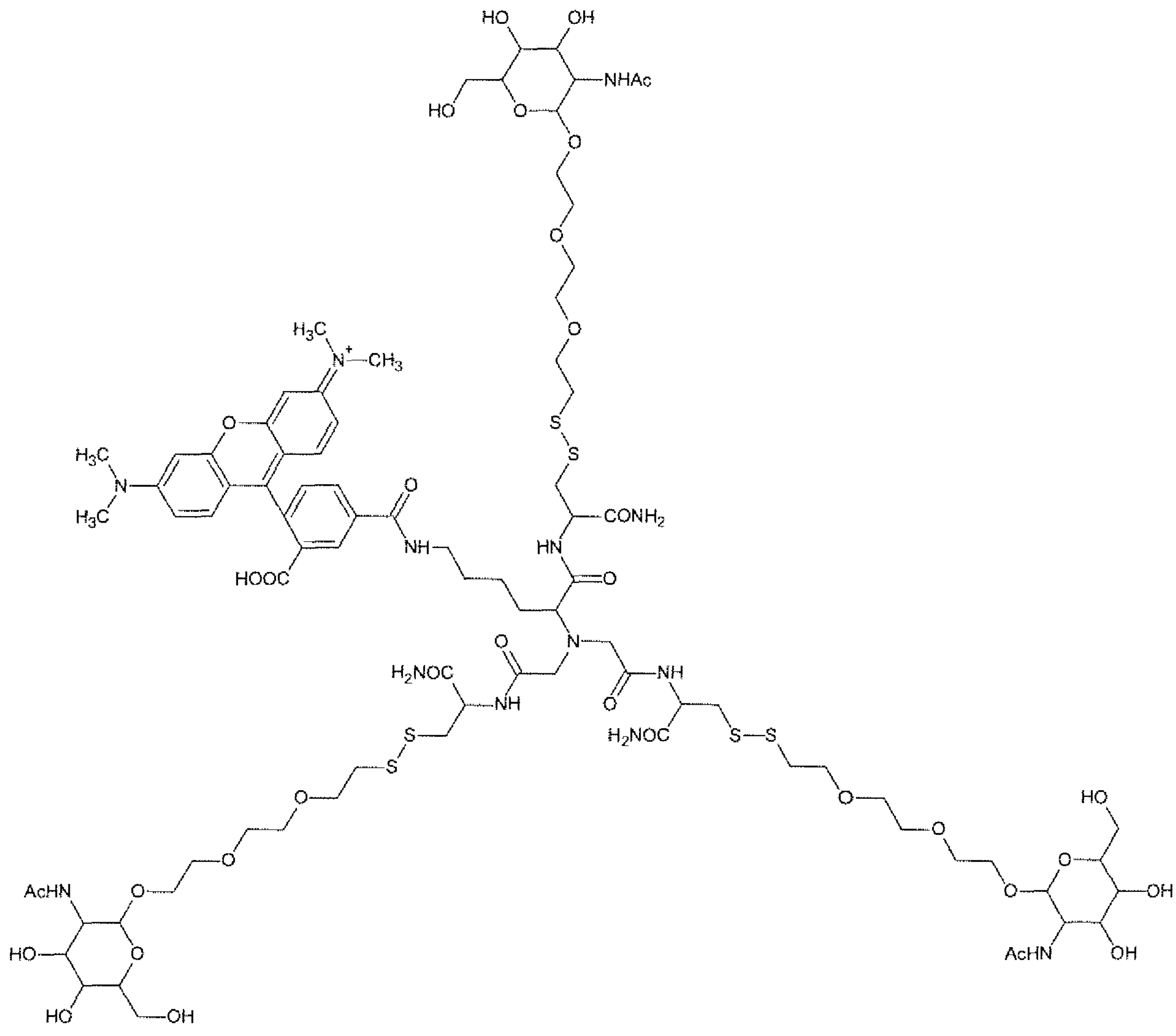

(III$_1$)

dans laquelle :

- les radicaux $R_1$, $R'_1$ et $R''_1$ sont des atomes d'hydrogène,
- les radicaux $R_2$, $R'_2$ et $R''_2$ sont des groupements $-NH_2$,
- les radicaux $R_3$, $R'_3$ et $R''_3$ représentent un groupement -S-E-L, où :

  ✔ S est un atome de soufre,
  ✔ E représente un polyéthylène glycol ayant 3 motifs oxyéthylène OE, et
  ✔ L représente le N-acétylgalactosamine,

- au moins un des groupements X, X', X", Y, Y', Y", Z, Z' ou Z" représente un groupement $-(CH_2)_4$-carboxytétraméthylrhodamine, les autres groupements représentant des atomes d'hydrogène.

On entend par marqueur toute entité susceptible d'être détectée par des moyens appropriés, les marqueurs utilisés dans le cadre de l'invention correspondant typiquement aux marqueurs utilisés par l'homme de l'art dans le domaine de la biologie pour marquer des molécules d'intérêt biologiques, notamment dans le cadre de la réalisation de diagnostic, d'études galéniques, ou encore de suivi de la métabolisation de composés actifs. Le marquage peut être de nature directe, et dans ce cas le marqueur est qualifié de « marqueur direct » et présente au moins une propriété physique détectable, ou le marquage peut être de nature indirecte, et dans ce cas le marqueur est qualifié de « marqueur indirect » et est susceptible de réagir sélectivement avec une entité tierce, cette dernière pouvant soit présenter au moins une propriété physique détectable, comme par exemple un anticorps présentant une activité fluorescente, soit être investie

dans un processus réactionnel à l'issu duquel une propriété physique pourra être détectée, comme par exemple lorsque le produit de dégradation de l'entité peut présenter au moins une propriété physique détectable telle que de la fluorescence. Le marquage indirect est souvent réalisé à l'aide d'anticorps ou de nanoparticules possédant une activité fluorescente. Dans ce cas, le marqueur indirect des composés de formule (III) possède une affinité pour l'entité tierce.

Ainsi, le marqueur de l'invention peut être soit une entité chimique de nature organique, soit une entité chimique de nature inorganique, telle qu'un complexe ou un cristal, ce dernier pouvant éventuellement être enrobé d'une couche organique, cette entité chimique de nature inorganique étant généralement de taille suffisamment faible, typiquement à l'échelle nanométrique, pour ne pas perturber le système biologique dans lequel elle est introduite.

La propriété physique détectable, directement ou indirectement, peut être une réactivité spécifique vis-à-vis d'une source électromagnétique telle qu'un champ magnétique, comme par exemple par imagerie par résonance magnétique, ou vis-à-vis d'un rayonnement lumineux pouvant être focalisé, comme par exemple par imagerie par fluorescence avec les fluorophores, ou encore vis-à-vis d'un rayonnement nucléaire, comme par exemple à l'aide d'isotopes.

Les marqueurs les plus préférés sont les marqueurs directs, et plus particulièrement les fluorophores. Typiquement, il s'agit de fluorophores organiques ou de nanoparticules.

Les fluorophores utilisés dans le cadre de l'invention peuvent être des composés fluorescents aromatiques dont les transitions $\pi$-$\pi$ sont caractérisées par des coefficients d'absorption molaires et des rendements quantiques de fluorescence élevées, lesdits fluorophores pouvant être choisis parmi la rhodamine, la fluorescéïne, la pyronine, la coumarine, la benzophénone, l'anthrone, la fluorénone, la pyridine, la quinoléine, l'acridine, le naphtalène, l'anthracène, la naphtacène, la pentacène, le xanthène et leurs dérivés.

Les différentes familles de marqueurs et les différentes techniques de détection associées sont connues de l'homme de l'art et décrites dans l'ouvrage Anti-Cancer Agents in Medicinal Chemistry, 2008, 8, 497-522. Plus spécifiquement, il est possible de se référer aux fluorophores cités dans Cytometry Part A 69A : 863-871 (2006) et aux nanoparticules mentionnées dans le document Anal. Bioanal. Chem., 384 : 620-630 (2006).

Ainsi, les composés marqués de formule (III) de l'invention peuvent être utilisés pour visualiser le cheminement desdits composés dans l'organisme, par luminescence.

Un autre objet de l'invention concerne l'utilisation des composés de formule (I), (II), ($II_{a'}$) ou (III) de l'invention pour leur application en tant que médicaments, et notamment pour la préparation d'un médicament destiné au diagnostic, à la prévention et/ou au traitement de maladies neurodégénératives, telles que les maladies de Wilson et d'Alzheimer. En effet, la chélation de certains ions métalliques tels que le cuivre ou le zinc peut s'avérer essentielle dans le traitement de maladies neurodégénératives, et plus particulièrement dans le traitement de la maladie d'Alzheimer (Buch A., Curent Opinion in Chemical Biology 2000, 4:184-191, Hui Hung Y. et al., J. Biol. Inorg. Chem (2010) 15:61-76).

Un objet supplémentaire de la présente invention concerne l'utilisation des composés de formule (I), (II), ($II_{a'}$) ou (III) de l'invention pour la préparation d'un médicament destiné au diagnostic, à la prévention et/ou au traitement d'intoxications avec des ions métalliques tels que les ions argent, cadmium, cobalt, cuivre, mercure, nickel, or, plomb et zinc, et encore plus préférentiellement avec les ions cuivre Cu(I) intracellulaires, les intoxications par de tels ions conduisant généralement à des inflammations sévères, des déficiences rénales, des hémorragies, des troubles neurologiques sévères du système nerveux central ; on parle alors de saturnisme dans le cas d'intoxications par le plomb ou d'hydrargie (ou d'hydrargyrisme) dans le cas d'intoxications par le mercure.

Enfin, la présente invention a également pour objet une composition pharmaceutique comprenant en tant que principe actif au moins un composé de formule (I), (II), ($II_{a'}$) ou (III) tel que défini précédemment, et au moins un véhicule pharmaceutiquement acceptable.

Lesdites compositions pharmaceutiques incluent aussi bien les compositions sous forme solide (comprimés, gélules, capsules, etc...), que les compositions sous forme liquide (solutions, suspensions ou émulsions) et incluent les excipients adaptés à une administration orale, topique ou parentérale.

L'administration des composés ou des compositions selon l'invention est effectuée de préférence par voie orale ou par voie parentérale (intraveineuse en perfusion ou injection, notamment).

Les doses de composés sont de préférence inférieures à 2 g de produit par jour, et varient selon la formulation sélectionnée, le mode d'administration et l'intoxication ou la maladie à traiter. D'autres facteurs tels que l'âge, le poids, la taille, le sexe ainsi que certains paramètres biologiques (taux d'excrétion, association avec d'autres médicaments, allergies...) sont également à prendre en compte.

Outre les dispositions qui précèdent, la description comprend encore d'autres dispositions qui ressortiront du complément de description qui suit, qui se rapporte à des exemples mettant en évidence les propriétés complexantes des composés de l'invention, ainsi qu'au dessin annexé dans lequel :

- la Figure 1 représente le dosage UV du $NTA(CysOC_2H_5)_3$ (composé 3) par du $Cu(CH_3CN)PF_6$ dans une solution tampon phosphate à 20 mM, de pH = 7,4, à une température de 298 K, et
- la Figure 2 représente l'évolution de la quantité de cuivre libre détectée par le BCS, mesurée par l'absorption UV du complexe $Cu(BCS)_2$, dans les conditions expérimentales suivantes : $[II_{a1}] = [4] = 50\ \mu M$ et $[Cu(I)] = 48\ \mu M$, en

tampon phosphate 20 mM, pH 7,4.

- La Figure 3 représente la pénétration du composé ($III_1$) dans les cellules hépatiques HepG2. Le composé ($III_1$) est visualisé à l'aide du fluorophore rhodamine par microscopie à fluorescence (grossissement X63) aux concentrations en composé ($III_1$) dans le milieu extracellulaires de 0,2 µM et 2 µM après deux heures.
- La Figure 4 représente la pénétration du composé ($III_1$) dans les cellules hépatiques HepG2. Le composé ($III_1$) est visualisé à l'aide du fluorophore rhodamine par microscopie à fluorescence (grossissement X63) aux concentrations en composé ($III_1$) dans le milieu extracellulaires de 0,2 µM et 2 µM

**Matières premières** :

**Tableau I :**

| Composé | Fournisseur |
|---|---|
| Acide nitriloacétique (NTA) | Sigma-Aldrich |
| N-éthyl-N'-(3-diméthylaminopropyl)carbodiimide | Sigma-Aldrich |
| Hydrate de 1-hydroxybenzotriazole | Sigma-Aldrich |
| Bromure de bromoacétyle | Sigma-Aldrich |
| Trihydrochlorure de 1,4,7-triazacyclononane | CheMatech |
| Disulfonate bathocuproïne (BCS) | Sigma-Aldrich |
| Acide 5,5'-dithiobis-2-nitrobenzoïque (DNTB) | Acros |
| Diméthylformamide (DMF) | Sigma-Aldrich |
| Acétate d'éthyle ($CH_3COOC_2H_5$) | Riedel de Haën |
| Hydrogénocarbonate de sodium ($NaHCO_3$) | Prolabo |
| Sulfate de sodium ($Na_2SO_4$) | Carlo Erba |
| Ethanol | Carlo Erba |
| Hydroxyde de lithium (LiOH) | Sigma-Aldrich |
| Acide chlorhydrique (HCl) | Sigma-Aldrich |
| Acide trifluoroacétique (TFA) | Riedel de Haën |
| Triéthylsilane $(HSiC_2H_5)_3$ | Sigma-Aldrich |
| Dichlorométhane ($CH_2Cl_2$) | Sigma-Aldrich |
| Bicarbonate de potassium ($KHCO_3$) | Sigma-Aldrich |
| Carbonate de potassium ($K_2CO_3$) | Sigma-Aldrich |
| Acétonitrile anhydre ($CH_3CN$) | Sigma-Aldrich |
| Ethylène-diamine-tétraacétique (EDTA) | Fischer Chemicals |

Les matières premières ont été utilisées sans purification supplémentaire.

Les solutions aqueuses ont été préparées à partir d'une eau ultra-pure, obtenue par filtration et purification par osmose inverse en utilisant une cartouche Millipore Milli-Q® (résistivité 18 MΩ.cm).

**Méthodes de caractérisation**:

**1/ Chromatographie sur couche mince (CCM)**

La CCM est réalisée sur un gel de silice 60 F254 (fournisseur : Merck).

**2/ Chromatographie-flash**

La chromatographie-flash est réalisée sur un gel de silice 60 d'épaisseur 40-63 μm (fournisseur : Merck).

**3/ Chromatographie en phase liquide à haute performance (HPLC)**

La chromatographie HPLC est réalisée sur un système VWR muni de colonnes RP18 (L = 250 mm, Ø = 4,6 mm et p = 5 μm pour la colonne analytique ; L = 250 mm, Ø = 50 mm et p = 10 μm pour la colonne préparatoire).

Les débits utilisés sont de 1 mL/min pour la colonne analytique et de 75 mL/min pour la colonne préparatoire, avec une détection UV à 214 nm.

Les conditions d'élution sont les suivantes :

- solvant A : mélange eau/acide trifluoroacétique (TFA) (99,925/0,075),
- solvant B = $CH_3CN$/eau/acide trifluoroacétique (TFA) (90/10/0,1).

**4/ Analyses RMN**

Les spectres RMN $^1H$ et $^{13}C$ ont été enregistrés sur un spectromètre Mercury Varian 400 et sur un spectromètre Bruker Avance 500.

Les déplacements chimiques sont indiqués en ppm avec le solvant comme référence interne.

**5/ Spectres de masse**

Les spectres de masse ont été réalisés sur un appareil Finigan LCQ-ion trap, équipé d'une source d'électrons.

Les analyses élémentaires ont été effectuées par le Service Central d'Analyse (Solaize, France).

**6/ Spectroscopie UV-visible**

Les spectres UV-visible ont été réalisés sur un spectrophotomètre Varian Cary 50.

**7/ Dichroïsme circulaire**

Les spectres de dichroïsme circulaire ont été réalisés avec un spectrophotomètre Chirascan (Applied Photophysics®).

**1/ Synthèses**

**Synthèse de la molécule HCysC($C_6H_5$)$_3$O$C_2H_5$ :**

La molécule HCysC($C_6H_5$)$_3$O$C_2H_5$ a été synthétisée à partir de L-cystéine selon le mode opératoire décrit dans la publication Bolzati et al., Bioconjugate chem., 2003, 14, 1231.

**Exemple 1** : Synthèse du composé 1 : NTA(CysC($C_6H_5$)$_3$O$C_2H_5$)$_3$

De l'acide nitriloacétique (0,196 g, 1,03 mmol) est ajouté à une solution de HCysC($C_6H_5$)$_3$O$C_2H_5$ (1,200 g, 3,06 mmol) dans 20 mL de diméthylformamide (DMF). Le mélange est ensuite refroidi à une température de 0°C, et du N-éthyl-N'-(3-diméthylaminopropyl)carbodiimide (0,587 g, 3,06 mmol) et de l'hydrate de 1-hydroxybenzotriazole (0,414 g, 3,06 mmol) sont ajoutés successivement. Le mélange réactionnel est ensuite mélangé à température ambiante pendant 24 h sous argon.

Après évaporation du solvant, le résidu est ensuite dissout dans 100 mL d'acétate d'éthyle. La phase organique est alors lavée avec 2 x 50 mL d'eau, puis 50 mL d'une solution saturée d'hydrogénocarbonate de sodium ($NaHCO_3$) et 2 x 50 mL d'une solution saturée de chlorure de sodium (NaCl).

La phase organique est ensuite séchée avec du sulfate de sodium ($Na_2SO_4$), puis concentrée sous pression réduite (20 mbars).

Le produit résultant (1,391 g) est purifié par chromatographie sur gel de silice (100 mL, éluant : $CH_2Cl_2$/acétate d'éthyle 80/20), pour donner le composé 1 (1,103 g, rendement = 82%) sous forme de poudre blanche.
RMN $^1H$ ($CD_3CN$, 400 MHz, 298 K) : $\delta$ = 1,05 (t, $J$= 7,0 Hz, 9H, $CH_3$) ; 2,39 et 2,68 (ABX, $J_{BX}$ = 4,1 Hz, $J_{AX}$ = 8,0 Hz, $J_{AB}$ = 12,7 Hz, 6H, $C\underline{H_2}S$) ; 3,17 et 3,29 (AB, $J_{AB}$ = 15,0 Hz, 6H, $C\underline{H_2}CO$) ; 3,84 et 3,96 ($ABX_3$, $J_{AX}$ = 7,0 Hz, $J_{BX}$ = 7,0

Hz, $J_{AB}$ = 10,9 Hz, 6H, $CH_2CH_3$) ; 4,34 (td, $J$ = 4,0 et 8,2 Hz, 3H, CH) ; 7,13-7,17 (m, 30H, $SC(C_6H_5)_3$) ; 7,22 (d, $J$ = 7,4 Hz, 15H, $SC(C_6H_5)_3$) ; 7,56 (d, $J$ = 8,6 Hz, 3H, NH).
RMN $^{13}C$ ($CD_3CN$, 100 MHz, 298 K) : δ = 14,39 ($CH_3$) ; 33,69 ($CH_2S$) ; 52,11 (CH) ; 57,97 ($CH_2CO$) ; 62,21 ($CH_2CH_3$) ; 129,90-127,16 ($(C_6H_5)_3$) ; 144,70 ($C(C_6H_5)_3$) ; 171,66 et 170,70 (2*CO).
ES-MS (m/z) : $[M + H^+]^+$ = 1310,8 et $[M + TEAH^+]^+$ = 1411,8, la valeur ES-MS correspondant à la masse sur la charge de l'ion détecté.
Analyse élémentaire calculée (en %) pour $C_{78}H_{78}N_4O_9S_2$, $2H_2O$ (1347,70 g/mol) : C, 69,51 ; H, 6,13 ; N, 4,16 ; trouvée : C, 69,42 ; H, 6,05 ; N, 3,9.

**Exemple 2 :** Synthèse du composé 2 : $NTA(CysC(C_6H_5)_3OH)_3$

Le composé 1 (0,310 g, 0,236 mmol) est dissout dans 6 mL d'éthanol, et de l'hydroxyde de lithium (LiOH) est ajouté (0,95 mL, 0,95 mmol). Le mélange réactionnel est ensuite agité pendant 1 h à température ambiante, puis évaporé. Le résidu obtenu est alors dissout dans 6 mL d'eau, et de l'acide chlorhydrique (HCl) à 1 mol/L est additionné jusqu'à pH = 4-5.
La phase aqueuse est ensuite extraite avec 15 mL d'acétate d'éthyle.
Le produit résultant (0,242 g, rendement = 83%) est ensuite utilisé sans purification supplémentaire.
RMN $^1H$ (DMSO-$d_6$, 400 MHz, 298 K) : 2,37-2,46 (m, 6H, $CH_2SC$) ; 3,32 (s, 6H, $CH_2CO$) ; 4,17-4,21 (m, 3H, CH) ; 7,20-7,37 (m, 45H, $C(C_6H_5)_3$) ; 8,46 (d, $J$ = 7,4 Hz, 3H, NH). RMN $^{13}C$ (DMSO-$d_6$, 100 MHz, 298 K) : δ = 34,03 ($CH_2S$) ; 52,34 (CH) ; 60,68 ($CH_2CO$) ; 130,00-127,67 ($(C_6H_5)_3$) ; 145,16 ($C(C_6H_5)_3$) ; 172,02 et 171,61 (2*CO).
ES-MS (m/z) : $[M + Na^+]^+$ = 1249,2.

**Exemple 3 :** Synthèse du composé 3 : $NTA(CysOC_2H_5)_3$

De l'acide trifluoroacétique (1,81 mL, 24,4 mmol) et du triéthylsilane (0,47 mL, 2,9 mmol) sont successivement additionnés au composé 1 (0,640 g, 0,49 mmol) dans 15 mL de dichlorométhane ($CH_2Cl_2$), sous argon.
Après 30 minutes d'agitation à température ambiante, le mélange est évaporé.
Le produit résultant (0,627 g) est ensuite purifié par HPLC : $t_R$ = 12,7 minutes (gradient linéaire 50/50 à 0/100, A/B en 15 minutes).
Le composé 3 obtenu est un solide blanc huileux (0,110g, rendement = 49%).
RMN $^1H$ ($CD_3CN$, 500 MHz, 298 K) : δ = 1,25 (t, $J$= 7,1 Hz, 9H, $CH_3$) ; 1,97 (t, $J$ = 8,8 Hz, 3H, SH) ; 2,95 et 3,00 (ABXY, $J_{AX}$ = 4,6 Hz, $J_{BX}$ = 6,1 Hz, $J_{BY}$ = 9,0, $J_{AY}$ = 9,3 Hz, $J_{AB}$ = 14,0 Hz, 6H, $CH_2SH$) ; 3,48 et 3,52 (AB, $J_{AB}$ = 16,3 Hz, 6H, $CH_2CO$) ; 4,18 et 4,22 ($ABX_3$ ; $J_{AX}$ = 7,1 Hz, $J_{BX}$ = 7,1 Hz, $J_{AB}$ = 10,8 Hz, 6H, $CH_2$-$CH_3$) ; 4,70 (ddd, $J$ = 4,7, 6,2 et 8,0 Hz, 3H, CH) ; 7,71 (d, $J$= 8,0 Hz, 3H, NH).
RMN $^{13}C$ ($CD_3CN$, 100 MHz, 298 K) : δ = 14,97 ($CH_3$) ; 27,40 ($CH_2SH$) ; 55,81 (CH) ; 59,75 ($CH_2CO$) ; 63,02 ($CH_2CH_3$) ; 171,61 et 172,02 (2*CO).
ES-MS (m/z): $[M + H^+]^+$ = 585,0 et $[M + Na^+]^+$ = 607,3.

**Exemple 4 :** Synthèse du composé 4 : $NTA(CysNH_2)_3$

De l'acide nitriloacétique (0,068 g, 0,357 mmol) est ajouté à une solution de $CysC(C_6H_5)_3(NH_2)$ (0,401 g, 1,10 mmol) dans 10 mL de diméthylformamide (DMF). Le mélange est ensuite refroidi à une température de 0°C, et du N-éthyl-N'-(3-diméthylaminopropyl)carbodiimide (0,212 g, 1,10 mmol) et de l'hydrate de 1-hydroxybenzotriazole (0,150 g, 1,11 mmol) sont ajoutés successivement. Le mélange réactionnel est ensuite mélangé à température ambiante pendant 24 h sous argon.
Après évaporation du solvant, le résidu est ensuite lavé avec 25 mL d'eau, puis filtré. Le solide est ensuite dissout dans 100 mL de dichlorométhane ($CH_2Cl_2$). La phase organique est alors lavée avec 3 x 50 mL d'eau et 1 x 50 mL d'une solution saturée de chlorure de sodium (NaCl). Elle est ensuite séchée avec du sulfate de sodium ($Na_2SO_4$), puis concentrée sous pression réduite (20 mbars) pour donner du $NTA(CysC(C_6H_5)_3(NH_2)_3)$ (0,404 g, rendement = 92%) sous forme de poudre blanche.
RMN $^1H$ ($CD_3CN$, 400 MHz, 298 K) : δ = 2,37-2,44 (m, 6H, $CH_2S$) ; 3,14 et 3,19 (AB, $J_{AB}$ = 16,4, 6H, $CH_2CO$) ; 4,00-4,06 (m, 3H, CH) ; 5,70 (s, 3H, $NH_2$) ; 6,24 (s, 3H, $NH_2$) ; 7,16-7,32 (m, 45H, $SC(C_6H_5)_3$) ; 7,85 (d, $J$= 7,2 Hz, 3H, NH).
RMN $^{13}C$ ($CD_3CN$, 100 MHz, 298 K) : δ = 38,89 ($CH_2S$) ; 57,78 (CH) ; 63,18 ($CH_2CO$), 132,37-134,89 ($(C_6H_5)_3$) ; 150,03 ($C(C_6H_5)_3$) ; 176,15 et 178,074 (2*CO).
ES-MS (m/z) : $[M + Na^+]^+$ = 1246,2 et $[M + K^+]^+$ = 1262,2.
Analyse élémentaire calculée (en %) pour $C_{72}H_{69}N_7O_6S_3$, $H_2O$ (1242,57 g/mol) : C, 69,60 ; H, 5,76 ; N, 7,89 ; O, 9,01 ; S, 7,74 ; trouvée : C, 69,60 ; H, 5,72 ; N, 7,95 ; O, 8,65 ; S, 7,83.
De l'acide trifluoroacétique (1,4 mL, 17,84 mmol) et du triéthylsilane (0,54 mL, 2,14 mmol) sont ensuite additionnés successivement au composé NTA(CysC($C_6H_5$)$_3$($NH_2$)$_3$) (0,437 g, 0,357 mmol) dans 15 mL de dichlorométhane ($CH_2Cl_2$) sous argon.

Après 50 minutes d'agitation à température ambiante, le mélange est évaporé.

Le produit résultant (0,600 g) est ensuite purifié par HPLC : $t_R$ = 16,4 minutes (gradient linéaire 50/50 à 0/100, A/B en 15 minutes).

Le composé 4 obtenu est un solide blanc (0,065 g, rendement = 37%).

RMN $^1$H ($D_2O$, 500 MHz, 298 K) : δ = 2,87 (ABX, $J_{AX}$ = 4,9, $J_{BX}$ = 7,5, $J_{AB}$ = 14,2, 6H, $CH_2$SH) ; 3,55 (s, 6H, $CH_2$CO) ; 4,45 (dd, *J*= 7,5, 3H, CH).

RMN $^{13}$C ($D_2O$, 100 MHz, 298 K) : δ = 28,18 ($CH_2$SH) ; 57,93 (CH) ; 60,85 ($CH_2$CO), 175,98 et 177,04 (2*CO).

ES-MS (m/z) : [M + H$^+$]$^+$ = 498,1.

**Exemple 5 :** Synthèse du composé 5: 2-(2-bromoacétamido)-3-(triétylthio)propanoate d'éthyle

Le composé 5 est synthétisé suivant la procédure décrite dans la littérature *(*Synthesis, 2003 (11), 1699-1704).

A une température de 0°C, une solution de bromure de bromoacétyle (0,235 mL, 2,69 mmol) dans 1 mL de dichlorométhane ($CH_2Cl_2$) est additionnée à un mélange de HCysC($C_6H_5$)$_3$O$C_2H_5$ (0,996 g, 2,54 mmol) dans 10 mL de dichlorométhane ($CH_2Cl_2$) et 10 mL d'une solution aqueuse à 10% de bicarbonate de potassium ($KHCO_3$).

Le mélange réactionnel est ensuite agité pendant 1 h à une température de 0°C.

Après décantation, la phase aqueuse est extraite avec 3 x 16 mL de dichlorométhane ($CH_2Cl_2$).

La phase organique est séchée avec du sulfate de sodium ($Na_2SO_4$), puis concentrée sous vide. Le composé 5 (1,29 g, rendement = 99%), de couleur jaune, est ensuite utilisé sans purification supplémentaire.

RMN $^1$H ($CD_3CN$, 400 MHz, 298 K) : δ = 1,29 (t, *J* = 3,6 Hz, 3H, $CH_3$) ; 2,62 et 2,74 (ABX, $J_{AX}$ = 4,8 Hz, $J_{BX}$ = 6,0 Hz, $J_{AB}$ = 12,4 Hz, 2H, $CH_2$SC($C_6H_5$)$_3$) ; 3,86 (s, 2H, Br$CH_2$CO) ; 4,23 (q, *J* = 7,0 Hz, 2H, $CH_2CH_3$) ; 4,56 (td, *J* = 2,4 et 7,2 Hz, 1H, CH) ; 6,95 (d, *J*= 8,0 Hz, 1H, NH) ; 7,24-7,35 (m, 10H, SC($C_6H_5$)$_3$) ; 7,43 (d, *J* = 7,6 Hz, 5H, SC($C_6H_5$)$_3$).

**Exemple 6** : Synthèse du composé 6 : NOTA(CysC($C_6H_5$)$_3$O$C_2H_5$)$_3$

Du trihydrochlorure de 1,4,7-triazacyclononane (0,423 g, 1,77 mmol) et du carbonate de potassium ($K_2CO_3$) (1,56 g, 11,31 mmol) sont ajoutés successivement à une solution du composé 5 (2,90 g, 5,66 mmol) dans 60 mL d'acétonitrile anhydre ($CH_3CN$).

Le mélange réactionnel est ensuite agité pendant 16 h à température ambiante, sous argon. Après évaporation du solvant, le résidu est dissout dans un mélange de 180 mL d'acétate d'éthyle ($CH_3COOC_2H_5$) et 100 mL d'eau.

La phase organique est lavée avec 2 x 70 mL d'eau et 70 mL d'une solution saturée de chlorure de sodium (NaCl), séchée avec du sulfate de sodium ($Na_2SO_4$), puis concentrée sous pression réduite (20 mbars).

Le produit résultant (2,66 g) est purifié par chromatographie sur gel de silice (40 mL, éluant : $CH_2Cl_2$/éthanol gradient allant de 100/0 à 96/4), pour donner un composé 6 (1,99 g, rendement 77%) sous forme de poudre blanche.

RMN $^1$H ($CD_3CN$, 400 MHz, 298 K) : δ = 1,21 (t, *J*= 7,0 Hz, 19H, $CH_3$) ; 2,58 et 2,67 (ABX, $J_{AX}$ = 4,9 Hz, $J_{BX}$ = 5,5 Hz, $J_{AB}$ = 12,1 Hz, 6H, $CH_2$S) ; 2,82 (s, 12H, N-$CH_2$-$CH_2$-N) ; 3,11 (s, 6H, $CH_2$CO) ; 4,14 (q, *J* = 7,0 Hz, 6H, $CH_2$-$CH_3$) ; 4,32-4,37 (m, 3H, CH), 7,17-7,27 (m, 30H, SC($C_6H_5$)$_3$) ; 7,33 (d, *J*= 7,4 Hz, 15H, SC($C_6H_5$)$_3$) ; 7,50 (d, *J* = 8,2 Hz, 3H, NH). RMN $^{13}$C ($CD_3CN$, 100 MHz, 298 K) : δ = 14,41 ($CH_3$) ; 34,20 ($CH_2$SC) ; 51,10 (CH) ; 57,12 et 61,98 (N-$CH_2$-$CH_2$-N) ; 63,01 ($CH_2CH_3$) ; 66,93 ($CH_2$CO), 128,25-129,71 (($C_6H_5$)$_3$) ; 144,53 (C($C_6H_5$)$_3$) ; 171,08 et 170,57 (2*CO).

ES-MS : m/z : [M + H$^+$]$^+$ = 1423,42 et [M + Cl$^-$]$^-$ = 1457,08.

Analyse élémentaire calculée (%) pour $C_{84}H_{90}N_6O_9S_3$, $H_2O$ (1441,85 g/mol) : C, 69,97 ; H, 6,43 ; N, 5,83 ; S, 6,67 ; trouvée : C, 69,75 ; H, 6,36 ; N, 5,77 ; S, 7,02.

**Exemple 7 :** Synthèse du composé 7 : NOTA(CysO$C_2H_5$)$_3$

De l'acide trifluoroacétique (1,25 mL, 16,85 mmol) et du triéthylsilane (0,325 mL, 2,02 mmol) sont successivement additionnés au composé 6 (0,480 g, 0,337 mmol) dans 17 mL de dichlorométhane ($CH_2Cl_2$), sous argon.

Après 30 minutes d'agitation à température ambiante, le mélange est évaporé.

Le produit résultant (703 mg) est ensuite purifié par HPLC ($t_R$ = 15,6 minutes (gradient linéaire 80/20 à 0/100, A/B en 15 minutes)).

Le composé 7 obtenu est un solide huileux (0,199 g, rendement = 84%).

RMN $^1$H ($CD_3CN$, 400 MHz, 298 K) : δ = 1,26 (t, *J* = 7,3 Hz, 9H, $CH_3$) ; 2,85-3,06 (m, 18H, $CH_2$SH et N-$CH_2$-$CH_2$-N); 3,68 (s, 6H, $CH_2$CO) ; 4,13-4,24 (m, 6H, $CH_2$-$CH_3$) ; 4,67 (td, *J* = 4,4 et 7,3 Hz, 3H, CH) ; 7,45 (d, *J* = 8,1 Hz, 3H, NH).

RMN $^{13}$C ($CD_3CN$, 100 MHz, 298 K) : δ = 14,99 ($CH_3$) ; 27,31 ($CH_2$SH) ; 50,41 et 51,33 (N-$CH_2$-$CH_2$-N) ; 56,29 (CH) ; 58,93 ($CH_2$CO) ; 63,13 ($CH_2CH_3$) ; 170,49 et 171,38 (2*CO).

ES-MS (m/z) : [M + H]$^+$= 697,6.

**Exemple 8** : Synthèse du composé intermédiaire 8 (précurseur des composés de formule (II) de l'invention)

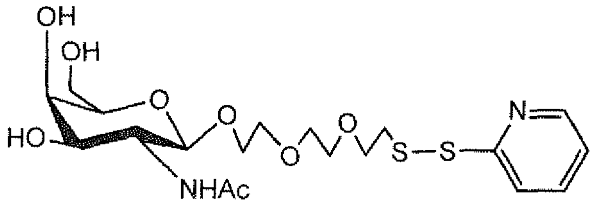

**intermédiaire 8**

Le schéma de synthèse du composé intermédiaire 8 est le suivant :

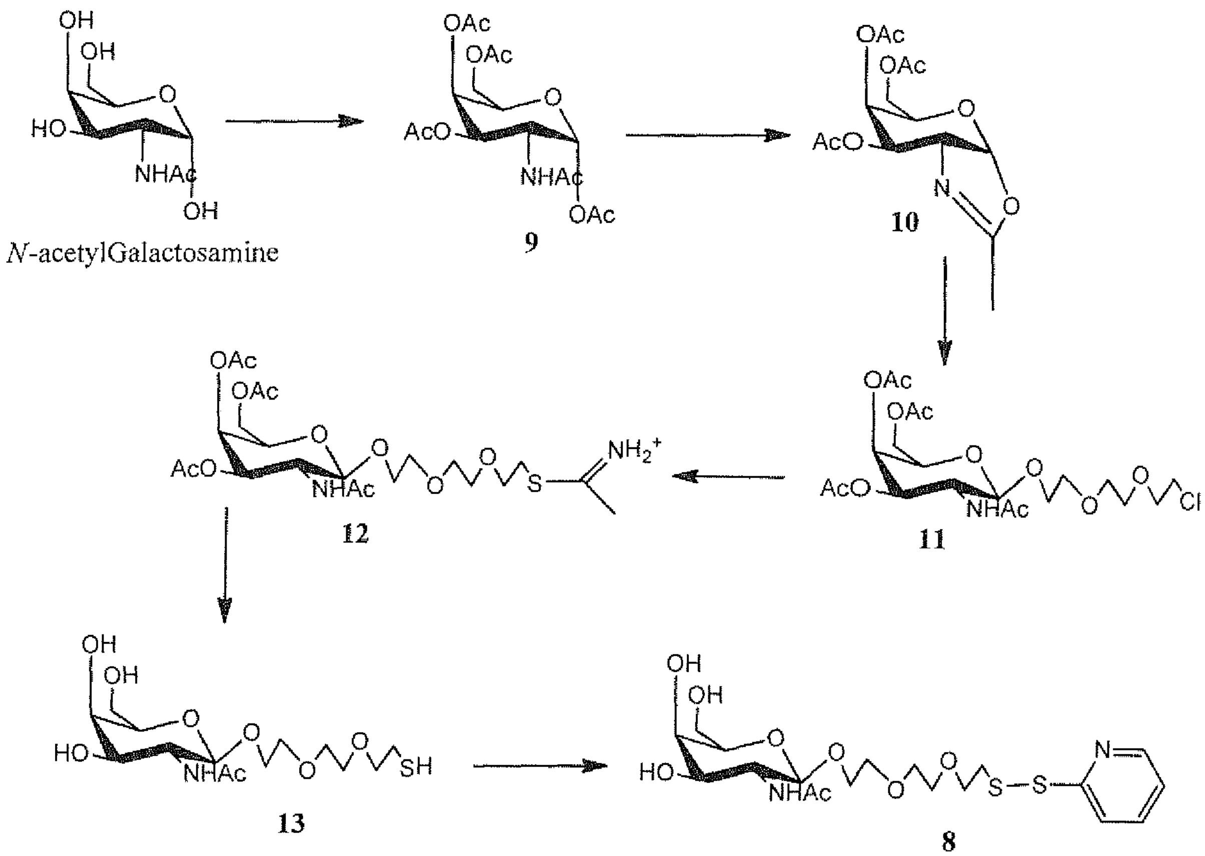

*N*-acetylGalactosamine 9 10 11 12 13 8

**Synthèse du composé 9 :** Péracétyl *D*-Galactosamine *(Bioconj. Chem,* 2006 (17), 1537-1544).

La *N*-acetyl-*D*-galactosamine (1,08 g, 4,87 mmol) est dissoute dans 4,1 mL d'anhydride acétique et 6,3 mL de pyridine. Le mélange réactionnel est agité sous argon pendant 25 h. Après évaporation du solvant, le résidu est redissout dans 200 mL d'acétate d'éthyle. La phase organique est lavée avec une solution aqueuse d'acide citrique à 10% (3 x 100 mL), puis avec une solution saturée de $NaHCO_3$ (1 x 100 mL), et avec de l'eau (1 x 100 mL), puis séchée sur $Na_2SO_4$ et concentrée sous pression réduite. Le produit brut 9 obtenu (1,80 g, 95%) est utilisé sans purification supplémentaire.

RMN $^1$H ($CDCl_3$, 500 MHz, 298 K) : δ = 1,95 (s, 3H, $CH_3$) ; 2,04 (s, 6H, $CH_3$) ; 2,18 (2*s, 6H, $CH_3$) ; 4,07, 4,12 (ABX, $J_{BX}$ = 6,6 , $J_{AX}$ = 6,9, $J_{AB}$ = 11,2, 2H, H-6) ; 4,21-4,23 (m, 1H, H-5); 4,74 (ddd, *J* = 3,6, 9,2, 11,5, 1H, H-2) ; 5,23 (dd, $^3J$ = 3,2, 11,5, 1H, H-3) ; 5,37 (d, $^3J$ = 9,2, 1H, NH) ; 5,43 (d, $^3J$ = 2,1 ,1H, H-4) ; 6,22 (d, $^3J$ = 3,6, 1H, H-1).

RMN $^{13}$C ($CDCl_3$, 100 MHz, 298 K) : δ = 21,00 ($CH_3$*2) ; 21,28 ($CH_3$*2) ; 23,53 ($CH_3$) ; 47,33 (CH, C-2) ; 61,60 ($CH_2$, C-6) ; 67,01 (CH, C-4) ; 68,15 (CH, C-3) ; 68,87 (CH, C-5) ; 91,69 (CH, C-1) ; 169,07 (CO) ; 170,30 (CO) ; 170,522 (CO) ; 170,68 (CO) ; 171,53 (CO).

ES-MS (m/z) : $[M + Na^+]^+$ = 412,2.

**Synthèse du composé 10** *(Glycoconjugate Journal,* 2004 (21), 227-241) :

Le composé 9 (1,8 g, 4,63 mmol) est dissout dans du dichlorométhane anhydre (21 mL) sur tamis moléculaire (4 Å). Du triflate de triméthylsilyle (2,85 mL, 15,74 mmol) est ajouté sous argon à température ambiante. Après agitation à 50°C pendant 15 h, de la triéthylamine (1,2 mL, 16,33 mmol) est ajoutée à 0°C. Le mélange réactionnel est ensuite dilué dans du dichlorométhane (200 mL). Cette phase organique est alors lavée avec une solution saturée de $NaHCO_3$ (1 x 100 mL), puis avec de l'eau (1 x 100 mL), et enfin séchée sur $Na_2SO_4$ et concentrée sous pression réduite. Le produit brut 10 obtenu (2,1 g) est utilisé sans purification supplémentaire.
RMN $^1H$ ($CDCl_3$, 500 MHz, 298 K) : δ = 2,06 (s, 3H, $CH_3$) ; 2,07 (s, 6H, $CH_3$) ; 2,12 (s, 3H, $CH_3$) ; 3,98-4,02 (m, 1H, H-2) ; 4,11, 4,20 (ABX, $J_{AX}$ = 5,8, $J_{BX}$ = 7,0, $J_{AB}$ = 11,2, 2H, H-6) ; 4,23-4,27 (m, 1H, H-2) ; 4,91 (dd, $^3J$ = 3,3, 7,4, 1H, H-3) ; 5,46 (t, $^3J$ = 3,0, 1H, H-4) ; 5,99 (d, $^3J$ = 6.8, 1H, H-1).
RMN $^{13}C$ ($CDCl_3$, 100 MHz, 298 K) : δ = 20,73-21,01 ($COCH_3$*3 et $CH_3$) ; 61,73 ($CH_2$, $C_6$) ; 63,73 (CH, $C_2$) ; 65,46 (CH, $C_4$) ; 69,70 (CH, $C_5$) ; 72,00 (CH, $C_3$) ; 101,68 (CH, $C_1$) ; 169,87-170,32 (NCO et CO*3).
ES-MS (m/z) : $[M + H^+]^+$ = 330,2 ; $[M + Na^+]^+$ = 352,2

**Synthèse du composé 11** *(Glycoconjugate Journal,* 2004 (21), 227-241) :

Le composé 10 (4,63 mmol) est dissout dans du dichlorométhane anhydre (25 mL) sur tamis moléculaire (4 Å) et du 2-(2-(2-chloroéthoxy)éthoxy)éthanol (1 mL, 6.88 mmol) est ajouté sous argon à température ambiante. Après agitation pendant 30 minutes, du triflate de triméthylsilyle (0,57 ml, 1,713 mmol) est ajouté. Après agitation du mélange réactionnel pendant une nuit à température ambiante, de la triéthylamine (0,46 mL, 3,33 mmol) est ajoutée à 0°C. Le mélange est alors filtré, puis dilué dans du dichlorométhane (200 mL) et les phases organiques sont lavées avec une solution saturée de $NaHCO_3$ (1 x 100 mL), puis avec de l'eau (1 x 100 mL), séchée sur $Na_2SO_4$ et enfin concentrée sous pression réduite. Le produit brut 11 obtenu (2,6 g) est purifié par chromatographie sur gel de silice (éluant : AcOEt/AcOH/EtOH : 8/0,2/0,2) pour donner le composé 11 (1,0 g, 43 %).
RMN $^1H$ ($CDCl_3$, 500 MHz, 298 K) : δ = 1,99 (s, 6H, $CH_3$*2) ; 2,05 (s, 3H, $CH_3$) ; 2,16 (s, 3H, $CH_3$) ; 3,63-3,91 (m, 13H, $CH_2$ et H-5) ; 4,12, 4,17 (ABX, $J_{AX}$ = 6,5, $J_{BX}$ = *6,9,* $J_{AB}$ = 11,2, 2H, H-6) ; 4,27 (ddd, $^3J$ = 2,0, 9,1, 11,1, 1H, H-2) ; 4,80 (d, *J* = 8,6, 1H, NH) ; 5,00 (dd, $^3J$ = 3,4, 11,2, 1H, H-3), 5,32-5,33 (m, 1H, H-4) ; 6,22 (d, $^3J$ = 9,4, 1H, H-1).
RMN $^{13}C$ ($CDCl_3$, 100 MHz, 298 K) : δ = 20,70 ($NHCOCH_3$) ; 20,81 (3*$COCH_3$) ; 43,08 ($CH_2Cl$) ; 50,73 (CH, $C_2$) ; 61,75 ($CH_2$, $C_6$) ; 66,85 (CH, $C_4$) ; 68,70 ($CH_2$) ; 70,47, 70,77, 70,84, 71,255, 71,493 ($CH_2$*4 et CH, $C_3$) ; 72,05 (CH, $C_5$) ; 102,61 (CH, $C_1$) ; 170,53, 170,69, 170,76, 170,84 (CO*4)
ES-MS (m/z) : $[M + Na^+]^+$ = 520,3

**Synthèse du composé 8 :**

De la thiourée (0,139 g, 1,820 mmol) est ajoutée à une solution de composé 11 (0,413 g, 0,829 mmol) dans de l'eau (6 mL). Le mélange réactionnel est agité pendant 24 h à reflux sous argon. La solution obtenue contenant le composé intermédiaire 12 est utilisée sans purification supplémentaire.
RMN $^1H$ du composé 12 ($D_2O$, 400 MHz, 298 K) : δ = 2,00 (s, 3H, $CH_3$) ; 2,02 (s, 3H, $CH_3$) ; 2,10 (s, 3H, $OCH_3$) ; 2,23 (s, 3H, $OCH_3$) ; 3,37 (t, *J*= 4,9, 2H, $CH_2S$) ; 3,72-4,25 (m, 15 H , H-2, H-4, H-5, H-6, 5 x $CH_2$) ; 5,11(d, *J* = 2,7, *J* = 11,1, 1H, H-3) ; 5,41(m, 1H, H-1).
La solution précédente contenant le composé intermédiaire 12 est diluée dans du méthanol pour obtenir 20 mL d'un mélange MeOH/eau (v/v : 2/1), et est maintenue sous argon. Le pH de cette solution est maintenu à 9,6 pendant 9 h par des ajouts d'une solution aqueuse de soude 1 M. Le pH est ensuite ramené à pH = 7 par addition d'une solution aqueuse de HCl 1 M, puis concentrée sous pression réduite. Le produit brut 13 est ensuite utilisé sans purification supplémentaire.
RMN $^1H$ du composé 13 ($D_2O$, 400 MHz, 298 K) : δ = 1,82 (t, *J* = 8,0, 1H, SH) ; 2,04 (s, 3H, $NHCH_3$) ; 2,62 (t, *J* = 7,0, 2H, $CH_2$-SH) ; 3,52 (t, J = 5,5, 2H, $CH_2$) ; 3,55-3,93 (m, 15H, 5 x $CH_2$ et H-2, H-3, H-4, H-6) ; 3,96-4,00 (m, 1H, CH, H-5) ; 4,44 (d, *J* = 8,5, 1H, CH, H-1).
Le composé brut 13 est redissout dans du méthanol anhydre (22 mL) et de l'aldrithiol (0,552 g, 2,5 mmol) est ajoutée à la solution obtenue. Le mélange réactionnel est agité pendant 1 h à température ambiante sous argon, puis concentré sous pression réduite. Le produit résultant est ensuite purifié par HPLC. Le composé intermédiaire 8 obtenu est un solide blanc (0,075 g, 19%).
RMN $^1H$ du composé 8 ($D_2O$, 400 MHz, 298 K) : δ = 2,03 (s, 3H, $CH_3$) ; 3,06 (t, *J* = 5,8, 2H, $CH_2SH$) ; 3,57-4,02 (m, 15H, $CH_2$ et CH) ; 4,51 (d, *J* = 8,4, 1H, CH, H-1) ; 7,33 (t, *J* = 6,0, 1H, CH, $H_{aro}$) ; 7,89 (m, 2H, CH, $H_{aro}$) ; 8,42 (d, *J* = 4,8, 1H, CH, $H_{aro}$).
RMN $^{13}C$ du composé 8 ($D_2O$, 100 MHz, 298 K) : δ = 23,10 ($CH_3$) ; 39,87 ($CH_2$S-S) ; 54,35 (CH, C-2) ; 62,55 ($CH_2$, C-6) ; 69,69-71,64 (CH, C-4, $CH_2$*5) ; 73,60 (CH, C-3) ; 76,78 (CH, C-5) ; 103,17 (CH, C-1) ; 121,23-122,40 ($CH_{pyr}$) ;

161,77 ($\underline{C}_{pyr}$); 174,16 ($\underline{CO}$NH).
ES-MS (m/z) : $[M + Na^+]^+ = 501,3$ ; $[M + H^+]^+ = 479,3$.

Exemple 9 : Synthèse du composé ($II_{a1}$)

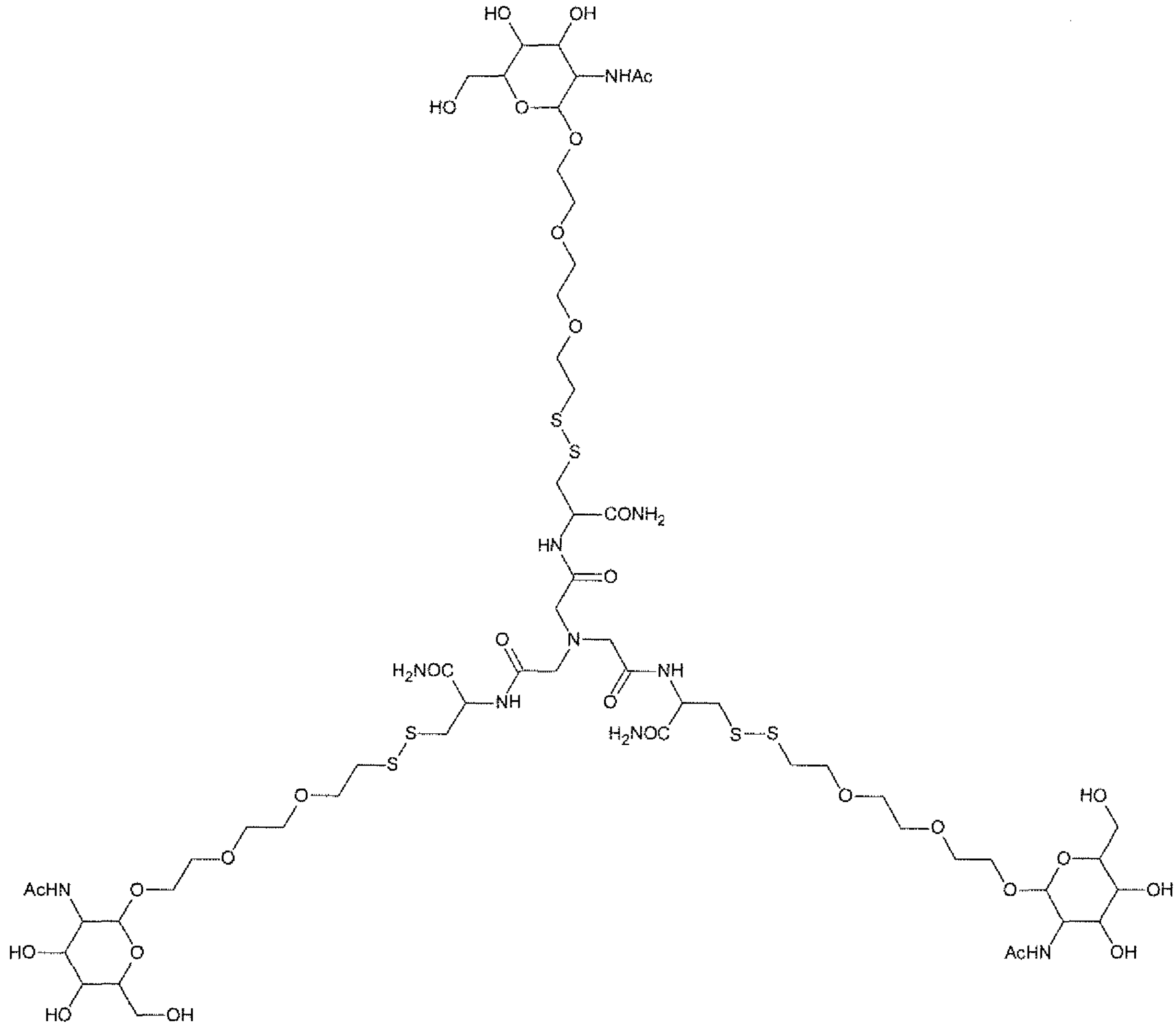

($II_{a1}$)

Une solution du composé 8 (0,045 g, 0,0941 mmol) dans du DMF (5 mL) est ajoutée à une solution du composé 4 (0,0137 g, 0,0275 mmol) dans du DMF (5 mL). Le mélange réactionnel est agité à température ambiante pendant 5 h, puis concentré sous pression réduite. Le produit résultant est ensuite purifié par HPLC. Le composé ($II_{a1}$) obtenu est un solide blanc (0,0157 g, 35 %).

RMN $^1H$ ($D_2O$, 500 MHz, 298 K) : $\delta$ = 2,04 (s, 9H, $CH_3NH$), 2,96 (t, $J$ = 6,0, 6H, $CH_2S$-S) ; 3,01 (dd, J = 10,1, 3H, $CHCH_2S$) ; 3,28 (dd, $J$ = 4,0, 3H, $CHCH_2S$) ; 3,56 (s, 6H, $CH_2CO$) ; 3,70-3,73 (m, 33H, $CH_2$ et $\underline{CH}$, H-3) ; 3,77 et 3,83 (ABX, $J_{AX}$ = 6,4, $J_{BX}$ = 6,8, $J_{AB}$ = 11,7, 6H, $CH_2$, H-6) ; 3,88-3,93 (m, 6H, C$\underline{H}$, H-4, H-2) ; 3,9-4,02 (m, 3H, C$\underline{H}$, H-5) ; 4,50 (d, J = 8,5, 3H, C$\underline{H}$, H-1) ; 4,72-4,75 (m, 3H, C$\underline{H}$).

RMN $^{13}C$ ($D_2O$, 100 MHz, 298 K) : $\delta$ = 22,27 ($\underline{C}H_3NH$*3) ; 37,19 ($\underline{C}H_2S$-S*3) ; 52,40 ($\underline{C}$H*3, C-4) ; 60,97 ($\underline{C}H_2CO$*3); 67,83-75,14 ($\underline{C}$H*9 : C-2, C-3, C-5, $\underline{C}H_2$*42, C-6) ; 101,53 ($\underline{C}$H*3, C-1) ; 173,20, 174,67, 174,68 ($\underline{CO}$*3, $\underline{CO}NH_2$*3, $\underline{CO}$NHAc*3).

ES-MS (m/z) : $[M + Na^+]^+ = 1621,3$.

**Exemple 10** : Synthèse d'un composé répondant à la formule ($II_{a1'}$) suivante :

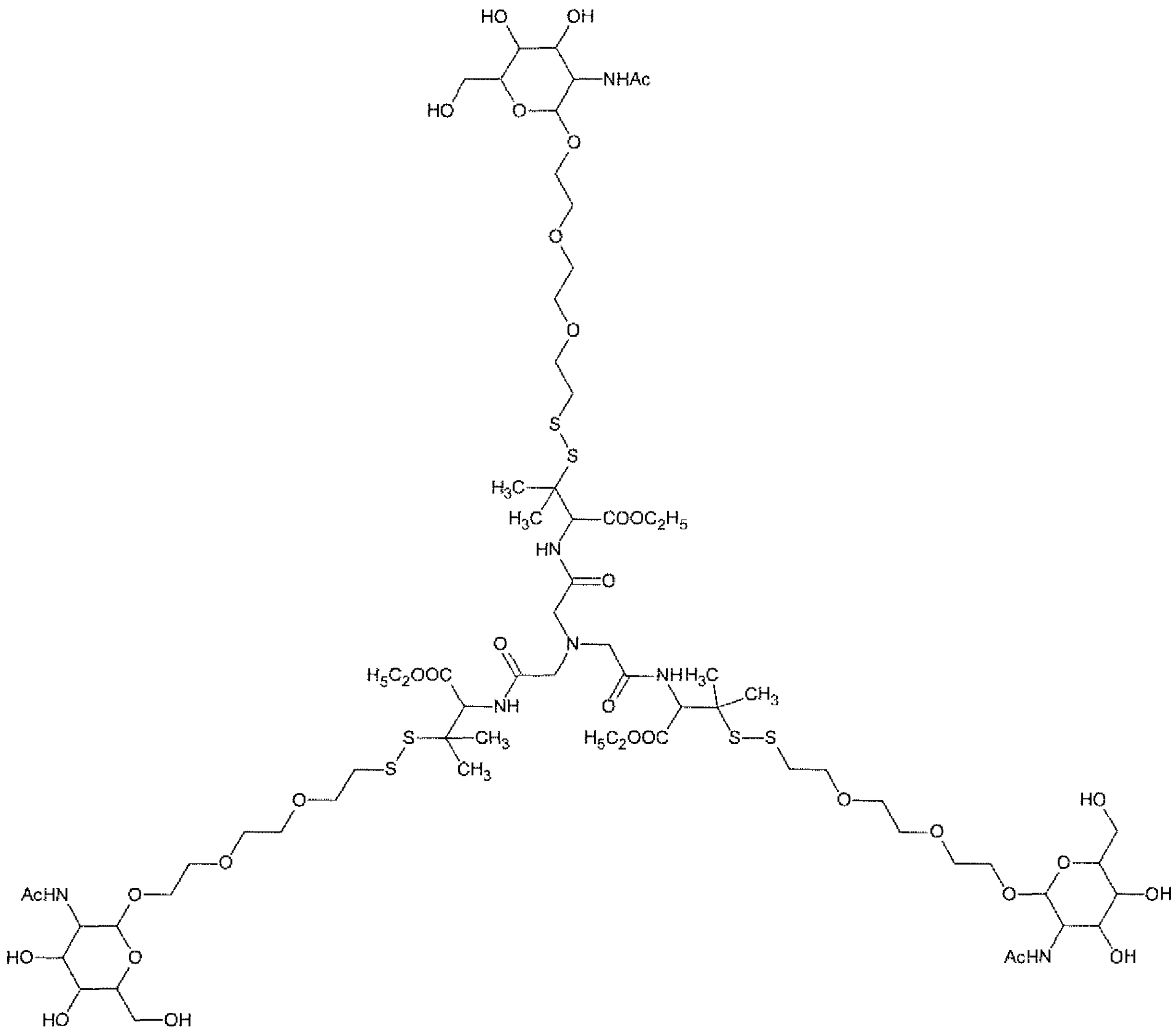

($II_{a1'}$)

La synthèse du composé ($II_{a1'}$) est réalisée en suivant le même protocole que celui décrit ci-dessus pour le composé ($II_{a1}$), mais en faisant réagir le composé intermédiaire 8 sur le composé 14 suivant :

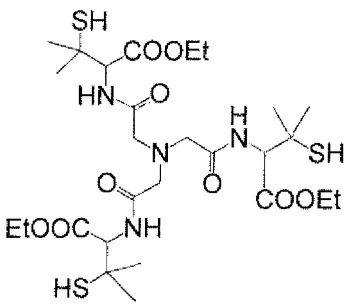

**composé 14**

Le composé 14 est obtenu de la même manière que le composé 3, mais en utilisant de la pénicillamine à la place de la cystéine comme produit de départ.

**Exemple 11** : Synthèse du composé ($III_1$)

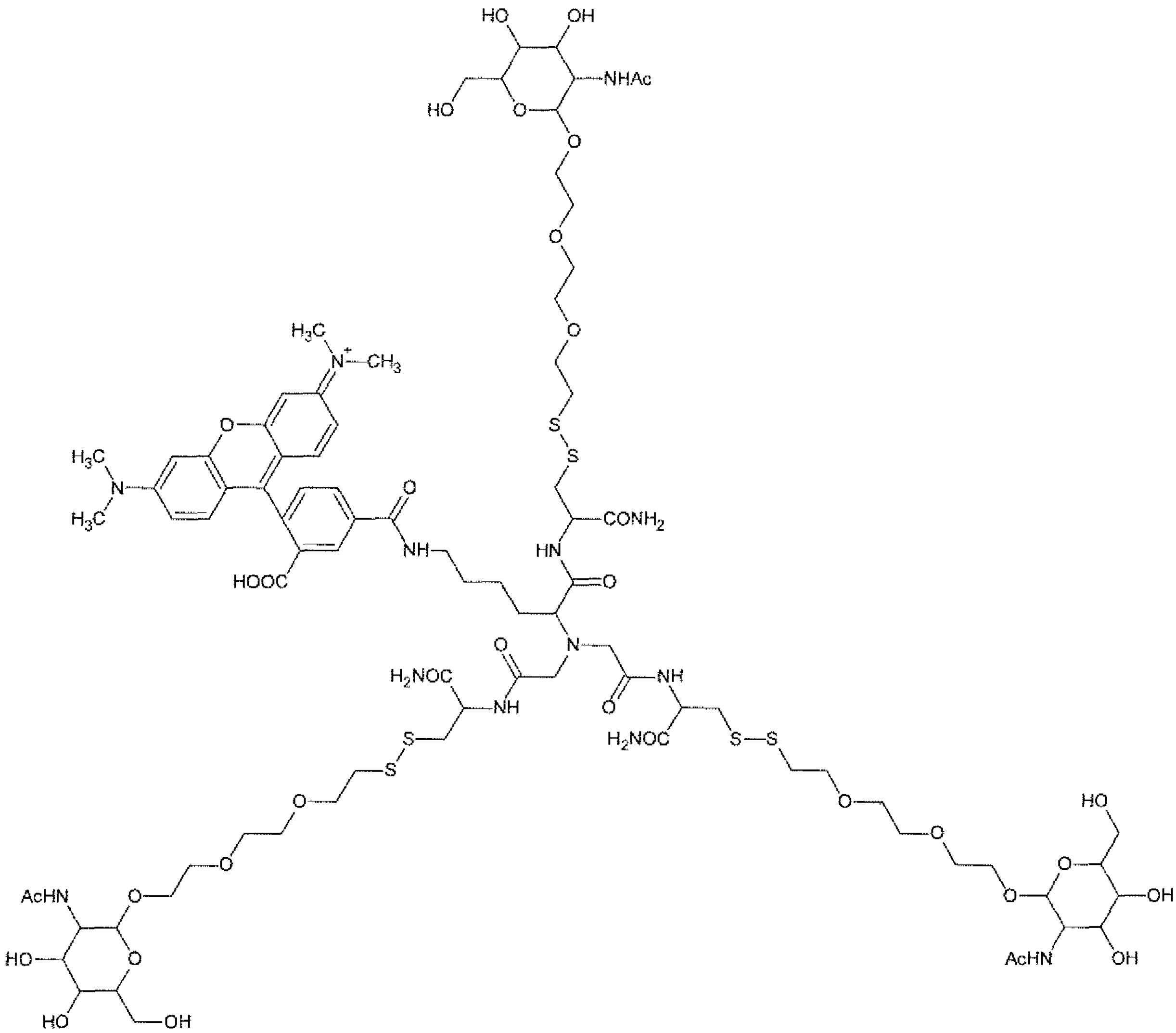

La synthèse du composé ($III_1$) est réalisée selon le schéma réactionnel détaillé ci-dessous, à partir de la $N_\alpha$,$N_\alpha$-bis(carboxyméthyl)-L-Lysine commerciale :

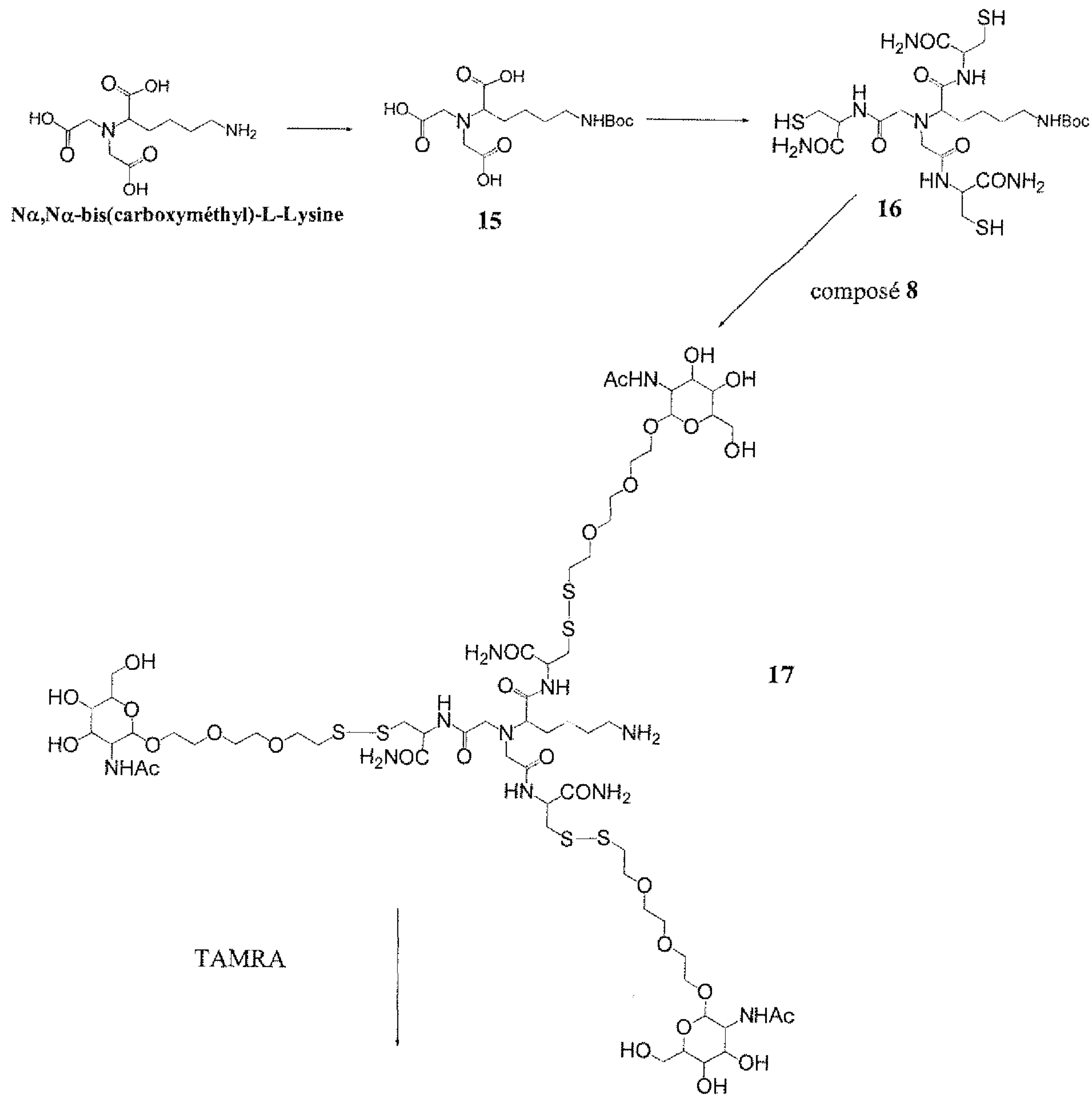


Nα,Nα-bis(carboxyméthyl)-L-Lysine

15

16

17

**Composé $III_1$**

**Synthèse du composé 15 :**

A une solution de $N_\alpha$,$N_\alpha$-bis(carboxyméthyl)-L-Lysine (0,100 g, 0,383 mmol) dans un mélange acétonitrile/eau (3 mL, v/v 1/1) sont ajoutés du di-*tert*-butyldicarbonate (0,251 g, 1,15 mmol) et du $NaHCO_3$ (0,165, 1,96 mmol) à 0°C. Le mélange réactionnel est agité à température ambiante pendant 24 h, puis concentré sous pression réduite. Le résidu est ensuite redissout dans de l'eau (15 mL) et extrait avec du diéthyléther (6 x 10 mL) et de l'acétate d'éthyle (6 x 10 mL). Les phases aqueuses sont acidifiées avec une solution aqueuse de $KHSO_4$ à 5% jusqu'à pH = 2,0. La phase aqueuse est extraite par de l'acétate d'éthyle (6 x 10 mL). Les phases organiques sont séchées sur $Na_2SO_4$ et concentrées sous pression réduite. Le produit brut obtenu (0,157 g, 64%) est utilisé sans purification supplémentaire.
RMN $^1H$ ($D_2O$ 400 MHz, 298 K) : $\delta$ = 1,44 (s, 9H, $CH_3$) ; 1,47-1,58 (m, 4H, $CH_2$) ; 1,83-2,02 (m, 2H, $CH_2$) ; 3,10 (t, $J$ = 5,8, 2H, $CH_2$) ; 3,97-4,01 (m, 1H, CHCO) ; 4,01 (s, 4H, $CH_2CO$). ES-MS (m/z) : $[M - H^+]^-$ = 361,3.

**Synthèse du composé 16 :**

Le composé 15 (0,157 g, 0,433mmol) en solution dans du DMF (12 mL) est ajouté à une solution de $CysC(C_6H_5)_3(NH_2)$ (0,485 g, 1,338 mmol) dans 10 mL de DMF. Le mélange est ensuite refroidi à 0°C, et du N-éthyl-N'-(3-diméthylaminopropyl)carbodiimide (0,254 g, 1,64 mmol) et de l'hydrate de 1-hydroxybenzotriazole (0,180 g, 1,33 mmol) sont ajoutés successivement. Le mélange réactionnel est ensuite agité à température ambiante pendant 16 h sous argon. Après évaporation du solvant, le résidu est ensuite lavé avec 25 mL d'eau, puis filtré. Le solide est ensuite dissout dans 50 mL de dichlorométhane ($CH_2Cl_2$). La phase organique est alors lavée avec 3 x 25 mL d'eau et 1 x 25 mL d'une solution saturée de chlorure de sodium (NaCl). La phase organique est ensuite séchée avec du sulfate de sodium ($Na_2SO_4$), puis concentrée sous pression réduite (20 mbars) pour donner un intermédiaire tritylé (0,584 g).

Le composé tritylé obtenu est redissout dans du dichlorométhane anhydre (3,5 mL). De l'acide trifluoroacétique (TFA) (3,1 mL, 41,4 mmol) et du triéthylsilane ($Et_3SiH$) (0,412 mL, 2,53 mmol) sont ajoutés. Après 1 h d'agitation à température ambiante, le mélange est évaporé. Le produit résultant est ensuite purifié par HPLC. Le composé 16 obtenu est un solide blanc (0,171 g, 72%).

RMN $^1$H ($D_2O$, 500 MHz, 298 K) : $\delta$ = 1,31-1,50 (m, 2H, $CH_2$lys) ; 1,64-1,77 (m, 4H, 2 x $CH_2$lys) ; 2,83-3,99 (m, 8H, 3 x $CH_{2\beta}$ et $CH_2$lys) 3,45-3,47 (m, 1 H, CHCO) ; 3,59 (AB, $J_{AB}$ = 17,2, 4H, $CH_2$CO) ; 4,47 (dd, $J$ = 7,9, 1H, CH$\alpha$) ; 4,51 (dd, $J$ = 7,3, 2H, CH$\alpha$).

ES-MS (m/z) : $[M + H^+]^+$ = 569,3.

**Synthèse du composé 17 :**

Le composé 17 est obtenu selon le même mode opératoire que celui réalisé pour la synthèse du composé ($II_{a1}$), avec un rendement de 20%.

RMN $^1$H ($D_2O$, 500 MHz, 298 K) : $\delta$ = 1,32-1,47 (m, 2 H, $CH_2$lys) ; 1,57-1,76 (m, 4H, $CH_2$-lys) ; 1,95 (s, 9H, $CH_3$NH) ; 2,88 (t, $J$ = 6,0, 6H, $CH_2$-S) ; 2,91-3,49 (m, 9H, $CH_2$lys, 3 x $CH_{2\beta}$, CHCO) ; 3,59-3,77 (m, 39H, 5 x $CH_2$, H-6, H-3) ; 3,81-3,86 (m, 6H, H-4, H-2) ; 3,92, 3,95 (ABX, $J_{AX}$ = 2,7, $J_{BX}$ = 2,8, $J_{AB}$ = 5,5, 3 H, H-5) ; 4,43 (d, $J$ = 8,5, 4 H, H-1) ; 4,41-4,43 (m, 1H, H-1) ; 4,65-4,77 (s, 3H, CH$\alpha$).

ES-MS (m/z) : $[M + H^+]^+$ = 1670,5.

**Synthèse du composé de formule ($III_1$) :**

Une solution de carboxytétraméthylrhodamine *N*-succinimidyl ester (TAMRA) (0,0081 g, 0,0154 mmol) dans du DMF (0,7 mL) est ajoutée à une solution du composé 17 (0,0114 g, 0,0068 mmol) dans du DMF (0,8 mL). Le pH est ajusté à 8-9 avec de la N,N-diisopropyléthylamine (DIPEA) (0,002 mL, 0,0111 mmol). Le mélange réactionnel est agité pendant 4 h, puis concentré sous pression réduite. Le produit brut obtenu est purifié par HPLC (éluant eau/acétonitrile). Le produit est obtenu sous la forme d'un solide rose (m = 0,0012 g, 8%).

ES-MS (m/z) : $[M + Na^+]^{2+} / 2$ = 1053,6.

**2/ <u>Caractérisation des complexes du cuivre Cu(I)</u>**

**2/ 1- Spectroscopie UV-visible et dichroïsme circulaire**

La formation des complexes du Cu(I) a été suivie par spectroscopie UV. La bande de transfert de charge thiolate $\rightarrow$ Cu(I) apparaît clairement autour de 260 nm. Cette bande croît jusqu'à 2 équivalents pour les deux composés NTA($CysOC_2H_5$)$_3$ et NOTA($CysOC_2H_5$)$_3$. Les complexes du Cu(I) obtenus ont donc une stoechiométrie globale de 2:1 (Cu:L) pour ces ligands (L) comportant trois cystéines.

La Figure 1 donne un exemple de dosage UV du NTA($CysOC_2H_5$)$_3$ (composé 3) par du $Cu(CH_3CN)PF_6$ (Cu(I)) en tampon phosphate à un pH de 7,4.

Le même type de dosage suivi par dichroïsme circulaire montre la formation successive de deux complexes entre 0 et 2 équivalents de Cu(I). Pour le NTA($CysOC_2H_5$)$_3$, le premier complexe formé ne possède pas de bande caractéristique de l'interaction Cu-Cu (au-delà de 300 nm), ce premier complexe est donc un composé mononucléaire. Le second complexe formé entre 1 et 2 équivalents possède une bande à 340 nm et est donc un composé polynucléaire du cuivre Cu(I).

Pour le NOTA($CysOC_2H_5$)$_3$, uniquement des complexes polynucléaires sont mis en évidence.

**<u>Mode opératoire</u> :**

Les fonctions thiols -SH des agents chélatants étant susceptibles de s'oxyder à l'air, toutes les solutions ont été préparées dans une boîte à gants sous atmosphère d'argon. Des solutions de ligands ont ensuite été préparées, avant chaque expérience, en utilisant une eau désoxygénée et purifiée par un système Millipore Milli-Q® contenant 20 mM d'une solution de tampon phosphate (pH = 7,4) et d'acétonitrile (v/v : 9/1).

La concentration finale de la solution de ligand a été déterminée par mesure de la concentration des fonctions

thiols libres dans le ligand, suivant la procédure d'Ellman décrite dans P. W. Riddles, R. L. Blakeley, B. Zemer, Methods Enzymol., 1983, 91, pp. 49-60. Cette méthode utilise de l'acide 5,5'-dithiobis-2-nitrobenzoïque (DNTB) comme indicateur, chaque groupement thiol libre présent dans le ligand conduisant à 1 équivalent de $TNB^{2-}$ ($\varepsilon^{412\ nm}$ ($TNB^{2-}$) = 14 150 $M^{-1}.cm^{-1}$, $\varepsilon^{412\ nm}$ étant le coefficient d'extinction molaire du $TNB^{2-}$ à 412 nm). Les concentrations de la solution de ligand sont comprises entre 30 et 100 $\mu$M.

Les solutions de cuivre Cu(I) ont été préparées en dissolvant une quantité appropriée de $Cu(CH_3CN)_4PF_6$ dans de l'acétonitrile désoxygénée. La concentration finale est déterminée par ajout d'un excès de disulfonate bathocuproïne de sodium ($Na_2BCS$) et par mesure de l'absorbance du $Cu(BCS)_2^{3-}$ ($\lambda_{max}$ = 483 nm, $\varepsilon$ = 13 300 $M^{-1}.cm^{-1}$).

Les spectres UV-visible ont été enregistrés avec un spectrophotomètre Varian Cary 50, et les titrages par dichroïsme circulaire avec un spectromètre Chirascan (Applied Photophysics®). 2,5 mL de la solution de ligand préparée est transférée dans une cellule UV de 1 cm de longueur fermé par un bouchon septum étanche. Des aliquotes correspondant à 0,1 équivalent de Cu(I) en solution dans de l'acétonitrile sont ajoutés dans la cellule UV via une seringue hermétique (Hamilton™), pour éviter une oxydation des fonctions thiols.

**2/2- Molécularité des complexes : coefficients de diffusion**

La formation des complexes a également été suivie par RMN $^1H$ à 500 MHz.

Pour le composé 3 $NTA(CysOC_2H_5)_3$ (noté ligand L), il apparait clairement que les complexes suivants sont formés :

- un complexe mononucléaire : L + Cu(I) $\rightarrow$ $CuL^{2-}$
- un complexe polynucléaire : $CuL^{2-}$ + Cu(I) $\rightarrow$ $(Cu_2L^-)_n$

Les coefficients de diffusion de ces complexes ont également été mesurés (cf. Tableau II). Ces coefficients de diffusion translationnels sont reliés à la masse moléculaire des composés et permettent d'évaluer la molécularité des complexes (P. Rousselot-Pailley, O. Sénèque, C. Lebrun, S. Crouzy, D. Boturyn, P. Dumy, M. Ferrand, P. Delangle, Inorg. Chem., 2006, 45, pp. 5510-5520). Ils indiquent que les complexes formés avec le composé 3 $NTA(CysOC_2H_5)_3$ sont bien le CuL puis le $Cu_6L_3$, tandis que l'espèce polymoléculaire $Cu_8L_4$ est observée pour le composé 7 NOTA($CysOC_2H_5)_3$.

**Tableau II** : Coefficients de diffusion D des complexes Cu(I)-composé 3 $NTA(CysOC_2H_5)_3$ et Cu(I)-composé 7 $NOTA(CysOC_2H_5)_3$

| D ($m^2s^{-1}$) x $10^{10}$ | L | CuL | $(Cu_2L^-)_n$ |
|---|---|---|---|
| Composé 3 $NTA(CysOC_2H_5)_3$ | 3,2 | 3,0 | 2,0 $\rightarrow$ n = 3 |
| Composé 7 $NOTA(CysOC_2H_5)_3$ | 3,0 | - | 1,8 $\rightarrow$ n = 4 |

**Mode opératoire :**

Les spectres RMN ont été enregistrés sur un spectromètre Bruker Avance sur 500 MHz, équipé d'une sonde indirecte proton de 5 mm munie de gradients 3 axes. Les mesures du coefficient de diffusion ont été réalisées en utilisant une séquence bipolaire (bipolar stimulated spin echo sequence) (A. Jershow, N. Müller, J. Magn. Reson., 1997, 125, pp. 372-375).

Les coefficients de diffusion ont été obtenus en utilisant la relation :

$$I(\delta, \Delta, g) = I_0 \exp[-\gamma^2 g^2 \delta^2 (\Delta^{-\delta/3}) D]$$

dans laquelle :

- I ($\delta$, $\Delta$, g) est l'intensité obtenue en présence des impulsions de gradient de force g,
- $I_0$ est l'intensité obtenue en l'absence des impulsions de pulsation,
- $\delta$ est la longueur de l'impulsion de gradient,
- $\Delta$ est le temps de diffusion, et
- $\gamma$ est le ratio gyromagnétique (pour les protons, $\gamma$ = 26,7520 $\times$ $10^7$ rad.$T^{-1}.s^{-1}$).

Les valeurs $\Delta$ et $\delta$ utilisées pour les mesures du coefficient de diffusion étaient respectivement de 100 ms et

de 2 ms.

Dans les expériences, g a été incrémenté de 2,95 à 41,2 $G.cm^{-1}$.

Les échantillons de ligands ont été préparés dans une solution tampon phosphate à 20 mM de pH = 7,4, préparés dans une solution de $D_2O$ et de $CD_3CN$ (v/v : 9/1), à une concentration de ~1 mM. Les aliquotes d'une solution de $Cu(CH_3CN)_4PF_6$ dans du $CD_3CN$ ont ensuite été ajoutés à l'échantillon de ligand.

**2/3- Constantes d'affinité**

L'affinité des agents chélatants synthétisés pour le Cu(I) est une donnée importante puisqu'elle permet de quantifier la capacité des agents chélatants à complexer cet ion.

Les constantes d'affinité ont été mesurées grâce à un compétiteur connu ayant une forte affinité pour le Cu(I), le disulfonate bathocuproïne (BCS), qui forment des complexes de Cu(I) de stabilité connue selon la réaction ci-dessous :

$$Cu(I) + 2\ BCS = Cu(BCS)_2 \qquad K = \frac{[Cu(BCS)_2]}{[Cu][BCS]^2} = 10^{19,8}$$

(P. Rousselot-Pailley, O. Sénèque, C. Lebrun, S. Crouzy, D. Boturyn, P. Dumy, M. Ferrand, P. Delangle, Inorg. Chem., 2006, 45, pp. 5510-5520 ; Z. Xiao, F. Loughlin, G. N. George, G. J. Howlett, A. G. Wedd, J. Am. Chem. Soc., 2004, 126, pp. 3081-3090).

La quantité de disulfonate bathocuproïne (BCS) ajoutée nécessaire pour déplacer 50% du Cu(I) complexé par ces ligands est une première indication de l'affinité pour le Cu(I). Plus ce pourcentage est élevé, plus l'affinité du ligand soufré pour le Cu(I) est forte. Ces données montrent immédiatement que les trois ligands étudiés peuvent être classés selon leur affinité croissante pour le Cu(I) :

Composé 3 $NTA(CysOH)_3$ < Composé 7 $NOTA(CysOC_2H_5)_3$ < Composé 3 $NTA(CysOC_2H_5)_3$ ~ Composé 5 $NTA(CysNH_2)_3$

Ces expériences de compétition ont permis de quantifier l'affinité de ces nouveaux agents chélatants pour le Cu(I) : les constantes apparentes de complexation du Cu(I) à pH = 7,4 dans un tampon phosphate à 20 mM, telles que définies ci-dessous, sont données au Tableau III.

$$K_{app} = \frac{[Cu]_{complexé}}{[Cu]_{libre}[L]_{libre}}$$

**Tableau III** : Résultats des expériences de compétition pour les différents ligands, dans une solution tampon phosphate à 20 mM de pH 7,4, à une température de 298 K

| | Composé 3 $NTA(CysOC_2H_5)_3$ | Composé 7 $NOTA(CysOC_2H_5)_3$ |
|---|---|---|
| Equivalents BCS* | 57 | 12 |
| $logK_{app}$ | 19 | 17,5 |
| * Nombre d'équivalents de BCS par rapport au Cu, nécessaire pour déplacer 50% du cuivre complexé par un ligand en partant des concentrations $[Cu]_0 = 0,9[L]_0$, dans un tampon phosphate à 20 mM de pH 7,4, à une température de 298 K. | | |

Il apparait clairement que :

- le composé 3 $NTA(CysC_2H_5)_3$ et le composé 5 $NTA(CysNH_2)_3$ ont une affinité pour le Cu(I) extrêmement élevée ($K_{app} = 10^{19}$),
- l'affinité est moins forte pour le composé comportant des fonctions acides, le composé 3 $NTA(CysOH)_3$, par rapport

au ligand neutre similaire représenté par le composé 3 NTA$(CysOC_2H_5)_3$.

**Mode opératoire :**

Les complexes du cuivre Cu(I) avec les ligands sont dosés par le disulfonate bathocuproïne (BCS) dans le but de mesurer leurs constantes d'affinité. Le complexe est préparé en ajoutant une solution d'acétonitrile ($CH_3CN$) contenant 0,5, 0,9 ou 1,8 équivalents de cuivre Cu(I) à la solution de ligand, dans une solution tampon phosphate de 20 mM de pH = 7,4 / acétonitrile (v/v : 9/1). La formation du complexe est ensuite réalisée par agitation du mélange pendant 10 minutes sous argon.

Des aliquotes d'une solution de disulfonate bathocuproïne (BCS) dans la même solution tampon sont ensuite ajoutés au complexe ligand-cuivre.

Les spectres UV-visible sont ensuite enregistrés, et la stabilité de l'absorbance est contrôlée avant l'addition des autres aliquotes.

**3/ Caractérisation des complexes d'autres ions métalliques**

D'une manière générale, la formation d'un complexe à partir d'un métal M et de n ligand L s'écrit :

$$M + nL \leftrightarrows [M(L)_n]$$

A cette réaction est associée une constante de complexation apparente log $K_{app}$, où :

$$K_{app} = [ML]/[M][L]_{tot}$$

dans laquelle :

- [ML] est la concentration en complexe,
- [M] est la concentration en métal, et
- $[L]_{tot}$ est la concentration en ligand libre (quelle que soit sa forme protonée).

Cette constante s'exprime de façon approximative en faisant apparaître les concentrations au lieu des activités des ions présents à l'équilibre, sans jamais faire apparaître les solides ni le solvant.

Les constantes de complexation apparentes de certains des agents chélatants connus sont reportées dans le tableau IV ci-dessous :

**Tableau IV :**

| Log $K_{app}$ à T = 298 K (à pH = 7,4) | EDTA | Trien | Pen | BAL |
|---|---|---|---|---|
| Ca(II) | 7,8 | - | - | - |
| Cu(I) | - | - | 8,3 | - |
| Cu(II) | 16,0 | 16,0 | - | - |
| Zn(II) | 13,7 | 7,9 | 5,8 | 9,0 |
| Cd(II) | 13,7 | 6,6 | 7,6 | - |
| Hg(II) | 18,7 | 20,6 | 14,9 | 21,2 |
| Pb(II) | 15,2 | 6,3 | 9,2 | - |
| Sélectivité Cu/Zn | 2,3 | 8,1 | 2,5 | - |
| Sélectivité Hg/Zn | 5 | 12,7 | 9,1 | 12,2 |

La sélectivité entre deux métaux M/M' correspond à la sélectivité du ligand pour le métal M par rapport à celle du métal M', cette sélectivité étant égale à :

$$\log (K_{app}(M)/K_{app}(M')) = \log K_{app}(M) - \log K_{app}(M')$$

Les agents chélatants présentés peuvent également avoir un intérêt pour la complexation de certains ions toxiques comme Hg(II), Cd(II) et Pb(II). Une étude approfondie a été réalisée dans le cas du composé 3 NTA($CysOC_2H_5$)$_3$ qui s'avère être le chélateur du Cu(I) le plus puissant. Quelques données disponibles pour le composé 7 NOTA($CysOC_2H_5$)$_3$ sont également présentées ci-dessous.

**3/1- Etude réalisée sur le composé 3 NTA($CysOC_2H_5$)$_3$**

Des dosages par UV visant à suivre l'apparition de la bande à transfert de charge $S^- \rightarrow M$ ont permis de montrer que les stoechiométries des complexes étaient 1:1 (M:L) pour Cd(II), Zn(II), Pb(II) et Hg(II). Seul le complexe de Hg(II) évolue ensuite vers un autre complexe impliquant vraisemblablement plusieurs ions métalliques ($Hg_3L_2$).

Les constantes d'affinité avec Pb(II) ont été déterminées par analyse des dosages des ligands par Pb(II) dans une solution tampon de Bis-Tris (2-bis(2-hydroxyéthyl)amino-2-(hydroxyméthyl)-1,3-propanediol) à 20 mM et à pH = 7, grâce au programme SPECFIT, qui utilise un algorithme de décomposition en valeur singulière et affine les données selon une analyse des moindre carrés, et suivant la procédure décrite dans l'article P. Rousselot-Pailley, O. Sénèque, C. Lebrun, S. Crouzy, D. Boturyn, P. Dumy, M. Ferrand, P. Delangle, Inorg. Chem., 2006, 45, pp. 5510-5520, en tenant compte de l'affinité du tampon pour Pb(II).

Les constantes d'affinité avec les ions Cd(II), Zn(II) et Ca(II) ont ensuite été déterminées en titrant le complexe de Pb(II) par un deuxième ion métallique suivant la réaction de compétition suivante :

$$PbL + M \rightarrow Pb + ML$$

Pour Hg(II), qui a une très forte affinité pour les ligands thiolates, nous avons réalisé une compétition avec l'EDTA de constante d'affinité connue (cf. Tableau IV) selon la réaction suivante :

$$HgL + EDTA \rightarrow L + HgEDTA$$

**Tableau V :** Constantes de stabilité apparentes des complexes M.NTA($CysOC_2H_5$)$_3^-$ à pH = 7, à une température de 298 K

| | log $K_{app}$ (à pH = 7) | $\lambda_{max}$ (nm) | $\varepsilon$ ($cm^{-1}M^{-1}$) |
|---|---|---|---|
| Ca(II) | < 3 | - | - |
| Zn(II) | 9,1 | 220 | 15 800 |
| Cd(II) | 10,6 | 250 | 20 000 |
| Hg(II) | > 22,5 | 237 | 15 000 |
| Pb(II) | 8,9 | 349 | 5 500 |

**Mode opératoire :**

La procédure est la même que celle suivie ci-dessus au paragraphe 2/ 1-.

Les solutions métalliques sont préparées à partir du sel correspondant ($CaCl_2$, $CdCl_2$, $PbCl_2$ ou $ZnCl_2$) dans une solution tampon Bis-Tris à 20 mM de pH = 7, et titrées avec une solution d'EDTA à 5 mM en présence d'un indicateur colorimétrique.

Pour les titrages, les aliquotes des solutions de Ca(II), Cd(II) ou Zn(II) sont ajoutées à la cellule UV contenant un complexe peptide/plomb dans une solution tampon de Bis-Tris à 20 mM de pH = 7, obtenu à partir d'1 équivalent de ligand et de 3 équivalents de Pb(II). L'expérience est réalisée jusqu'à la disparition du signal correspondant au complexe peptide/plomb, ou jusqu'à ce que le signal du complexe peptide/plomb soit inférieur à 10%. Pour Ca(II), aucune évolution du spectre du complexe du plomb n'a été observée même pour 1000 équivalents de Ca(II) ajoutés par rapport au ligand.

Les spectres ont été analysés en utilisant le programme SPECFIT, comme précédemment. La solution tampon Bis-Tris a été choisie parce qu'elle forme un complexe stable et soluble avec le Pb(II), empêchant ainsi la formation et

la précipitation de $Pb(OH)_2$ (J. C. Payne, M. A. terHorst, H. A. Godwin, J. Am. Chem. Soc, 1999, 121, pp. 6850-6855). L'affinité des ions métalliques pour la solution tampon de Bis-Tris étant par ailleurs connue, elle a été inclue en tant que paramètre dans l'ajustement (logβ1 = 2,25 pour le Ca(II), 2,47 pour le Cd(II), 2,38 pour le Zn(II) et 4,32 pour le Pb(II) (K. H. Scheller, T. H. Abel, P. E. Polanyi, P. K. Wenk, B. E. Fischer, H. Sigel, Eur. J. Biochem., 1980, 107, pp. 455-466).

Pour le titrage avec Ca(II), aucune évolution de la bande LMCT du complexe Pb(II) n'a été mise en évidence, ainsi les données ont été simulées avec moins de 1% de Pb(II), ce qui a permis d'obtenir une limite supérieure pour la constante d'affinité apparente $\log ß_{CaNTACys(OC2H5)3} < 3$.

Une solution de mercure est préparée par dissolution de $HgCl_2$ dans l'eau. Un complexe HgL est formé par addition d'aliquotes de cette solution de mercure à une solution de ligand dans une solution tampon phosphate de 20 mM à pH = 7,4 jusqu'à 0,9 équivalent. L'évolution de la bande LMCT de Hg(II) à 290 nm est ensuite suivie par titrage avec une solution de $Na_2EDTA$ à 5 mM. Aucune évolution de cette bande n'est observée jusqu'à 63 équivalents d'EDTA ajoutés. Ces données ont été simulées avec moins de 5% de Hg(II) déplacés par l'EDTA, ce qui conduit à une constante d'affinité apparente $\log ß_{HgNTACys(OC2H5)3} > 22,5$.

**3/ 2- Etude réalisée sur le composé 7 NOTA($CysOC_2H_5$)$_3$**

La même procédure que pour le composé 3 nous a permis d'évaluer les affinités suivantes :

**Tableau VI** : Constantes de stabilité apparente des complexes M.NOTA($CysOC_2H_5$)$_3^-$ à pH = 7, à une température de 298 K

| | log $K_{app}$ (à pH = 7) | $\lambda_{max}$ (nm) | ε ($cm^{-1}M^{-1}$) |
|---|---|---|---|
| Ca(II) | - | - | - |
| Zn(II) | 8-10 | - | - |
| Cd(II) | 8-10 | - | - |
| Hg(II) | - | 240 | 14000 |
| Pb(II) | 10 | 342 | 3 700 |

**Résultats comparatifs :**

Les constantes de stabilité apparentes de trois composés représentatifs de l'état de l'art, et plus particulièrement les constantes de stabilité des composés 1, 2 et 3 décrits dans l'article de Plush et al., Dalton Trans., 2004, No. 9, 1410-1417, mesurées dans des conditions similaires (à pH = 7 et à une température de 298 K) sont récapitulées dans le Tableau VI' ci-dessous.

Pour rappel, les composés 1 et 2 de l'article de Plush *et al.* sont des dérivés du NOTA porteurs de résidus phénylalanines, et le composé 3 de l'article de Plush *et al.* est un dérivé du NOTA porteurs de résidus tryptophanes.

**Tableau VI' :** Constantes de stabilité apparente des composés 1,2 et 3 de l'article de Plush *et al.* à pH = 7, à une température de 298 K

| **log $K_{app}$ (à pH = 7)** | **Composé 1 de l'article de Plush *et al.*** | **Composé 2 de l'article de Plush *et al.*** | **Composé 3 de l'article de Plush *et al.*** |
|---|---|---|---|
| Cd(II) | 4,8 | 2,9 | 6,7 |
| Cu(II) | 8,3 | 10,4 | 9,0 |

La fonctionnalisation des agents chélatants de l'invention par des acides aminés cystéines, porteurs d'atomes de soufre capables de se lier aux métaux « mous », conduit à une meilleure affinité vis-à-vis des ions métalliques « mous », tel que le Cd(II), en comparaison aux composés connus de l'état de l'art.

**Avantages des agents chélatants de l'invention en comparaison à d'autres agents chélatants connus :**

Le Tableau VII rassemble les constantes d'affinité mesurées avec les agents chélatants de l'invention. Les valeurs mesurées avec un peptide modèle ($P^C$) de la boucle de liaison du cuivre Cu(I) d'une métallochaperonne Atx1 (protéine impliquée dans le transport du cuivre Cu(I)), sont également données, pour comparaison avec les protéines chélatant le Cu(I) naturellement dans les cellules.

Le ligand $P^C$ lie les ions métalliques grâce à deux fonctions thiolates de deux cystéines insérées dans une séquence d'acides aminés MxCxxC. L'introduction de trois cystéines dans le composé 3 NTA($CysOC_2H_5$)$_3$, le composé 5 NTA($CysNH_2$)$_3$ et le composé 7 NOTA($CysOC_2H_5$)$_3$ a permis d'obtenir des complexes stables et de très forte sélectivité par rapport aux ions essentiels potentiellement compétiteurs, Ca(II) et Zn(II).

**Tableau VII** : Bilan des constantes d'affinité avec les chélateurs cystéines

| **log $K_{app}$** | **$P^C$** | **Composé 3 NTA($CysOC_2H_5$)$_3$** | **Composé 7 NOTA($CysOC_2H_5$)$_3$** |
|---|---|---|---|
| Ca(II) | - | < 3 | - |
| Cu(I) | 16,5 | 19 | 17,5 |
| Cu(II) | - | - | - |
| Zn(II) | 6,8 | 9,1 | 8-10 |
| Cd(II) | 9,2 | 10,6 | 8-10 |
| Hg(II) | > 18,6 | > 22,5 | |
| Pb(II) | 8,0 | 8,9 | 10 |
| Sél. Cu/Zn | 9,7 | 9,9 | 7,5-9,5 |
| Sél. Cu/Ca | - | > 16 | - |
| Sél. Hg/Zn | > 11,8 | > 13,4 | - |
| Sél. Hg/Ca | | > 19,5 | |

**Résultats comparatifs :**

Les sélectivités pour le cuivre par rapport au zinc ont également été mesurées pour trois composés représentatifs de l'état de l'art, i. e. les composés 1, 2 et 3 décrits dans l'article de Plush et al., Dalton Trans., 2004, No. 9, 1410-1417, et les résultats sont récapitulés dans le Tableau VII' ci-dessous.

**Tableau VII'** : Sélectivité Cu/Zn des composés 1, 2 et 3 de l'article de Plush *et al.*

| | **Composé 1 de l'article de Plush *et al*.** | **Composé 2 de l'article de Plush *et al*.** | **Composé 3 de l'article de Plush *et al.*** |
|---|---|---|---|
| Sél. Cu/Zn | 1,0 | 1,8 | 0,6 |

La fonctionnalisation des agents chélatants de l'invention par des acides aminés cystéines, porteurs d'atomes de soufre capables de se lier aux métaux « mous », conduit à une sélectivité Cu/Zn considérablement améliorée, en comparaison aux composés connus de l'état de l'art. Afin de montrer l'intérêt des agents chélatants de l'invention, nous avons également comparé les données obtenues, répertoriées dans le Tableau VII, avec celles connues d'agents chélatants commerciaux connus répertoriés (cf. Tableau IV).

Pour le composé 3 NTA($CysOC_2H_5$)$_3$ :

L'affinité du composé 3 NTA($CysOC_2H_5$)$_3$ pour les ions Cu(I) et Hg(II) est très élevée. Ce ligand complexe mieux le cuivre que l'EDTA et le Trien, et est un complexant très efficace de Hg(II), d'affinité plus forte que les autres agents chélatants tabulés.

Un point important pour l'utilisation d'agents chélatants *in vivo* est leur sélectivité vis-à-vis des ions essentiels tels que Ca(II) et Zn(II). Là encore, les sélectivités mesurées pour le composé 3 NTA($CysOC_2H_5$)$_3$ sont nettement supérieures à celles des agents chélatants connus.

Pour le composé 7 NOTA($CysOC_2H_5$)$_3$ :

Moins de données ont été mesurées avec ce ligand. On peut néanmoins noter une forte affinité entre le composé 7 NOTA($CysOC_2H_5$)$_3$ et le Cu(I), ainsi qu'une sélectivité intéressante pour le cuivre par rapport au zinc. Ses propriétés

sont donc très intéressantes en comparaison à l'EDTA et au Trien.

Les composés de l'invention présentent des affinités et sélectivités qui les rendent très prometteurs pour la complexation sélective du Cu(I), ayant un degré d'oxydation favorisé dans le milieu intracellulaire, et qui peut être ciblé pour les maladies de type Wilson ou Alzheimer. Ces composés peuvent également être candidats pour la complexation sélective du mercure lors d'intoxications par ce métal.

**4/ Utilisation des composés de formule (II) et (III) pour complexer le cuivre en milieu cellulaire**

**4/ 1- Libération des fonctions thiols des composés de formule (II) en milieu réducteur, mimant le milieu intracellulaire**

Nous avons démontré expérimentalement que le composé de formule ($II_{a1}$) ne complexait pas le cuivre en milieu non réducteur, alors qu'il le complexe fortement en milieu réducteur, c'est-à-dire autant que le composé 4. Ce résultat montre que les composés de formule (II) sont des agents chélatants de métaux uniquement dans les cellules ciblées, et par conséquent présentent moins d'effets secondaires liés à une complexation non désirée des métaux dans le milieu extracellaire comme le sang circulant.

**Résultats expérimentaux :**

Le BCS a été utilisé pour déterminer la concentration en cuivre (I) libre.

La quantité de cuivre libre détectée a été déterminée dans différentes conditions, telles que résumées sur la Figure 2 annexée.

Lorsque le composé ($II_{a1}$) est seul, il ne complexe pas le cuivre (100% détecté par le BCS), ce qui s'explique par le fait que les fonctions thiols sont masquées par les ponts disulfure (S-S) de la molécule.

Par contre, en présence d'un réducteur tel que le GSH ou le TCEP, qui sont capables de réduire les ponts disulfure (S-S) pour régénérer les fonctions thiols libres, le composé de formule ($II_{a1}$) devient un agent chélatant efficace du cuivre (le composé 4), puisque la quantité de cuivre détecté par le BCS chute à 8 et 2%.

**4/ 2- Expériences sur cellules hépatiques**

**Entrée de l'agent chélatant dans la cellule hépatique :**

L'entrée de l'agent chélatant répondant à la formule ($III_1$) dans des cellules hépatiques de type HEPG2, WIF-B ou CAN10 est étudiée en suivant la fluorescence dans le rouge de la rhodamine par microscopie. Des cinétiques d'entrée sont réalisées pour mesurer les temps d'entrée. L'influence de la concentration initiale en agent chélatant de formule ($III_1$) dans le milieu extracellulaire sur le temps d'incorporation est également analysée.

Il a été montré que le composé de formule ($III_1$) pénétrait dans des cellules hépatiques HEPG2 au bout de 2 heures pour des concentrations de 0,2 $\mu$M et 2 $\mu$M dans le milieu extracellulaire. En effet la figure 3 jointe montre l'apparition de la luminescence rouge caractéristique de la rhodamine dans les cellules. La fluorescence est plus intense au bout de 7 heures comme on peut l'observer sur la figure 4, ce qui signifie que l'incorporation de la molécule de formule ($III_1$) se poursuit entre 2 heures et 7 heures.

**Complexation du cuivre dans les cellules :**

Il a été montré que la position de la protéine de Wilson (ou ATP7B) membranaire dépendait de la concentration en cuivre intracellulaire dans des cellules hépatiques WIF-B (référence Guo et al., J. Am. Physiol Gastrointest Liver Physiol 289:G904-G916, 2005). Cette protéine est donc utilisée comme indicateur de la présence ou de l'absence de cuivre intracellulaire par marquage avec des anticorps fluorescents dans le vert.

La position de l'ATP7B peut donc être enregistrée par microscopie de fluorescence. En absence de cuivre, cette protéine se situe dans la région du Golgi, alors qu'en excès de cuivre elle se déplace vers la membrane apicale et donc vers les canalicules pour excréter ce métal en excès. La comparaison des images obtenues en présence et en absence d'agent chélatant de formule ($II_{a1}$) dans des cellules hépatiques dans un milieu riche en cuivre permet de voir si le composé ($II_{a1}$), qui est un agent chélatant très efficace *in vitro,* est également un agent chélatant du cuivre *in cellulo,* dans les cellules hépatiques testées.

**Revendications**

**1.** Composés de formule (I) suivante :

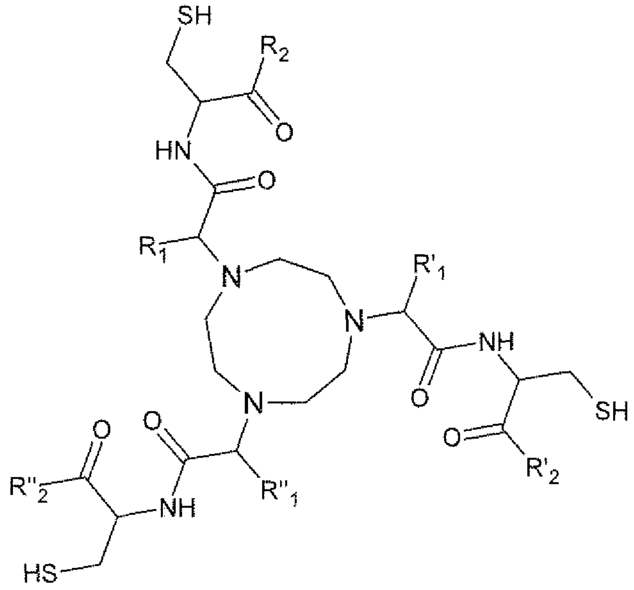

(I)

dans lesquels :

- les radicaux $R_1$, $R'_1$ et $R''_1$, identiques ou différents, représentent un atome d'hydrogène ou un radical alkyle ayant 1 à 12 atomes de carbone,
- les radicaux $R_2$, $R'_2$ et $R''_2$, identiques ou différents, sont choisis parmi les groupements -OH, -OR, -NHR et -NRR' dans lesquels R et R', identiques ou différents, représentent un atome d'hydrogène ou un radical alkyle ayant 1 à 12 atomes de carbone.

**2.** Procédé de préparation des composés de formule (I) tels que définis selon la revendication 1 **caractérisé en ce qu'**il comprend au moins les étapes suivantes :

(i) réaction d'un équivalent d'une molécule $R_bCHR_1C(O)R_a$, où :

- $R_a$ est sélectionné parmi les atomes d'halogène, de préférence les atomes de chlore ou de brome, les groupements hydroxyles -OH,-$OCOR_{a'}$ dans lesquels $R_{a'}$ représente un groupement alkyle ayant 1 à 12 atomes de carbone, $R_{a'}$ étant de préférence un groupement méthyle ou éthyle,
- $R_b$ est sélectionné parmi les atomes d'halogène, de préférence les atomes de chlore ou de brome, les groupements tosylates tels que le paratoluènesulfonate, et les groupements mésylates tels que le méthanesulfonate et le trifluorométhanesulfonate,

avec un équivalent d'un dérivé de la cystéine de formule :

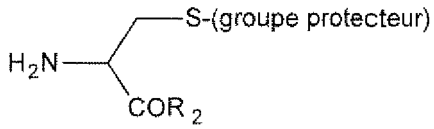

en présence d'une base faible, telle que l'hydrogénocarbonate de potassium, l'hydrogénocarbonate de sodium, le carbonate de potassium, le carbonate de sodium et les amines tertiaires comme la diisopropyléthylamine ou la triéthylamine, et en milieu solvant, ledit solvant pouvant être choisi parmi les solvants polaires tels que le dichlorométhane, le chloroforme, l'acétate d'éthyle, l'acétonitrile, la diméthylformamide et l'eau, de préférence

à une température comprise entre -10°C et 10°C, pendant une durée comprise typiquement entre 30 minutes et 2 heures,

(ii) réaction d'un équivalent de 1,4,7-triazacyclononane avec trois équivalents d'un dérivé bromo-acétamide obtenu lors de l'étape (i), de formule :

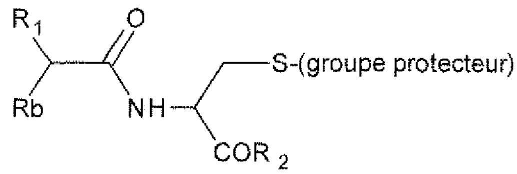

en présence d'une base faible, telle que l'hydrogénocarbonate de potassium, l'hydrogénocarbonate de sodium, le carbonate de potassium, le carbonate de sodium et les amines tertiaires comme la diisopropyléthylamine ou la triéthylamine, et en milieu solvant, ledit solvant pouvant être choisi parmi le dichlorométhane, le chloroforme, l'acétate d'éthyle, l'acétonitrile et la diméthylformamide,

(iii) déprotection de la fonction -S-(groupe protecteur) en fonction thiol -SH, ladite déprotection pouvant être réalisée par addition d'un acide fort en large excès, tel que l'acide trifluoroacétique lorsque le groupe protecteur est le triphénylméthane $C(C_6H_5)_3$, de préférence à une température comprise entre 20°C et 40°C pendant une durée comprise typiquement entre 15 minutes et 1 heure.

**3.** Utilisation non thérapeutique des composés de formule (I) selon la revendication 1 comme agents chélatants des ions métalliques de la classification périodique de Mendeleiev, et de préférence comme agents chélatants des ions Ag(I), Cd(II), Co(II), Cu(I), Hg(II), Ni(II), Au(I), Pb(II) et Zn(II).

**4.** Utilisation des composés de formule (I) selon la revendication 1 comme agents dépolluants.

**5.** Composés susceptibles d'être utilisés comme agents précurseurs des composés de formule (I) tels que définis selon la revendication 1, répondant à la formule (II) suivante :

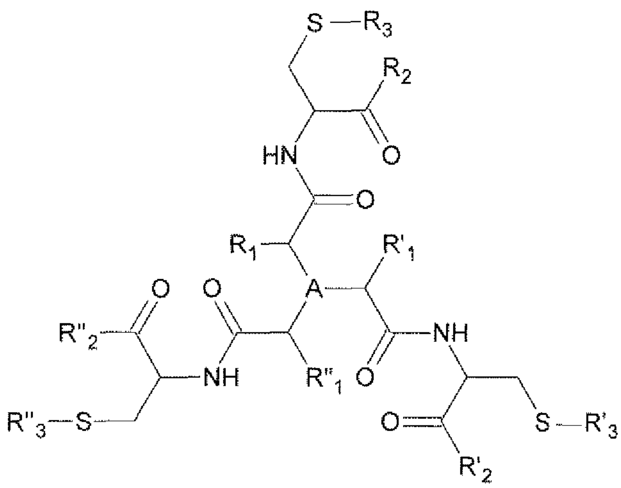

(II)

dans laquelle le groupement A représente :

- soit un atome d'azote,
- soit un cycle répondant à la formule ci-dessous, et dans lequel la substitution se fait sur les atomes d'azote :

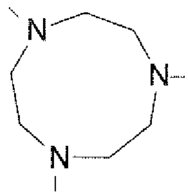

et dans laquelle :

- les radicaux $R_1$, $R'_1$ et $R''_1$, identiques ou différents, représentent un atome d'hydrogène ou un radical alkyle ayant 1 à 12 atomes de carbone,
- les radicaux $R_2$, $R'_2$ et $R''_2$, identiques ou différents, sont choisis parmi les groupements -OH, -OR, -NHR et -NRR' dans lesquels R et R', identiques ou différents, représentent un atome d'hydrogène ou un radical alkyle ayant 1 à 12 atomes de carbone,
- les radicaux $R_3$, $R'_3$ et $R''_3$, identiques ou différents, représentent un groupement -S-W ou -S-E-L, où :

  ✔ S est un atome de soufre,
  ✔ W est un radical alkyle ayant 1 à 12 atomes de carbone,
  ✔ E est un bras espaceur sélectionné parmi les groupements alkyles ayant 1 à 12 atomes de carbone, et les polyols tels que le polyéthylène glycol ayant de préférence 1 à 8 motifs oxyéthylène OE, et
  ✔ L est un ligand sélectionné parmi le glucose, le galactose et le N-acétylgalactosamine.

**6.** Composés de formule (II) selon la revendication 5 répondant à la formule spécifique ($II_a$) suivante :

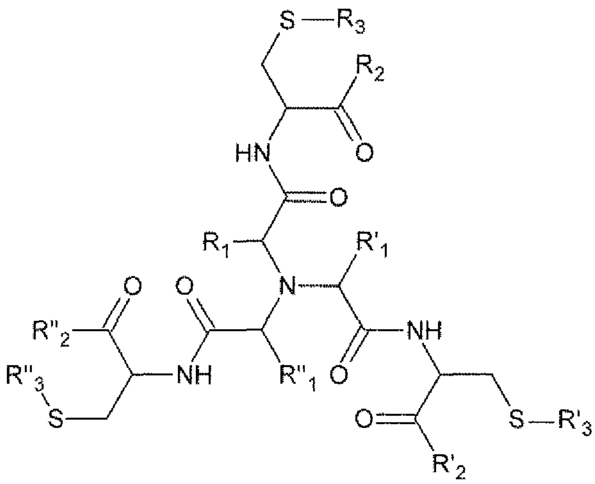

($II_a$)

**7.** Composés de formule ($II_a$) selon la revendication 6, dans lesquels:

- les radicaux $R_1$, $R'_1$ et $R''_1$ sont des atomes d'hydrogène,
- les radicaux $R_2$, $R'_2$ et $R''_2$ sont des groupements $-NH_2$, et
- les radicaux $R_3$, $R'_3$ et $R''_3$, identiques ou différents, représentent un groupement -S-E-L, où :

  ✔ S est un atome de soufre,
  ✔ E représente un polyéthylène glycol ayant 3 motifs oxyéthylène OE, et
  ✔ L représente le N-acétylgalactosamine.

**8.** Composés de formule (II) selon la revendication 5 répondant à la formule spécifique ($II_b$) suivante :

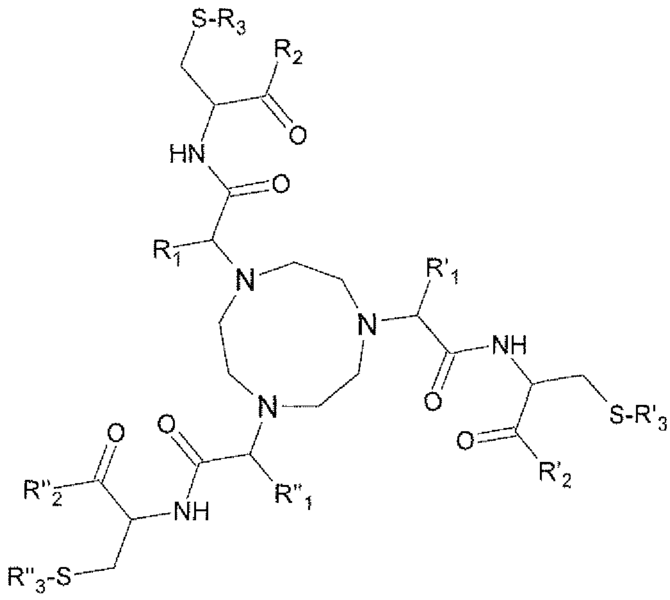

(II_b)

**9.** Composés de formule ($II_{a'}$) suivante :

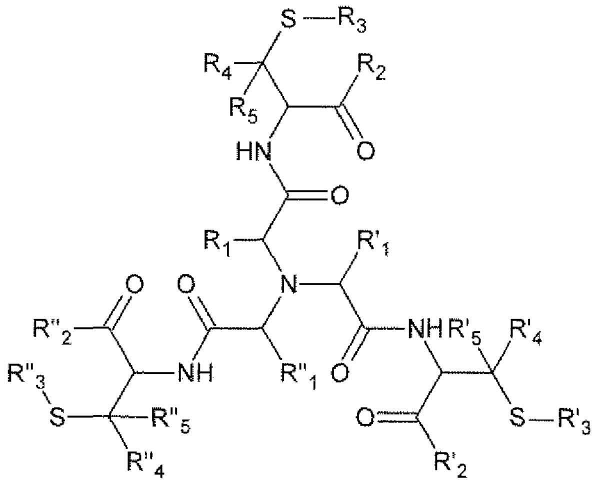

($II_{a'}$)

dans lesquels :

- les radicaux $R_1$, $R'_1$, $R''_1$, $R_4$, $R'_4$, $R''_4$, $R_5$, R's, $R''_5$, identiques ou différents, représentent un atome d'hydrogène ou un radical alkyle ayant 1 à 12 atomes de carbone,
- les radicaux $R_2$, $R'_2$ et $R''_2$, identiques ou différents, sont choisis parmi les groupements -OH, -OR, -NHR et -NRR' dans lesquels R et R', identiques ou différents, représentent un atome d'hydrogène ou un radical alkyle ayant 1 à 12 atomes de carbone,
- les radicaux $R_3$, $R'_3$ et $R''_3$, identiques ou différents, représentent un groupement -S-W ou -S-E-L, où :

✔ S est un atome de soufre,
✔ W est un radical alkyle ayant 1 à 12 atomes de carbone,
✔ E est un bras espaceur sélectionné parmi les groupements alkyles ayant 1 à 12 atomes de carbone, et les polyols tels que le polyéthylène glycol ayant de préférence 1 à 8 motifs oxyéthylène OE,
✔ L est un ligand sélectionné parmi le glucose, le galactose et le N-acétylgalactosamine,

**10.** Composés de formule (III) suivante :

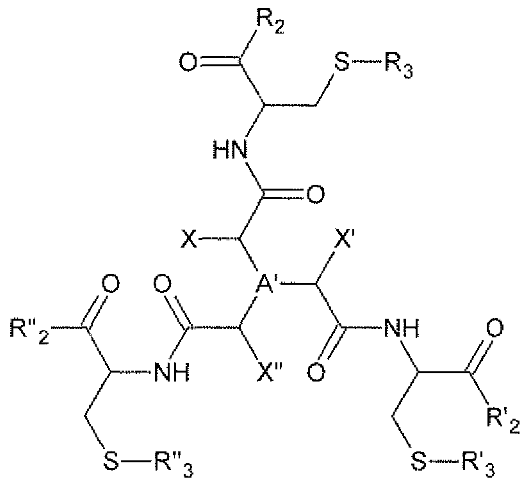

(III)

dans lesquels le groupement A' représente :

- soit un atome d'azote,
- soit un cycle répondant à la formule ci-dessous, et dans lequel la substitution se fait sur les atomes d'azote :

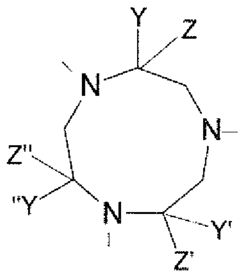

et dans lesquels :

- les radicaux $R_2$, $R'_2$ et $R''_2$, identiques ou différents, sont choisis parmi les groupements -OH, -OR, -NHR et -NRR' dans lesquels R et R', identiques ou différents, représentent un atome d'hydrogène ou un radical alkyle ayant 1 à 12 atomes de carbone,
- les radicaux $R_3$, $R'_3$ et $R''_3$, identiques ou différents, représentent un groupement -S-W ou -S-E-L, où :

✓ S est un atome de soufre,
✓ W est un radical alkyle ayant 1 à 12 atomes de carbone,
✓ E est bras espaceur sélectionné parmi les groupements alkyles ayant 1 à 12 atomes de carbone, et les polyols tels que le polyéthylène glycol ayant de préférence 1 à 8 motifs oxyéthylène OE,
✓ L est un ligand sélectionné parmi le glucose, le galactose et le N-acétylgalactosamine,

- les groupements X, X', X", Y, Y', Y", Z, Z' et Z", identiques ou différents, représentent un atome d'hydrogène, un groupement -$(CH_2)_n$-NH-CO-marqueur, un groupement -$(CH_2)_n$-NH-C(S)NH-marqueur, un groupement -$(CH_2)$n-NH-$SO_2$-marqueur, un groupement -$(CH_2)_n$-N=C-marqueur, un groupement -$(CH_2)_n$-NH-Ar-marqueur, ou -$(CH_2)_n$-triazole-marqueur, où Ar est un groupement aryle choisi parmi le phényle, le triazole, l'oxadiazole, l'oxazole, l'imidazole, le thiadiazole, le pyrrole, le tétrazole, le furane, le thiophène, le pyrazole, la pyrazoline, la pyrazidine, le thiazole, l'isothiazole, la pyridine, la pyrimidine, la pipéridine, le pyranne, la pyrazine et la pyridazine, et dans lesquels n est compris entre 1 et 12, et à la condition qu'au moins un desdits groupements X, X', X", Y, Y', Y", Z, Z' ou Z" soit un groupement-$(CH_2)_n$-NH-CO-marqueur, -$(CH_2)_n$-NH-C(S)NH-marqueur, -$(CH_2)_n$-NH-$SO_2$-marqueur, -$(CH_2)_n$-N=C-marqueur ou -$(CH_2)_n$-NH-Ar-

marqueur ou -$(CH_2)_n$-triazo le -marqueur.

**11.** Composés de formule (III) selon la revendication 10, dans lesquels :

- les radicaux $R_2$, $R'_2$ et $R''_2$ sont des groupements -$NH_2$,
- les radicaux $R_3$, $R'_3$ et $R''_3$ représentent un groupement -S-E-L, où :

  ✓ S est un atome de soufre,
  ✓ représente un polyéthylène glycol ayant 3 motifs oxyéthylène OE, et
  ✓ représente le N-acétylgalactosamine,

- au moins un des groupements X, X', X", Y, Y', Y", Z, Z' ou Z" représente un groupement -$(CH_2)_4$-carboxytétraméthylrhodamine, les autres groupements représentant des atomes d'hydrogène.

**12.** Composés de formule (I), (II), ($II_{a'}$) ou (III) tels que définis selon l'une des revendications 1 ou 5 à 7 ou 8 à 10 pour leur utilisation en tant que médicaments.

**13.** Utilisation des composés de formule (I), (II), ($II_{a'}$) ou (III) tels que définis selon l'une des revendications 1 ou 5 à 11 pour la préparation d'un médicament destiné au diagnostic, à la prévention et/ou au traitement de maladies neurodégénératives, telles que les maladies de Wilson et d'Alzheimer.

**14.** Composés de formule (I), (II), ($II_{a'}$) ou (III) tels que définis selon l'une des revendications 1 ou 5 à 11 pour leur utilisation pour le diagnostic, la prévention et/ou le traitement d'intoxications avec des ions métalliques tels que les ions argent, cadmium, cobalt, cuivre, mercure, nickel, or, plomb et zinc.

**15.** Composition pharmaceutique **caractérisée en ce qu'**elle comprend en tant que principe actif au moins un composé de formule (I), (II), ($II_{a'}$) ou (III) tel que défini selon l'une des revendications 1 ou 5 à 11, et au moins un véhicule pharmaceutiquement acceptable.

**Patentansprüche**

**1.** Verbindungen der folgenden Formel (I):

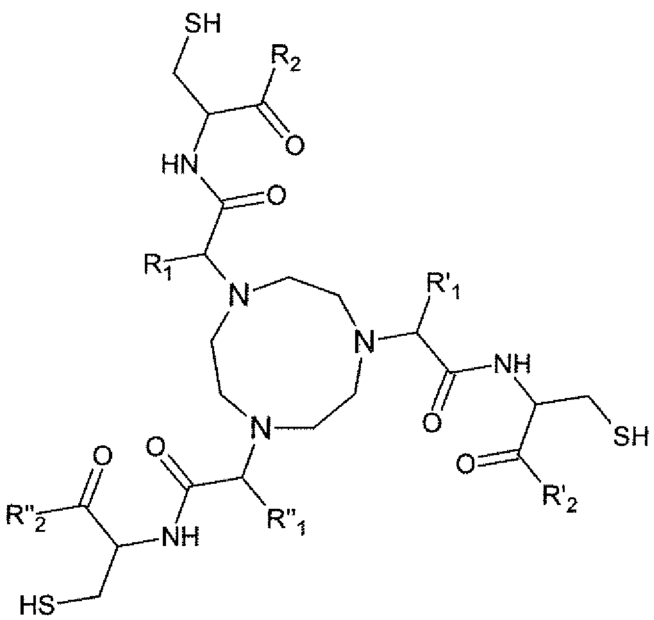

(I)

in denen:

- die Reste $R_1$, $R'_1$ und $R''_1$, gleich oder verschieden, für ein Wasserstoffatom oder einen Alkylrest mit 1 bis 12 Kohlenstoffatomen stehen,
- die Reste $R_2$, $R'_2$ und $R''_2$, gleich oder verschieden, aus den Gruppen -OH, -OR, -NHR und -NRR' ausgewählt sind, in denen R und R', gleich oder verschieden, für ein Wasserstoffatom oder einen Alkylrest mit 1 bis 12 Kohlenstoffatomen stehen.

2. Verfahren zur Herstellung von Verbindungen der Formel (I), wie nach Anspruch 1 definiert, **dadurch gekennzeichnet, dass** es mindestens die folgenden Schritte umfasst:

(i) Umsetzen eines Äquivalents eines Moleküls $R_bCHR_1C(O)R_a$, worin:

- $R_a$ aus Halogenatomen, vorzugsweise Chlor- oder Bromatomen, Hydroxylgruppen -OH, -OCOR$_{a'}$, ausgewählt ist, in denen $R_{a'}$ für eine Alkylgruppe mit 1 bis 12 Kohlenstoffatomen steht, wobei $R_{a'}$ vorzugsweise eine Methyl- oder Ethylgruppe ist,
- $R_b$ aus Halogenatomen, vorzugsweise Chlor- oder Bromatomen, Tosylatgruppen wie Paratoluolsulfonat und Mesylatgruppen wie Methansulfonat und Trifluormethansulfonat ausgewählt ist,

mit einem Äquivalent eines Derivats des Cysteins der Formel:

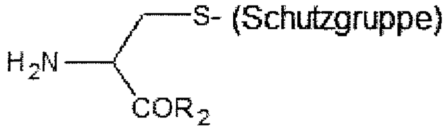

in Gegenwart einer schwachen Base wie Kaliumhydrogencarbonat, Natriumhydrogencarbonat, Kaliumcarbonat, Natriumcarbonat und tertiären Aminen wie Diisopropylethylamin oder Triethylamin und in einem Lösungsmittelmedium, wobei das besagte Lösungsmittel aus polaren Lösungsmitteln wie Dichlormethan, Chloroform, Ethylacetat, Acetonitril, Dimethylformamid und Wasser ausgewählt sein kann, vorzugsweise bei einer Temperatur, die zwischen -10 °C und 10 °C liegt, für eine Dauer, die in der Regel zwischen 30 Minuten und 2 Stunden liegt,
(ii) Umsetzen eines Äquivalents von 1,4,7-Triazacyclononan mit drei Äquivalenten eines während des Schritts (i) erhaltenen Bromacetamid-Derivats der Formel:

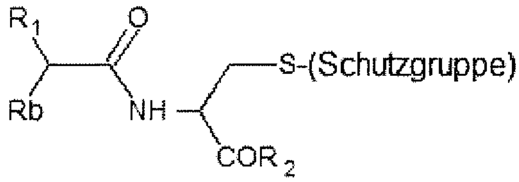

in Gegenwart einer schwachen Base wie Kaliumhydrogencarbonat, Natriumhydrogencarbonat, Kaliumcarbonat, Natriumcarbonat und tertiären Aminen wie Diisopropylethylamin oder Triethylamin und in einem Lösungsmittelmedium, wobei das besagte Lösungsmittel aus Dichlormethan, Chloroform, Ethylacetat, Acetonitril und Dimethylformamid ausgewählt sein kann,
(iii) Entschützen der Funktion -S-(Schutzgruppe) in eine Thiolfunktion -SH, wobei das besagte Entschützen durch Zugabe einer starken Säure in hohem Überschuss, wie Trifluoressigsäure, wenn die Schutzgruppe Triphenylmethan $C(C_6H_5)_3$ ist, vorzugsweise bei einer Temperatur, die zwischen 20 °C und 40 °C liegt, für eine Dauer, die in der Regel zwischen 15 Minuten und 1 Stunde liegt, durchgeführt werden kann.

3. Nichttherapeutische Verwendung von Verbindungen der Formel (I) nach Anspruch 1 als Chelatbildner von Metallionen des Periodensystems nach Mendelejew und vorzugsweise als Chelatbildner der Ionen Ag(I), Cd(II), Co(II), Cu(I), Hg(II), Ni(II), Au(I), Pb(II) und Zn(II).

4. Verwendung von Verbindungen der Formel (I) nach Anspruch 1 als Entgiftungsmittel.

5. Verbindungen, die zur Verwendung als Vorläufer von Verbindungen der Formel (I), wie nach Anspruch 1 definiert, geeignet sind und die der folgenden Formel (II) entsprechen:

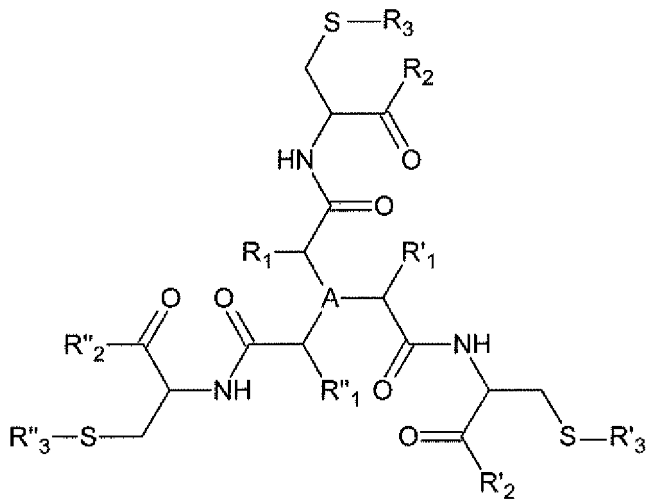

(II)

in der die Gruppe A für Folgendes steht:

- entweder ein Stickstoffatom,
- oder ein Ring, der der obigen Formel entspricht und in dem die Substitution an den Stickstoffatomen vorgenommen wird:

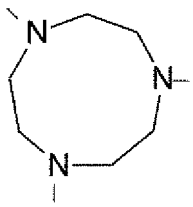

und in der:

- die Reste $R_1$, $R'_1$ und $R''_1$, gleich oder verschieden, für ein Wasserstoffatom oder einen Alkylrest mit 1 bis 12 Kohlenstoffatomen stehen,
- die Reste $R_2$, $R'_2$ und $R''_2$, gleich oder verschieden, aus den Gruppen -OH, -OR, -NHR und -NRR' ausgewählt sind, in denen R und R', gleich oder verschieden, für ein Wasserstoffatom oder einen Alkylrest mit 1 bis 12 Kohlenstoffatomen stehen,
- die Reste $R_3$, $R'_3$ und $R''_3$, gleich oder verschieden, für eine Gruppe -S-W oder -S-E-L stehen, worin:

  ✓ S ein Schwefelatom ist,
  ✓ W ein Alkylrest mit 1 bis 12 Kohlenstoffatomen ist,
  ✓ E ein Spacer-Arm ist, der aus Alkylgruppe mit 1 bis 12 Kohlenstoffatomen und Polyolen wie Polyethylenglykol mit vorzugsweise 1 bis 8 Oxyethylen-Motiven OE ausgewählt ist, und
  ✓ L ein Ligand ist, der aus Glukose, Galaktose und N-Acetylgalaktosamin ausgewählt ist.

**6.** Verbindungen der Formel (II) nach Anspruch 5, die der folgenden spezifischen Formel ($II_a$) entsprechen:

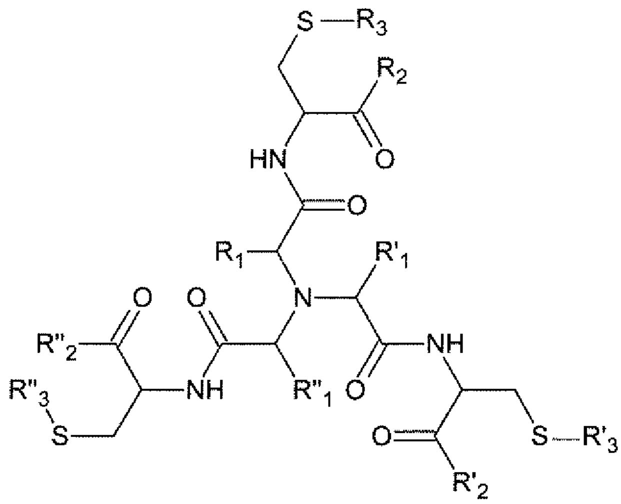

($II_a$)

**7.** Verbindungen der Formel ($II_a$) nach Anspruch 6, in denen:

- die Reste $R_1$, $R'_1$ und $R''_1$ Wasserstoffatome sind,
- die Reste $R_2$, $R'_2$ und $R''_2$ Gruppen $-NH_2$ sind und
- die Reste $R_3$, $R'_3$ und $R''_3$, gleich oder verschieden, für eine Gruppe -S-E-L stehen, worin:

✔ S ein Schwefelatom ist,
E für ein Polyethylenglykol mit 3 Oxyethylen-Motiven OE steht und
✔ L für N-Acetylgalaktosamin steht.

**8.** Verbindungen der Formel (II) nach Anspruch 5, die der folgenden spezifischen Formel ($II_b$) entsprechen:

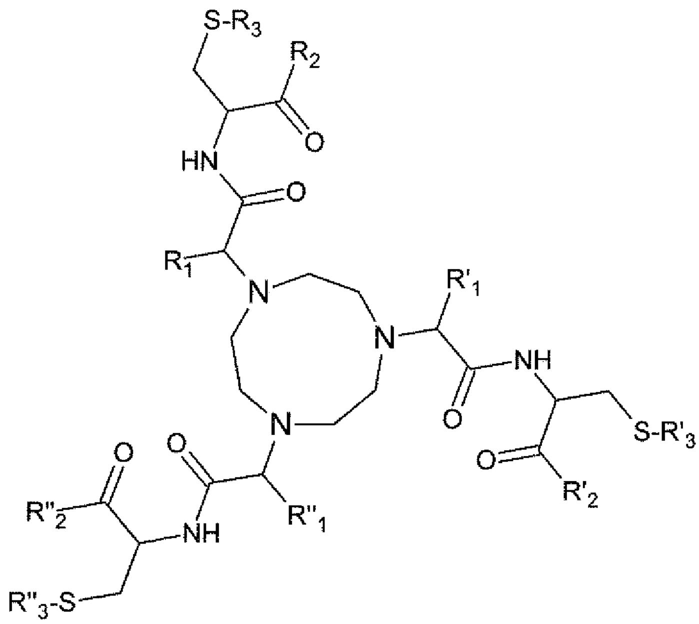

($II_b$)

**9.** Verbindungen der folgenden Formel ($II_{a'}$):

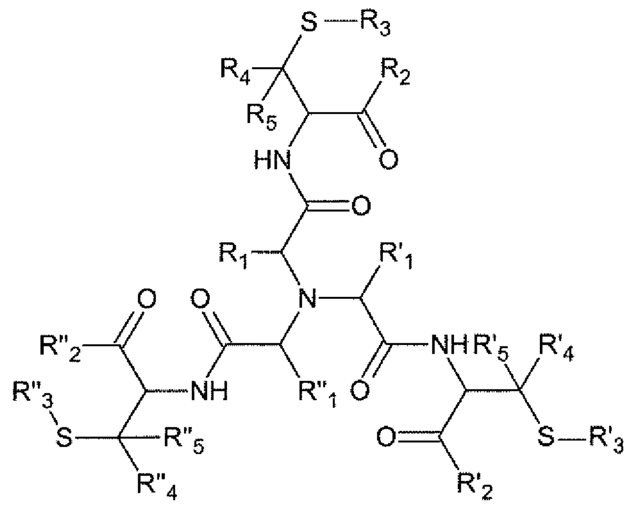

(IIa')

in denen:

- die Reste $R_1$, $R'_1$, $R''_1$, $R_4$, $R'_4$, $R''_4$, $R_5$, $R'_5$, $R''_5$, gleich oder verschieden, für ein Wasserstoffatom oder einen Alkylrest mit 1 bis 12 Kohlenstoffatomen stehen,
- die Reste $R_2$, $R'_2$ und $R''_2$, gleich oder verschieden, aus den Gruppen -OH, -OR, -NHR und -NRR' ausgewählt sind, in denen R und R', gleich oder verschieden, für ein Wasserstoffatom oder einen Alkylrest mit 1 bis 12 Kohlenstoffatomen stehen,
- die Reste $R_3$, $R'_3$ und $R''_3$, gleich oder verschieden, für eine Gruppe -S-W oder -S-E-L stehen, worin:

  ✓ S ein Schwefelatom ist,
  ✓ W ein Alkylrest mit 1 bis 12 Kohlenstoffatomen ist,
  ✓ E ein Spacer-Arm ist, der aus Alkylgruppen mit 1 bis 12 Kohlenstoffatomen und Polyolen wie Polyethylenglykol mit vorzugsweise 1 bis 8 Oxyethylen-Motiven OE ausgewählt ist,
  ✓ L ein Ligand ist, der aus Glukose, Galaktose und N-Acetylgalaktosamin ausgewählt ist.

**10.** Verbindungen der folgenden Formel (III) :

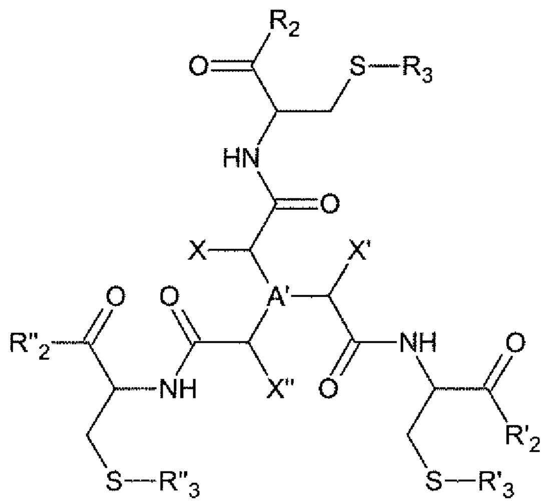

(III)

in denen die Gruppe A' für Folgendes steht:

✓ entweder ein Stickstoffatom,

✓ oder ein Ring, der der obigen Formel entspricht und in dem die Substitution an den Stickstoffatomen vorgenommen wird:

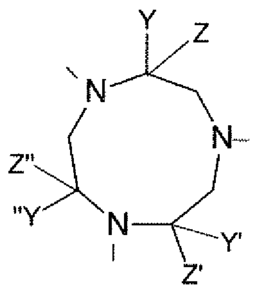

und in denen:

- die Reste $R_2$, $R'_2$ und $R''_2$, gleich oder verschieden, aus den Gruppen -OH, -OR, -NHR und -NRR' ausgewählt sind, in denen R und R', gleich oder verschieden, für ein Wasserstoffatom oder einen Alkylrest mit 1 bis 12 Kohlenstoffatomen stehen,
- die Reste $R_3$, $R'_3$ und $R''_3$, gleich oder verschieden, für eine Gruppe -S-W oder -S-E-L stehen, worin:

  ✓ S ein Schwefelatom ist,
  ✓ W ein Alkylrest mit 1 bis 12 Kohlenstoffatomen ist,
  ✓ E ein Spacer-Arm ist, der aus Alkylgruppen mit 1 bis 12 Kohlenstoffatomen und Polyolen wie Polyethylenglykol mit vorzugsweise 1 bis 8 Oxyethylen-Motiven OE ausgewählt ist,
  ✓ L ein Ligand ist, der aus Glukose, Galaktose und N-Acetylgalaktosamin ausgewählt ist,

- die Gruppen X, X', X", Y, Y', Y'', Z, Z' und Z" , gleich oder verschieden, für ein Wasserstoffatom, eine Gruppe $-(CH_2)_n$-NH-CO-Marker, eine Gruppe $-(CH_2)_n$-NH-C(S)NH-Marker, eine Gruppe $-(CH_2)_n$-NH-$SO_2$-Marker, eine Gruppe $-(CH_2)_n$-N=C-Marker, eine Gruppe $-(CH_2)_n$-NH-Ar-Marker oder $-(CH_2)_n$-Triazol-Marker stehen, worin Ar eine Arylgruppe ist, die aus Phenyl, Triazol, Oxadiazol, Oxazol, Imidazol, Thiadiazol, Pyrrol, Tetrazol, Furan, Thiophen, Pyrazol, Pyrazolin, Pyrazidin, Thiazol, Isothiazol, Pyridin, Pyrimidin, Piperidin, Pyran, Pyrazin und Pyridazin ausgewählt ist, und in denen n zwischen 1 und 12 liegt, und unter der Bedingung, dass mindestens eine der besagten Gruppen X, X', X" , Y, Y', Y", Z, Z' oder Z" eine Gruppe $-(CH_2)_n$-NH-CO-Marker,$-(CH_2)_n$-NH-C(S)NH-Marker, $-(CH_2)_n$-NH-$SO_2$-Marker, $-(CH_2)_n$-N=C-Marker oder $-(CH_2)_n$-NH-Ar-Marker oder - $(CH_2)_n$-Triazol-Marker ist.

**11.** Verbindungen der Formel (III) nach Anspruch 10, in denen:

- die Reste $R_2$, $R'_2$ und $R''_2$ Gruppen $-NH_2$ sind,
- die Reste $R_3$, $R'_3$ und $R''_3$ für eine Gruppe -S-E-L stehen, worin:

  ✓ S ein Schwefelatom ist,
  ✓ E für ein Polyethylenglykol mit 3 Oxyethylen-Motiven OE steht und
  ✓ L für N-Acetylgalaktosamin steht,

- mindestens eine der Gruppen X, X', X", Y, Y', Y" , Z, Z' oder Z" für eine Gruppe $-(CH_2)_4$-Carboxytetramethylrhodamin steht, wobei die anderen Gruppen für Wasserstoffatome stehen.

**12.** Verbindungen der Formel (I), (II), ($II_{a'}$) oder (III), wie nach einem der Ansprüche 1 oder 5 bis 7 oder 8 bis 10 definiert, zu deren Verwendung als Arzneimittel.

**13.** Verbindungen der Formel (I), (II), ($II_{a'}$) oder (III), wie nach einem der Ansprüche 1 oder 5 bis 11 definiert, zur Herstellung eines Arzneimittels, das zur Diagnose, Verhinderung und/oder Behandlung von neurodegenerativen Krankheiten, wie Wilson-Krankheit und Alzheimer-Krankheit, vorgesehen ist.

**14.** Verbindungen der Formel (I), (II), ($II_{a'}$) oder (III), wie nach einem der Ansprüche 1 oder 5 bis 11 definiert, zu deren Verwendung zur Diagnose, Verhinderung und/oder Behandlung von Vergiftungen mit Metallionen wie Silber-, Kadmium-, Kobalt-, Kupfer-, Quecksilber-, Nickel-, Gold-, Blei- und Zinkionen.

**15.** Pharmazeutische Zusammensetzung, **dadurch gekennzeichnet, dass** sie als Wirkstoff mindestens eine Verbindung der Formel (I), (II), ($II_{a'}$) oder (III), wie nach einem der Ansprüche 1 oder 5 bis 11 definiert, und mindestens ein pharmazeutisch annehmbares Vehikel umfasst.

## Claims

**1.** Compounds of formula (I) below:

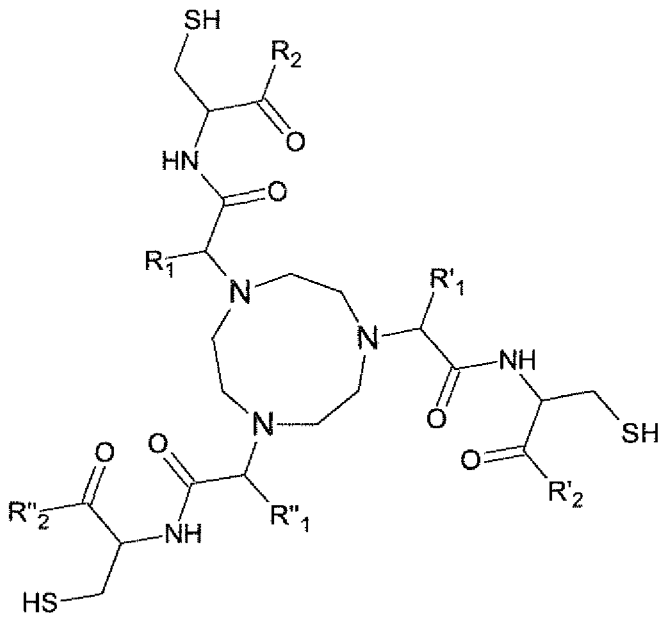

(I)

in which:

- the $R_1$, $R'_1$ and $R''_1$ radicals, which may be identical or different, represent a hydrogen atom or an alkyl radical containing 1 to 12 carbon atoms,
- the $R_2$, $R'_2$ and $R''_2$ radicals, which may be identical or different, are chosen from -OH, -OR, -NHR and -NRR' groups in which R and R', which may be identical or different, represent a hydrogen atom or an alkyl radical containing 1 to 12 carbon atoms.

**2.** Method for preparing the compounds of formula (I) as defined in Claim 1, **characterized in that** it comprises the following steps:

(i) reacting one equivalent of a molecule of $R_bCHR_1C(O)R_a$, in which:

- $R_a$ is selected from halogen atoms, preferably chlorine or bromine atoms, hydroxyl -OH, $-OCOR_{a'}$ groups in which $R_{a'}$ represents an alkyl group having 1 to 12 carbon atoms, $R_{a'}$ preferably being a methyl or ethyl group,
- $R_b$ is selected from halogen atoms, preferably chlorine or bromine atoms, tosylate groups such as para-toluenesulfonate, and mesylate groups such as methanesulfonate and trifluoromethanesulfonate,

with one equivalent of a cysteine derivative of formula:

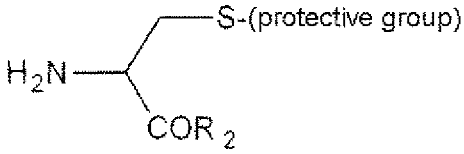

in the presence of a weak base, such as potassium hydrogen carbonate, sodium hydrogen carbonate, potassium carbonate, sodium carbonate and tertiary amines such as diisopropylethylamine or triethylamine, and in a solvent medium, said solvent being chosen from polar solvents such as dichloromethane, chloroform, ethyl acetate, acetonitrile, dimethylformamide and water, preferably at a temperature of between -10°C and 10°C, for a period of typically between 30 minutes and 2 hours,

(ii) reacting one equivalent of 1,4,7-triazacyclononane with three equivalents of a bromoacetamide derivative, obtained during step (i), of formula:

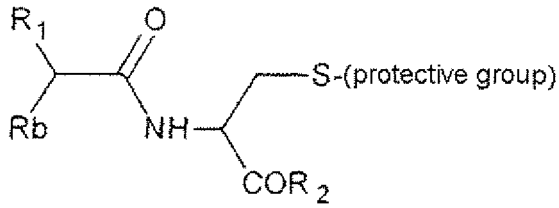

in the presence of a weak base, such as potassium hydrogen carbonate, sodium hydrogen carbonate, potassium carbonate, sodium carbonate and tertiary amines such as diisopropylethylamine or triethylamine, and in a solvent medium, said solvent being chosen from dichloromethane, chloroform, ethyl acetate, acetonitrile and dimethylformamide,

(iii) deprotecting the -S-(protective group) function to give a thiol-SH function, said deprotection being carried out by adding a large excess of a strong acid, such as trifluoroacetic acid when the protective group is triphenylmethane $C(C_6H_5)_3$, preferably at a temperature of between 20°C and 40°C for a period of typically between 15 minutes and 1 hour.

**3.** Non-therapeutic use of compounds of formula (I) according to claim 1 as chelating agents of metal ions of Mendeleev's periodic table, and preferably as chelating agents for Ag(I), Cd(II), Co(II), Cu(I), Hg(II), Ni(II), Au(I), Pb(II) and Zn(II) ions.

**4.** Use of the compounds of formula (I) according to of Claim 1, as depolluting agents.

**5.** Compounds susceptible to be used as agents that are precursors of the compounds of formula (I) as defined in Claim 1, corresponding to formula (II) below:

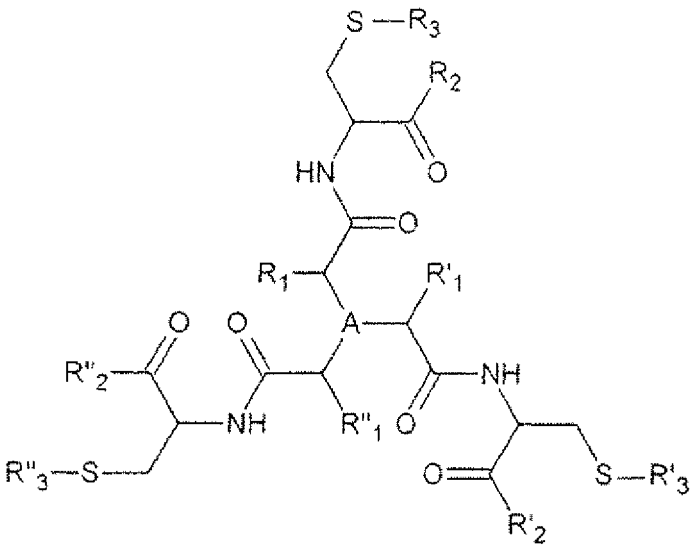

(II)

in which the group A represents:

- either a nitrogen atom,
- or a ring corresponding to the formula below, and in which the substitution takes place on the nitrogen atoms:

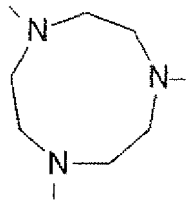

and in which:

- the $R_1$, $R'_1$ and $R''_1$ radicals, which may be identical or different, represent a hydrogen atom or an alkyl radical containing 1 to 12 carbon atoms,
- the $R_2$, $R'_2$ and $R''_2$ radicals, which may be identical or different, are chosen from-OH, -OR, -NHR and -NRR' groups in which R and R', which may be identical or different, represent a hydrogen atom or an alkyl radical containing 1 to 12 carbon atoms,
- the $R_3$, $R'_3$ and $R''_3$ radicals, which may be identical or different, represent a group -S-W or -S-E-L, wherein:

/ S is a sulfur atom,
/ W is an alkyl radical containing 1 to 12 carbon atoms,
/ E is a spacer arm that can be selected from alkyl groups containing 1 to 12 carbon atoms, and polyols such as polyethylene glycol preferably having 1 to 8 oxyethylene OE units,
/ L is a ligand selected from sugars such as glucose, galactose and N-acetylgalactosamine.

**6.** Compounds of formula (II) according to Claim 5, having the following specific formula ($II_a$):

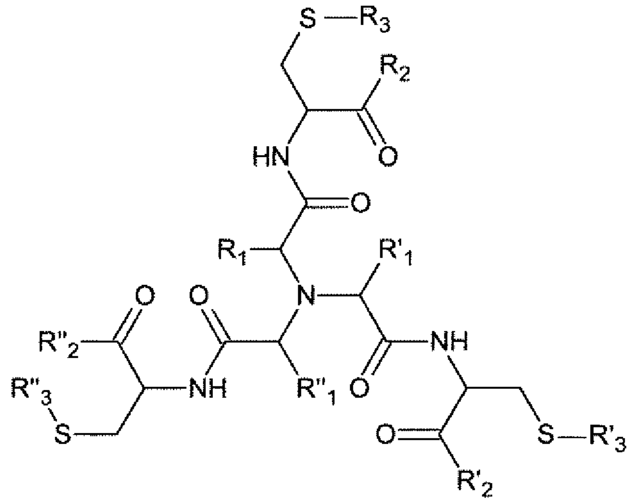

$(II_a)$

**7.** Compounds of formula ($II_a$) according to Claim 6, in which:

- the $R_1$, $R'_1$ and $R''_1$ radicals are hydrogen atoms,
- the $R_2$, $R'_2$ and $R''_2$ radicals are $-NH_2$ groups, and
- the $R_3$, $R'_3$ and $R''_3$ radicals, which may be identical or different, represent a group -S-E-L, wherein:

/ S is a sulfur atom,
/ E represents a polyethylene glycol having 3 oxyethylene OE units, and
/ L represents a N-acetylgalactosamine.

**8.** Compounds of formula (II) according to Claim 5, having the following specific formula ($II_b$):

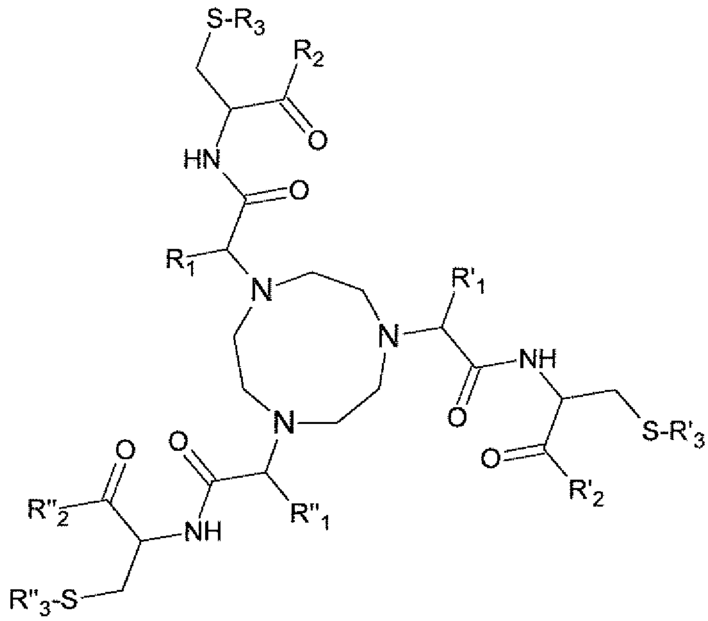

$(II_b)$

**9.** Compounds of following formula ($II_a$'):

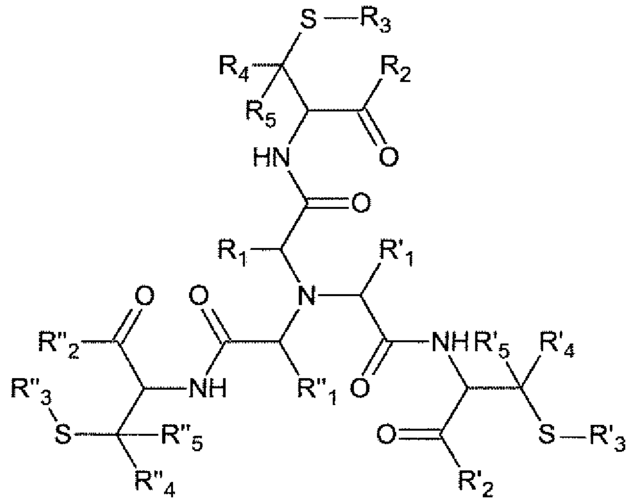

(II$_{a'}$)

in which :

- the $R_1$, $R'_1$, $R''_1$, $R_4$, $R'_4$, $R''_4$, $R_5$, $R'_5$, $R''_5$, radicals, which may be identical or different, represent a hydrogen atom or an alkyl radical containing 1 to 12 carbon atoms,
- the $R_2$, $R'_2$ and $R''_2$ radicals, which may be identical or different, are chosen from-OH, -OR, -NHR and -NRR' groups in which R and R', which may be identical or different, represent a hydrogen atom or an alkyl radical containing 1 to 12 carbon atoms,
- the $R_3$, $R'_3$ and $R''_3$ radicals, which may be identical or different, represent a group -S-W or -S-E-L, wherein:

  / S is a sulfur atom,
  / W is an alkyl radical containing 1 to 12 carbon atoms,
  / E is a spacer arm that can be selected from alkyl groups containing 1 to 12 carbon atoms, and polyols such as polyethylene glycol preferably having 1 to 8 oxyethylene OE units,
  / L is a ligand selected from sugars such as glucose, galactose and N-acetyl galactosamine.

**10.** Compounds of formula (III) below:

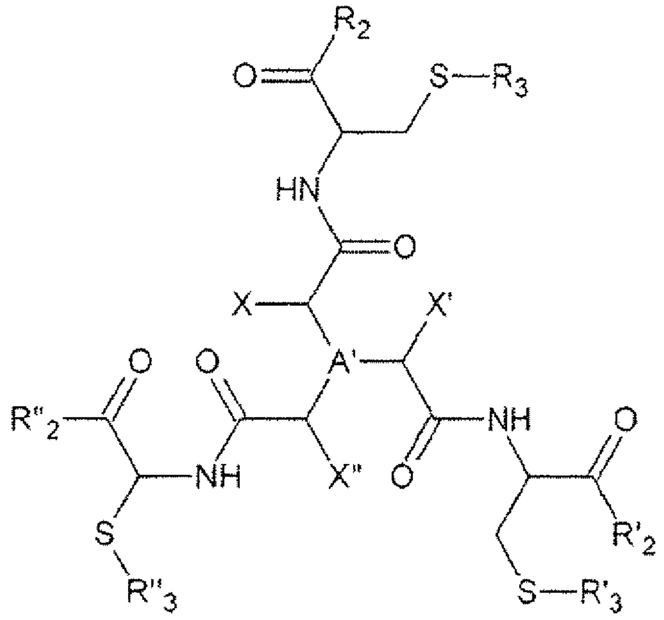

(III)

in which the group A' represents:

- either a nitrogen atom,
- or a ring corresponding to the formula below, and in which the substitution takes place on the nitrogen atoms:

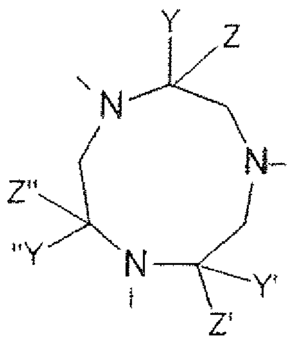

and in which:

- the $R_2$, $R'_2$ and $R''_2$ radicals, which may be identical or different, are chosen from -OH, -OR, -NHR and -NRR' groups in which R and R', which may be identical or different, represent a hydrogen atom or an alkyl radical containing 1 to 12 carbon atoms,
- the $R_3$, $R'_3$ and $R''_3$ radicals, which may be identical or different, represent a group -S-W or -S-E-L, wherein:

/ S is a sulfur atom,
/ W is an alkyl radical containing 1 to 12 carbon atoms,
/ E is a spacer arm that can be selected from alkyl groups containing 1 to 12 carbon atoms, and polyols such as polyethylene glycol preferably having 1 to 8 oxyethylene OE units,
/ L is a ligand selected from sugars such as glucose, galactose and N-acetylgalactosamine,

- X, X', X", Y, Y', Y", Z, Z' et Z", groups, which may be identical or different, represent a hydrogen atom, a $-(CH_2)_n$-NH-CO-marker group, a-$(CH_2)_n$-NH-C(S)NH-marker group, a $-(CH_2)_n$-NH-$SO_2$-marker group, a-$(CH_2)_n$-N=C-marker group, a $-(CH_2)_n$-NH-Ar-marker, or a $-(CH_2)_n$-triazole-marker, wherein Ar is an aryl group chosen from phenyl, triazole, oxadiazole, oxazole, imidazole,

thiadiazole, pyrrole, tetrazole, furan, thiophene, pyrazole, pyrazoline, pyrazidine, thiazole, isothiazole, pyridine, pyrimidine, piperidine, pyran, pyrazine and pyridazine, and in which n is comprised between 1 and 12, and with the proviso that at least one of said groups X, X', X", Y, Y', Y", Z, Z' or Z" is a $-(CH_2)_n$-NH-CO-marker group, $-(CH_2)_n$-NH-C(S)NH-marker,$-(CH_2)_n$-NH-$SO_2$-marker, $-(CH_2)_n$-N=C-marker or $-(CH_2)_n$-NH-Ar-marker or-$(CH_2)_n$-triazole-marker.

**11.** Compounds of formula (III) according to claim 10 in which:

- the $R_2$, $R'_2$ and $R''_2$ radicals $-NH_2$ groups,
- the $R_3$, $R'_3$ and $R''_3$ radicals, represent a group -S-E-L, wherein:

  / S is a sulfur atom,
  / E is a polyethylene glycol having 3 oxyethylene OE units, and
  / L represents a N-acetylgalactosamine,

- at least one of X, X', X", Y, Y', Y", Z, Z' or Z" groups represents a $-(CH_2)_4$-carboxytetramethylrhodamine group, the other groups being hydrogen atoms.

**12.** Compounds of formula (I), (II), ($II_{a'}$) or (III) as defined according to one of Claims 1 or 5 to 7 or 8 to 10, for use thereof as a medicament.

**13.** Use of the compounds of formula (I), (II), ($II_{a'}$) or (III) as defined according to one of Claims 1 or 5 to 11, for the manufacture of a medicament for the diagnosis, prevention and/or treatment of neurodegenerative diseases, such as Wilson's disease and Alzheimer's disease.

**14.** Compounds of formula (I), (II), ($II_{a'}$) or (III) as defined according to one of Claims 1 or 5 to 11, for use thereof for the diagnosis, prevention and/or treatment of poisoning with metal ions such as silver, cadmium, cobalt, copper, mercury, nickel, gold, lead or zinc ions.

**15.** Pharmaceutical composition, **characterized in that** it comprises, as active ingredient, at least one compound of formula (I), (II), ($II_{a'}$) or (III) as defined according to one of Claims 1 or 5 to 11, and at least one pharmaceutically acceptable carrier.

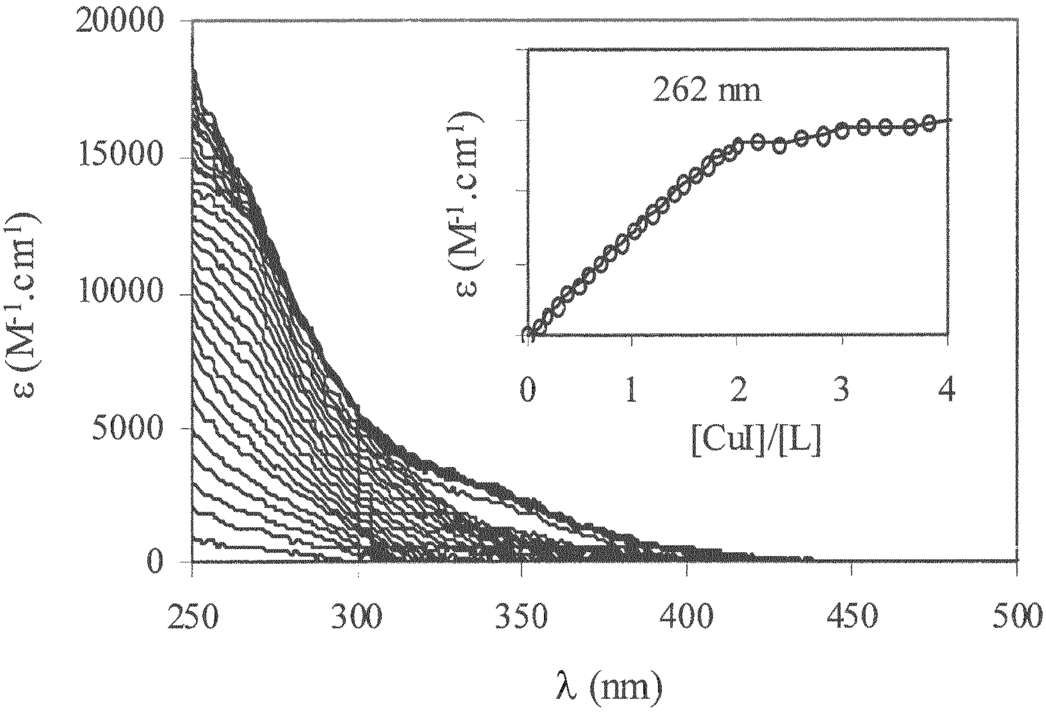
20000
15000
10000
5000
0
250
300
350
400
450
500
λ (nm)
ε (M-1.cm-1)
262 nm
0
1
2
3
4
[CuI]/[L]
ε (M-1.cm-1)

**FIGURE 1**

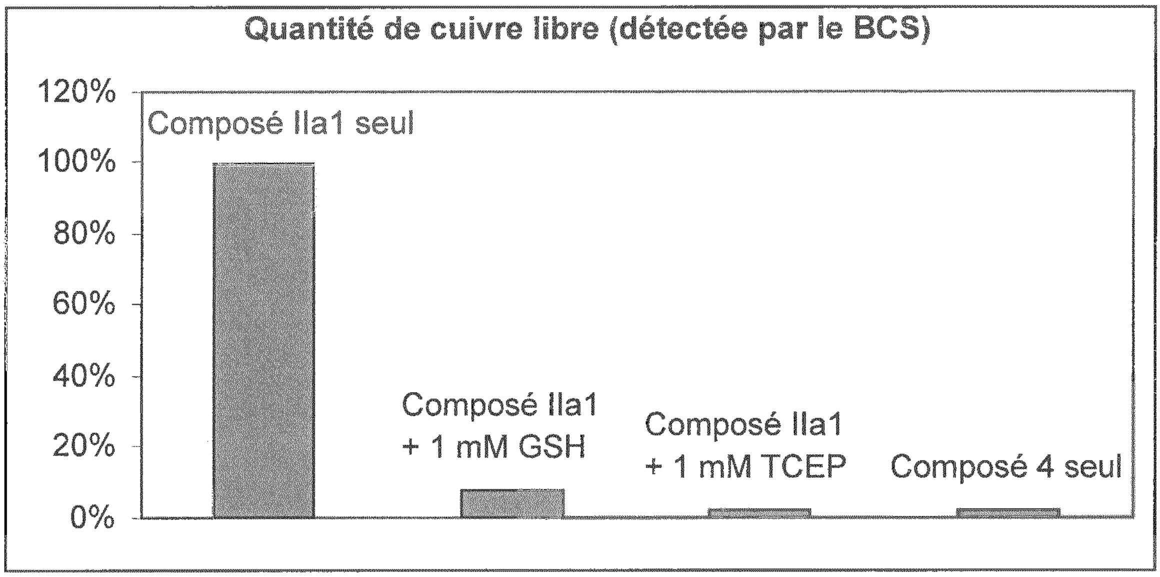
Quantité de cuivre libre (détectée par le BCS)
120%
100%
80%
60%
40%
20%
0%
Composé IIa1 seul
Composé IIa1
+ 1 mM GSH
Composé IIa1
+ 1 mM TCEP
Composé 4 seul

FIGURE 2

Composé $III_1$ - 2 heures
Cellules HepG2

$[III_1] = 0.2\ \mu M$ | $[III1] = 2\ \mu M$

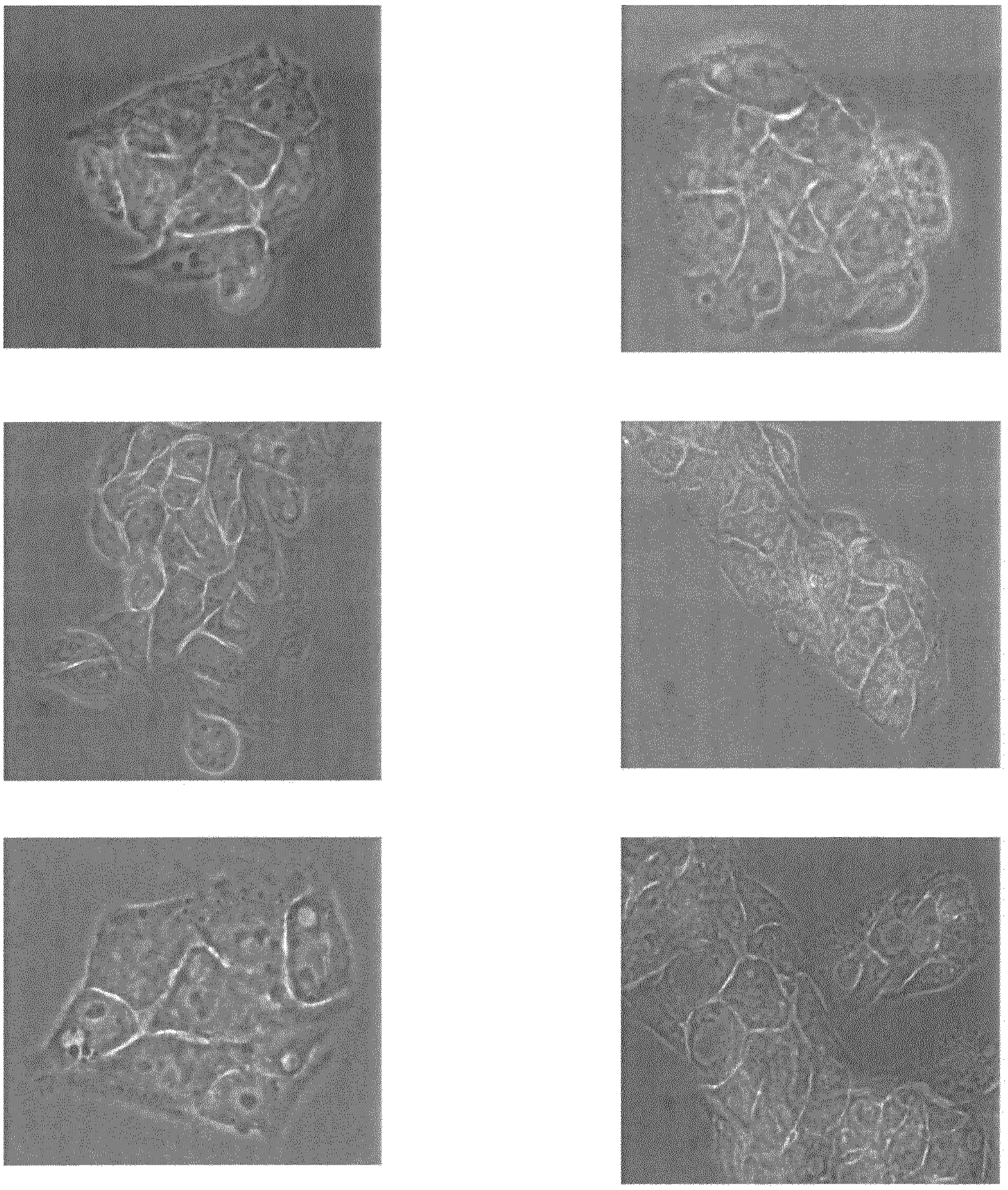

FIGURE 3

Composé $III_1$ - 7 heures
Cellules HepG2

$[III_1] = 0.2\ \mu M$ | $[III1] = 2\ \mu M$

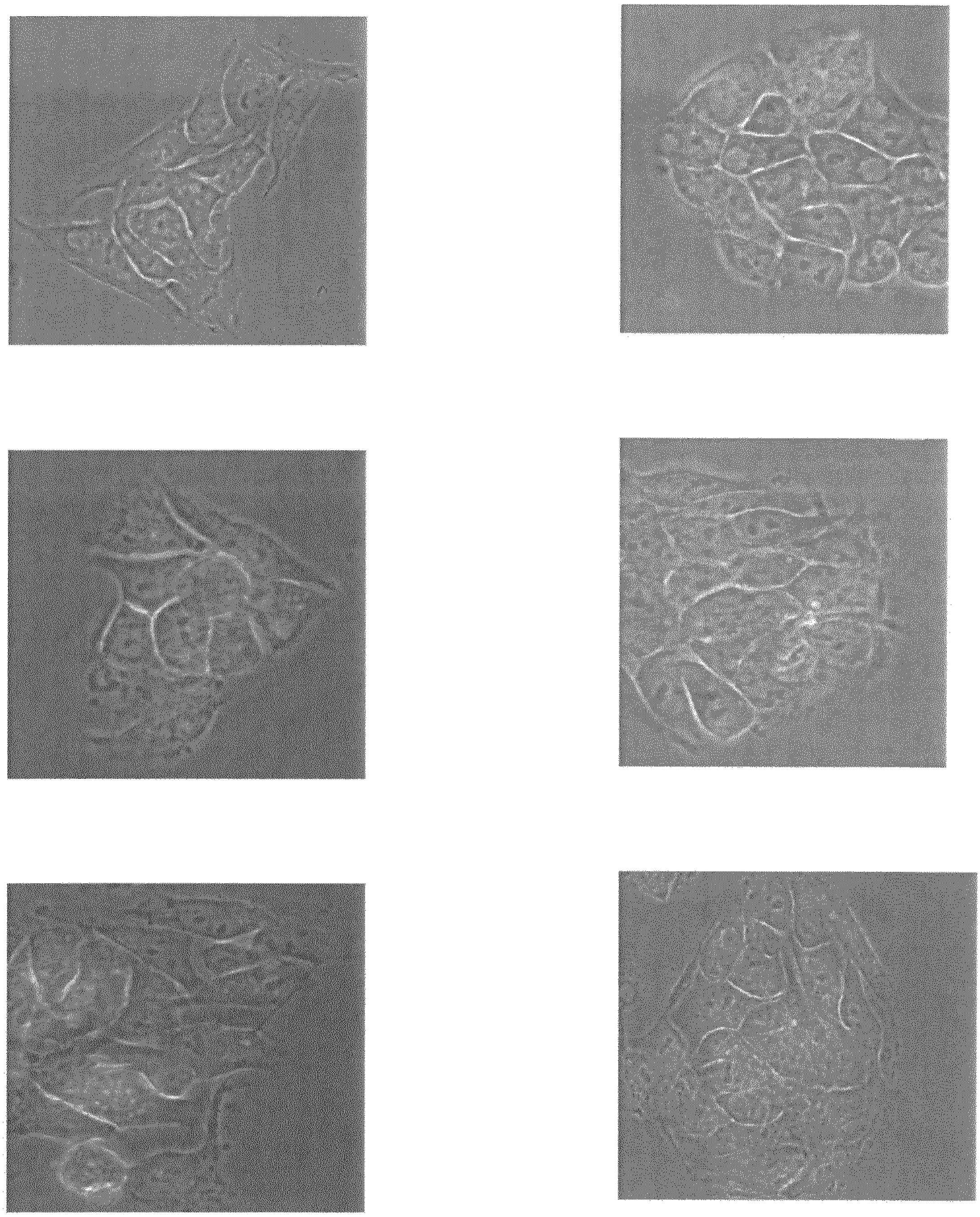

FIGURE 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Littérature non-brevet citée dans la description


- **G. J. BREWER.** *DDT,* 2005, vol. 10, 1103-1109
- **B. SARKAR.** *Chem. Rev.,* 1999, vol. 99, 2535-2544
- **E. GAGGELLI ; H. KOZLOWSKI ; D. VALENSIN ; G. VALENSIN.** *Chem. Rev.,* 2006, vol. 106, 1995-2044
- **O. ANDERSEN.** *Chem. Rev.,* 1999, vol. 99, 2683-2710
- **G. J. BREWER ; F. K. ASKARI.** *J. Hepatol.,* 2005, vol. 42, S13-S21
- *Dalton Trans.,* 2004, vol. 9, 1410-1417
- *Aust. J. Chem.,* 2008, vol. 61, 297-302
- **T. W. GREENE ; P. G. M. WUTS.** Protective groups in Organic Synthesis. Wiley, 1999
- **R. G. PEARSON.** *J. Am. Chem. Soc.,* 1963, vol. 85, 3533-3539
- Anti-Cancer Agents in Medicinal Chemistry. 2008, vol. 8, 497-522
- *Cytometry,* 2006, vol. 69A, 863-871
- *Anal. Bioanal. Chem.,* 2006, vol. 384, 620-630
- **BUCH A.** *Curent Opinion in Chemical Biology,* 2000, vol. 4, 184-191
- **HUI HUNG Y. et al.** *J. Biol. Inorg. Chem,* 2010, vol. 15, 61-76
- **BOLZATI et al.** *Bioconjugate chem.,* 2003, vol. 14, 1231
- *Synthesis,* 2003, vol. 11, 1699-1704
- **P. W. RIDDLES ; R. L. BLAKELEY ; B. ZEMER.** *Methods Enzymol.,* 1983, vol. 91, 49-60
- **P. ROUSSELOT-PAILLEY ; O. SÉNÈQUE ; C. LEBRUN ; S. CROUZY ; D. BOTURYN ; P. DUMY ; M. FERRAND ; P. DELANGLE.** *Inorg. Chem.,* 2006, vol. 45, 5510-5520
- **A. JERSHOW ; N. MÜLLER.** *J. Magn. Reson.,* 1997, vol. 125, 372-375
- **Z. XIAO ; F. LOUGHLIN ; G. N. GEORGE ; G. J. HOWLETT ; A. G. WEDD.** *J. Am. Chem. Soc.,* 2004, vol. 126, 3081-3090
- **J. C. PAYNE ; M. A. TERHORST ; H. A. GODWIN.** *J. Am. Chem. Soc,* 1999, vol. 121, 6850-6855
- **K. H. SCHELLER ; T. H. ABEL ; P. E. POLANYI ; P. K. WENK ; B. E. FISCHER ; H. SIGEL.** *Eur. J. Biochem.,* 1980, vol. 107, 455-466
- **PLUSH et al.** *Dalton Trans.,* 2004, vol. 9, 1410-1417
- **GUO et al.** *J. Am. Physiol Gastrointest Liver Physiol,* 2005, vol. 289, G904-G916